# EUROPEAN PATENT SPECIFICATION

(11) **EP 3 592 337 B1**
(45) Date of publication and mention of the grant of the patent: **05.07.2023**
(21) Application number: 18713502.5
(22) Date of filing: 08.03.2018
(51) Int. Cl.: A61K 9/16, A61K 9/24, A61K 9/50, A61K 31/196, A61K 9/48

(54) **PHARMACEUTICAL FORMULATIONS OF PHLOROGLUCINOL AND TRIMETHYLPHLOROGLUCINOL**
PHARMAZEUTISCHE FORMULIERUNGEN VON PHLOROGLUCINOL UND TRIMETHYLPHLOROGLUCINOL
FORMULATIONS PHARMACEUTIQUES DE PHLOROGLUCINOL ET DE TRIMÉTHYLPHLOROGLUCINOL

(30) Priority: 08.03.2017 US 201762468501 P
(43) Date of publication of application: 15.01.2020
(73) Proprietor: Cinrx Pharma, LLC, Cincinnati, OH 45227 (US)
(72) Inventor: PATEL, Piyush, Garnet Valley, PA 19060 (US); PEARCE, Catherine, Montgomery, OH 45242 (US); ISAACSOHN, Jonathan, Cincinnati, OH 45237 (US)
(74) Representative: Høiberg P/S
(86) International application number: PCT/US2018/021505
(87) International publication number: WO 2018/165404

(56) References cited:
- WO-A1-2013/087410
- "ANNEX I , SUMMARY OF PRODUCT CHARACTERISTICS: SPASFON, coated tablet ED - ANNEX I SUMMARY OF PRODUCT CHARACTERISTICS", INTERNET CITATION, 21 October 2008 (2008-10-21), pages 103-105, XP002675674, Retrieved from the Internet: URL:http://www.legemiddelverket.no/upload/ Core%20safety%20profiles/CSP%20phlorogluci nol%20200909.PDF [retrieved on 2012-05-07]
- ANITA ANNAH?ZI ET AL: "Role of antispasmodics in the treatment of irritable bowel syndrome", WORLD JOURNAL OF GASTROENTEROLOGY, vol. 20, no. 20, 28 May 2014 (2014-05-28), pages 6031-6043, XP055356010, CN ISSN: 1007-9327, DOI: 10.3748/wjg.v20.i20.6031

## Description

### FIELD OF THE INVENTION

The present disclosure relates to pharmaceutical compositions comprising phloroglucinol, trimethylphloroglucinol, pharmaceutically acceptable salts, or combinations thereof.

### BACKGROUND OF THE INVENTION

Phloroglucinol is chemically a benzenetriol, specifically 1,3,5-benzenetriol. It has a symmetric arene substitution pattern of a trisubstituted benzene. As a type of enol, it exists in two tautomeric forms which are in equilibrium: 1,3,5-trihydroxybenzene which has phenol-like character, and 1,3,5-cyclohexanetrione (phloroglucin) which has ketone-like character. 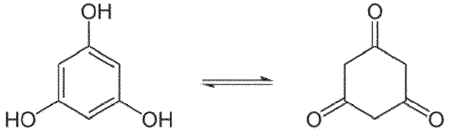 The three hydroxyl groups can be methylated, resulting in 1,3,5-trimethoxybenzene (trimethylphloroglucinol). Phloroglucinol acylated derivatives have a fatty acid synthase inhibitory activity.

Phloroglucinol (Phloroglucin^{™}, Phloroglucinol^{™}, Spasfon) is used medically as a non-specific antispasmodic. It has very weak anticholinergic properties and exerts its main action by directly relaxing smooth muscle cells. It is used to treat spasms, defined as a sudden involuntary muscle contraction, of blood and other vessels, bronchi, intestines, ureters, and gall bladder. Specific uses include treatment of e.g., urinary tract spasms, gallstones, spasmodic pain and related gastrointestinal disorders, renal colicky pain, and spastic conditions of the biliary tract associated with moderate abdominal pain. Smooth muscle cell relaxation appears highly selective, affecting the ureter and biliary tract more than the intestine and vascular beds. Part of its relaxant properties is due to its inhibition of the enzyme catechol-O-methyltransferase. Trimethylphloroglucinol has a similar pharmacological and toxicological profile to phloroglucinol, but the duration of action of trimethylphloroglucinol is about six time the duration of phloroglucinol.

Phloroglucinol administration is contraindicated in patients with a hypersensitivity to phloroglucinol or its methylated form, but precautions have not been determined. It is known to infrequently cause cutaneous hypersensitivity (allergic skin reactions). Anaphylactic shock has been reported with either intravenous or intramuscular administration of phloroglucinol.

Phloroglucinol is used to treat functional bowel disorders (FBD), also termed functional bowel disease. Diagnostic criteria (Rome III) are symptoms lasting more than six months that occur at least three days per month based on three monthly assessments; typical initial complaints are abdominal pain relieved by defecation and transit disorder. Diagnosis is by exclusion of underlying organic disorders such as Crohn's disease or colorectal cancer. The primary aim of treatment is restoration of normal gastrointestinal transit and alleviation of pain by relieving predominant symptoms of constipation and diarrhea.

Irritable bowel syndrome (IBS) is the most common cause of FBD. The current estimated prevalence of IBS in the general adult population is about 8%. IBS is a chronic condition with acute episodes characterized by abdominal pain and/or bloating associated with defecation and/or changes in bowel habit (diarrhea and/or constipation). Symptoms fluctuate and are typically exacerbated upon life stress events. Pathogenesis includes visceral hypersensitivity and/or increased or disorganized motor activity in the small bowel and/or colon. Individuals with diarrhea-predominant IBS have more jejunal contractions during phase II of the migrating motor complex and postprandial than healthy subjects, with a relationship between the occurrence of pain episodes and the onset of clusters of jejunal motor activity. Pain episodes have been also related to altered colonic phasic contractions and increased responsiveness to both the effects of eating and to stress. Visceral pain and altered gut motility may depend upon altered motility reflexes resulting from increased sensitivity of the digestive tract, providing rationale for using antispasmodic agents such as phloroglucinol for short-term treatment of acute painful episodes.

Overactive bladder or urge incontinence are colloquial terms for a condition in which the sensation of needing to void the bladder occurs suddenly, often severely, and without warning. The bladder muscle squeezes, forcing urine from the bladder and causing leakage. The spasms have been described as a cramping pain akin to severe menstrual cramps or labor contractions, and sometimes with a burning sensation. The etiology of bladder spasms may be diet, medication, changes in vascular supply to nerves enervating the bladder, infection, as a result of recent surgery, nerve damage, muscle damage, etc.

Quality of life is compromised in individuals with FBS, IBS, and overactive bladder. A goal of therapy is to restore regular bowel transit, controlled bladder voiding, and elimination of pain. Therapy is combined with lifestyle (avoid foods that exacerbate symptoms, initiate regular exercise) and dietary changes (increase fiber consumption if constipation is a symptom, and reduce fiber consumption if diarrhea is a symptom). Pharmacological treatment is administration of antispasmodics, particularly when abdominal pain and bloating are the predominant symptoms.

Phloroglucinol may treat FBS and IBS by enhancing rectosigmoid motor response. Regulatory evidence, however, was inconclusive, so it has not been approved for therapy in the U.S.

Phloroglucinol is orally administered, in one embodiment, at a dose of 80 mg up to 6 times a day, and in another embodiment, at a dose of 80 mg up to 3 times a day. A typical oral dose to manage spastic conditions of the urinary tract is 80 mg six times daily; some studies reported a dose of 80 mg 3 times a day orally administered. A parental route of 40 mg 2-3 times a day has been used but is not currently recommended. A typical rectal administration dose for bladder spasms and biliary tract spams is 150 mg 3 times a day.

Phloroglucinol has a peak blood concentration of 677 ng/ml achieved 20 minutes after a single oral dose of 160 mg. Its bioavailability (absorption) after an oral dose is 47%, with primarily renal metabolism. Phloroglucinol is excreted in urine mainly as hydroxylated metabolites, glucurono- and sulfo- conjugates, and partially as unmodified drug. It has a short plasma half-life of 1.5 hr.

The prevalence of IBS is linked to country and the diagnostic criteria used; it varies from 1% to 20%. One French study was survey based, and conducted by a self-administered questionnaire in 20,000 individuals, yielding 4.7% prevalence defined according to Rome II criteria (4.36%-5.04%). Another French study was survey based from telephone questions to 8,221 individuals, yielding an estimated 12% prevalence defined according to Manning criteria (with no reference to symptom duration), 2.5% including symptom duration), 2.1% according to Rome I and 1% according to Rome II criteria. No epidemiological studies assessed the prevalence of IBS according to the current criteria (Rome Ill); prevalence according to Rome III criteria should be higher than that for Rome II criteria, because Rome III criteria are less restrictive in terms of duration of active symptoms (symptoms had to have been present for at least six months for Rome III criteria compared with one year for Rome II criteria).

New compositions containing one or more of phloroglucinol, trimethylphloroglucinol, or a pharmaceutically acceptable salt thereof, which release phloroglucinol, trimethylphloroglucinol, or a pharmaceutically acceptable salt thereof for more are needed.
The applicability of both phloroglucinol and trimethylphloroglucinol as non-specific antispasmodics that reduce pain is disclosed in WO 2013/087410 A1, which describes an oral dosage form (immediate release form). In addition, "Annex 1, Summary of Product Characteristics: SPASFON, coated tablet ED; 21 October 2008" describes a coated tablet comprising phloroglucinol and trimethylphloroglucinol for use in the treatment of acute and chronic various spasmodic painful disorders.

### SUMMARY OF THE INVENTION

In some embodiments, the disclosure provides oral dosage units. The oral dosage units comprise an immediate release formulation comprising phloroglucinol, trimethylphloroglucinol, or a pharmaceutically acceptable salt thereof, wherein at least 90% by weight, based on the weight of the immediate release formulation, of phloroglucinol, trimethylphloroglucinol, or a pharmaceutically acceptable salt thereof is released from the dosage unit from 5 minutes to 2 hours, as measured by the USP 2 paddle method at 50 rpm in 750 mL of an aqueous solution comprising 0.1N HCl solution at 37°C. The oral dosage units also comprise a modified release formulation comprising phloroglucinol, trimethylphloroglucinol, or a pharmaceutically acceptable salt thereof, wherein at least 90% by weight, based on the weight of the modified release formulation, of phloroglucinol, trimethylphloroglucinol, or a pharmaceutically acceptable salt thereof, is released from the dosage unit after at least 2 hours, as measured by the USP 2 paddle method at 50 rpm in 1000 mL of an aqueous solution comprising 0.1N HCl and 20 mM sodium phosphate tribasic at a pH of 6.8 at 37°C.

In other embodiments, the disclosure provides oral dosage units, comprising a plurality of beads, each bead comprising an immediate release formulation comprising phloroglucinol, trimethylphloroglucinol, or a pharmaceutically acceptable salt thereof, wherein at least 90% by weight, based on the weight of the immediate release formulation, of phloroglucinol, trimethylphloroglucinol, or a pharmaceutically acceptable salt thereof is released from the dosage unit from 5 minutes to 2 hours, as measured by the USP 2 paddle method at 50 rpm in 750 mL of an aqueous solution comprising 0.1N HCl solution at 37°C; and a plurality of beads, each bead comprising a modified release formulation comprising phloroglucinol, trimethylphloroglucinol, or a pharmaceutically acceptable salt thereof, wherein at least 90% by weight, based on the weight of the modified release formulation, phloroglucinol, trimethylphloroglucinol, or a pharmaceutically acceptable salt thereof, is released from the dosage unit after at least 2 hours, as measured by the USP 2 paddle method at 50 rpm in 1000 mL of an aqueous solution comprising 0.1N HCl and 20 mM sodium phosphate tribasic at a pH of 6.8 at 37°C.

In further embodiments, the disclosure provides oral dosage units comprising a plurality of beads. In some aspects, each bead comprises a core that is in the form of an immediate release formulation comprising phloroglucinol, trimethylphloroglucinol, or a pharmaceutically acceptable salt thereof, wherein at least 90% by weight, based on the weight of the immediate release formulation, of phloroglucinol, trimethylphloroglucinol, or a pharmaceutically acceptable salt thereof is released from the dosage unit from 5 minutes to 2 hours, as measured by the USP 2 paddle method at 50 rpm in 750 mL of an aqueous solution comprising 0.1N HCl solution at 37°C. The beads also comprise a coating over the core that is (i) a modified release formulation comprising phloroglucinol, trimethylphloroglucinol, or a pharmaceutically acceptable salt thereof, wherein at least 90% by weight, based on the weight of the modified release formulation, phloroglucinol, trimethylphloroglucinol, or a pharmaceutically acceptable salt thereof, is released from the dosage unit after at least 2 hours, as measured by the USP 2 paddle method at 50 rpm in 1000 mL of an aqueous solution comprising 0.1N HCl and 20 mM sodium phosphate tribasic at a pH of 6.8 at 37°C; (ii) a modified release formulation comprising phloroglucinol, trimethylphloroglucinol, or a pharmaceutically acceptable salt thereof, wherein at least 90% by weight, based on the weight of the modified release formulation, phloroglucinol, trimethylphloroglucinol, or a pharmaceutically acceptable salt thereof, is released from the dosage unit after between 4 to 6 hours, as measured by the USP 2 paddle method at 50 rpm in 1000 mL of an aqueous solution comprising 0.1N HCl and 20 mM sodium phosphate tribasic at a pH of 6.8 at 37°C; or (iii) a combination of (i) and (ii).

In yet other embodiments, the disclosure provides methods of treating a spasmodic condition in a subject, comprising administering an oral dosage unit described herein to the subject.

Other aspects and embodiments of the invention will be readily apparent from the following detailed description of the invention.

### BRIEF DESCRIPTION OF THE FIGURES

The present application is further understood when read in conjunction with the appended drawings. For the purpose of illustrating the subject matter, there are shown in the drawings exemplary embodiments of the subject matter; however, the presently disclosed subject matter is not limited to the specific compositions, methods, devices, and systems disclosed. In addition, the drawings are not necessarily drawn to scale.
FIG. 1 shows a representative drug release profile for an immediate release (IR) formulation and a modified release formulation of the disclosure.
FIG. 2 is a simulated plasma profile of phloroglucinol from an immediate release portion and a modified release portion of the disclosure.
FIG. 3 shows a bilayer tablet of the disclosure with immediate release and modified release layers.
FIG. 4 shows a trilayer tablet of the disclosure containing immediate release, modified release, and buffer layers.
FIG. 5 shows a tablet of the disclosure with a modified release matrix and immediate release coating.
FIG. 6 shows a capsule of the disclosure containing an immediate release tablet, a plug, and a modified release tablet with an osmotic system.
FIG. 7 shows a capsule of the disclosure containing immediate release and modified release beads.
FIG. 8 shows a capsule of the disclosure containing immediate and modified release mini-tablets.
FIG. 9 shows a capsule of the disclosure containing immediate release and modified release granules.
FIG. 10 shows a capsule of the disclosure containing a modified release bead coated with an immediate release layer.
FIG. 11 shows a compressed tablet of the disclosure containing immediate release granules and a coated modified release tablet embedded within the compressed tablet.
FIG. 12 shows a compressed immediate release tablet of the disclosure with a modified release tablet embedded within the immediate release tablet.
FIG. 13 shows a modified release tablet of the disclosure suspended in an immediate release liquid.
FIG. 14 shows a sachet of the disclosure containing a mixture of immediate release and modified release granules or beads.
FIG. 15 shows a sachet of the disclosure containing effervescent immediate release granules or beads and coated modified release granules or beads.
FIG. 16 shows a tablet of the disclosure with intermediate layers separated by bands.
FIG. 17 shows an orally disintegrating tablet of the disclosure containing coated, delayed/modified release drug particles, beads or granules; the inset shows a drug in a polymer matrix.
FIG. 18 shows a capsule of the disclosure containing drug solution and coated, delayed/modified release drug particles, beads or granules.
FIG. 19 shows a softgel of the disclosure containing drug solution and coated, delayed/modified release drug particles, beads or granules.
FIG. 20 shows a liquid vehicle of the disclosure containing coated, modified release drug particles, beads or granules.
FIG. 21 is the delayed release profile for the immediate release/ modified release formulation of Example 1.

### DETAILED DESCRIPTION OF THE INVENTION

In the present disclosure the singular forms "a", "an" and "the" include the plural reference, and reference to a particular numerical value includes at least that particular value, unless the context clearly indicates otherwise. Thus, for example, a reference to "a material" is a reference to at least one of such materials and equivalents thereof known to those skilled in the art, and so forth.

The modifier "about" should be considered as disclosing the range defined by the absolute values of the two endpoints. For example, the expression "from about 2 to about 4" also discloses the range "from 2 to 4." When used to modify a single number, the term "about" may refer to plus or minus 10% of the indicated number and includes the indicated number. For example, "about 10%" may indicate a range of 9% to 11%, and "about 1" means from 0.9 to 1.1.

When a list is presented, unless stated otherwise, it is to be understood that each individual element of that list and every combination of that list is to be interpreted as a separate embodiment. For example, a list of embodiments presented as "A, B, or C" is to be interpreted as including the embodiments, "A," "B," "C," "A or B," "A or C," "B or C," or "A, B, or C."

It is to be appreciated that certain features of the invention which are, for clarity, described herein in the context of separate embodiments, may also be provided in combination in a single embodiment. That is, unless obviously incompatible or excluded, each individual embodiment is deemed to be combinable with any other embodiment(s) and such a combination is considered to be another embodiment. Conversely, various features of the invention that are, for brevity, described in the context of a single embodiment, may also be provided separately or in any sub-combination. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation. Finally, while an embodiment may be described as part of a series of steps or part of a more general structure, each said step may also be considered an independent embodiment in itself.

"Pharmaceutically acceptable" means approved or approvable by a regulatory agency of the Federal or a state government or the corresponding agency in countries other than the United States, or that is listed in the U.S. Pharmacopoeia or other generally recognized pharmacopoeia for use in animals, and more particularly, in humans.

The terms "patient" or "subject" as used herein refer to a mammalian animal and are used interchangeably. In some embodiments, the patient or subject is a human. In other embodiments, the patient or subject is a veterinary or farm animal, a domestic animal or pet, or animal normally used for clinical research.

"Treating" any disease or disorder refers, in some embodiments, to ameliorating a disease or disorder (i.e., arresting or reducing the development of the disease or at least one of the clinical symptoms thereof). The "treating" refers to ameliorating a disease or disorder using phloroglucinol, trimethylphloroglucinol, or a combination thereof. In some embodiments, "treating" or "treatment" refers to ameliorating at least one physical parameter, which may not be discernible by the subject. In other embodiments, "treating" or "treatment" refers to modulating the disease or disorder, either physically, (e.g., stabilization of a discernible symptom), physiologically, (e.g., stabilization of a physical parameter), or both. In further embodiments, "treating" or "treatment" refers to delaying the onset of the disease or disorder.

The term "phloroglucinol" as used herein refers to the following compound.

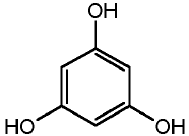

Phloroglucinol also includes any tautomeric forms thereof, including its known keto tautomer shown below.

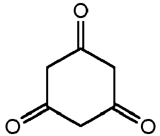

Similarly, the term "trimethylphloroglucinol" as used herein refers to the following compound.

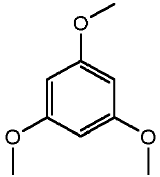

As discussed herein, the present disclosure provides dosage units that are formulated for oral administration, i.e., oral dosage units. The oral dosage unit may take a variety of delivery forms. In some embodiments, the dosage unit is a tablet, capsule (hard or soft), sachet, soft gel, liquid, gel, strip, film, or tablet-in-capsule. In other embodiments, the dosage unit is a tablet, capsule, sachet, softgel, or liquid. In further embodiments, the oral dosage unit is a tablet. In other embodiments, the oral dosage unit is a capsule. In yet further embodiments, the oral dosage unit is a sachet.

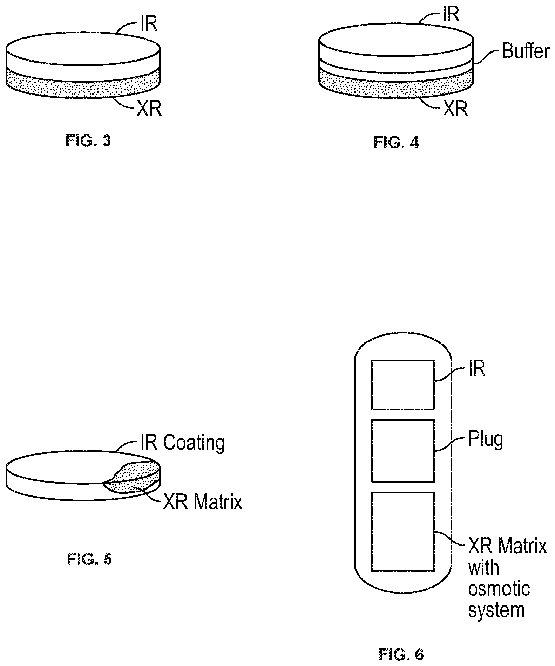

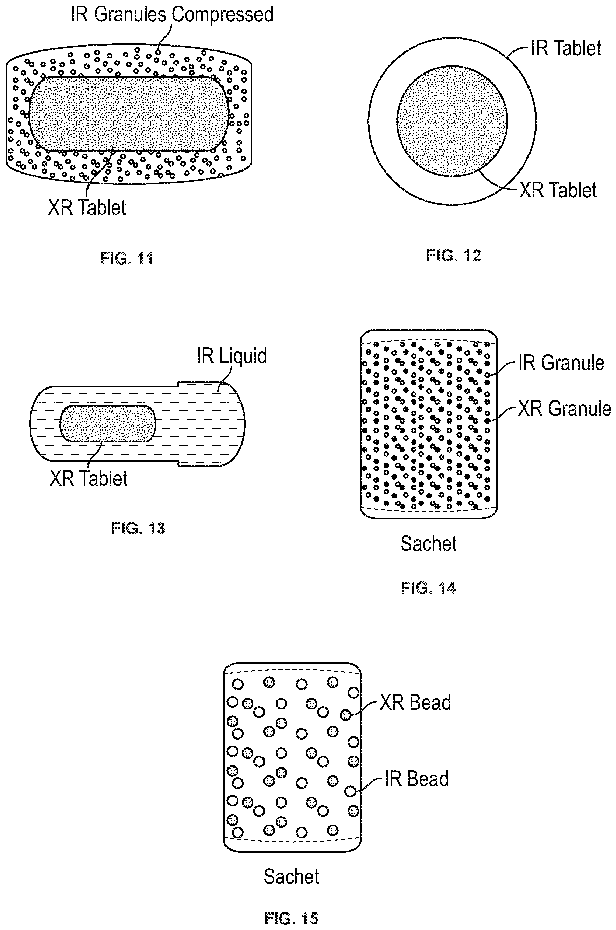

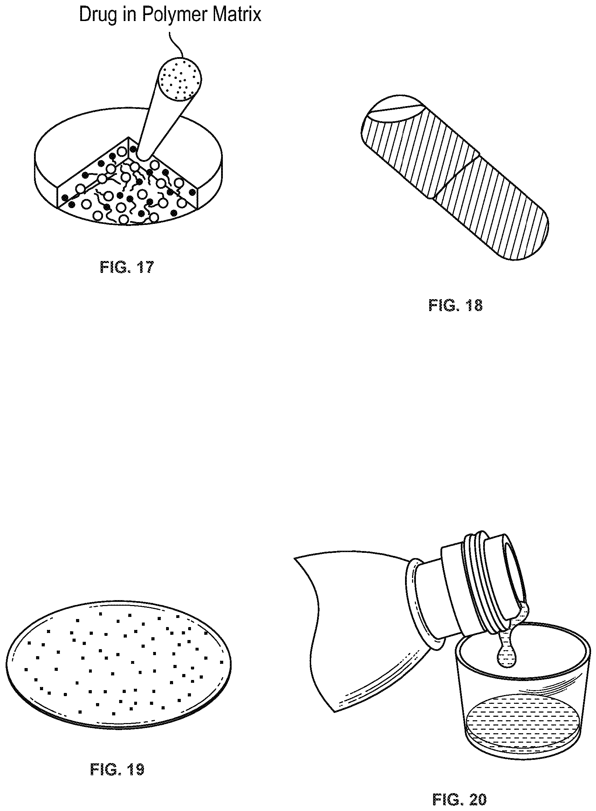

The term "tablet" as used herein refers to a solid dosage unit. The tablet may be of any shape or size convenient for oral administration, e.g., circular, elliptical, etc. A tablet is prepared by compacting one or both of the immediate release and modified release formulations. In some embodiments, the tablet is prepared by compressing the immediate release formulation. In other embodiments, the tablet is prepared by compressing one or more modified release formulations. In further embodiments, the tablet is prepared by compressing the immediate release and one or more modified release formulations. Depending on the base of the tablet, it may be coated with a layer comprising the immediate release formulation or modified release formulation. In some embodiments, tablet is a bilayer tablet containing immediate release (IR) and modified release layers adjacent to each other. See, e.g., FIG. 3. In other embodiments, the tablet is a trilayer tablet containing immediate release and modified release layers separated by a layer, for example, a buffer layer. See, e.g., FIG. 4. In further embodiments, the tablet contains embedded within the tablet, granules coated with the immediate release formulation and beads coated with the modified release formulation. See, e.g., FIG. 11. In yet other embodiments, the tablet contains a tablet comprising the modified release formulation embedded within a tablet comprising the immediate release formulation. See, e.g., FIG. 12. In still further embodiments, the tablet contains a tablet comprising a modified release formulation that is suspended in a liquid solution comprising the immediate release formulation, wherein the liquid solution is contained within a capsule. See, e.g., FIG. 13. In other embodiments, a capsule of the disclosure contains a solution comprising the immediate release formulation and coated, beads or granules coated with a modified release formulation. See, e.g., FIG. 18. In further embodiments, a softgel of the disclosure contains a solution comprising the immediate release formulation and beads or granules are coated with the modified release formulation. See, e.g., FIG. 19. In yet other embodiments, FIG. 20 shows a liquid vehicle comprising the immediate release formulation and beads or granules coated with a modified release formulation.

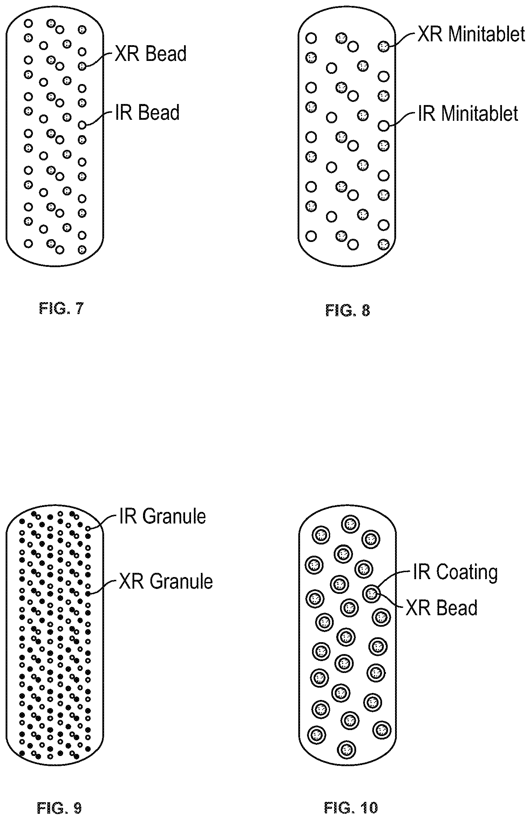

The term "capsule" as used herein refers to a solid dosage unit. The capsule is typically elliptical in shape, but can adopt other forms, as determined by those skilled in the art. The capsule may be a hard or soft gelatin capsule, as needed. In some embodiments, the capsule contains a tablet comprising the immediate release formulation and a tablet comprising the modified release formulation. In further embodiments, the capsule contains an immediate release tablet, a plug, and a modified release tablet. See, e.g., FIG. 6. In other embodiments, the capsule contains beads coated with an immediate release formulation and beads coated with a modified release formulation. See, e.g., FIG. 7. In further embodiments, the capsule contains immediate release mini-tablets and modified release mini-tablets. See, e.g., FIG. 8. In still other embodiments, the capsule contains immediate release granules and the granules are coated with a modified release formulation. See, e.g., FIG. 9. In yet other embodiments, the capsule contains a plurality of beads coated with modified release and immediate release formulations as layers.

The term "sachet" as used herein refers to a package that contains a mixture of immediate release and modified release granules or beads comprising the immediate release formulation and granules or beads comprising the modified release formulation. See, e.g., FIG. 14. The package may be selected by those skilled in the art.

Regardless of the form of the dosage unit, it may alternatively or in addition contain beads, granules, or a combination thereof. As used herein, the "beads" are solid particles that are prepared by extrusion and spheronization of the immediate release formulation, modified release formulation, or a combination thereof. Similarly, the "granules" are solid particles, but they are prepared via a granulation. One of skill in the art would be able to select a suitable granulation method to prepare the granules for use herein. In some embodiments, the granulation method includes high-shear granulation, melt granulation, dry granulation, or wet granulation, among others. In some embodiments, dosage unit contains beads comprising the immediate release formulation. In other embodiments, the dosage unit contains beads comprising the modified release formulation. In further embodiments, the dosage unit contains bead comprising the immediate release formulation and beads comprising the modified release formulation. In yet other embodiments, dosage unit contains beads comprising the immediate release formulation. In other embodiments, the dosage unit contains beads comprising the modified release formulation. In further embodiments, the dosage unit contains bead comprising the immediate release formulation and beads comprising the modified release formulation.

Typically, a plurality of beads or granules is incorporated into the dosage unit described herein. The term "plurality" as used herein refers to a number of beads or granules that provide the amount of phloroglucinol, trimethylphloroglucinol, or pharmaceutically acceptable salt required by the dosage unit. In some embodiments, the dosage unit comprises a plurality of beads. In further embodiments, the dosage unit comprises a plurality of granules. In other embodiments, the dosage unit comprises a plurality of beads and a plurality of granules.

The beads and/or granules contain one or both of the immediate release or modified release formulations. In some embodiments, the beads comprise the immediate release formulation. In other embodiments, the beads comprise the modified release formulation. In further embodiments, the beads comprise the immediate release and modified release formulations. In yet other embodiments, the granules comprise the immediate release formulation. In still further embodiments, the granules comprise the modified release formulation. In other embodiments, the granules comprise the immediate release and modified release formulations.

Each bead or granule comprises a core and one or more optional coating layers. Thus, the core contains one or both of the immediate release or modified release formulation. In some embodiments, the core also contains an inactive pharmaceutical agent such as an excipient as described herein. The cores have a diameter of about 50 to about 1500 µm. In some embodiments, the cores have a diameter of about 50 to about 1300 µm, about 50 to about 1100 µm, about 50 to about 900 µm, about 50 to about 800 µm, about 50 to about 700 µm, about 50 to about 600 µm, about 50 to about 500 µm, about 50 to about 400 µm, about 50 to about 300 µm, about 50 to about 200 µm, about 100 to about 1500 µm, about 100 to about 1300 µm, about 100 to about 1100 µm, about 100 to about 900 µm, about 100 to about 800 µm, about 100 to about 700 µm, about 100 to about 600 µm, about 100 to about 500 µm, about 100 to about 400 µm, about 100 to about 300 µm, about 100 to about 200 µm. In other embodiments, the core diameter is about 100 to about 800 µm.

The dosage unit may have multiple cores of active with varying dissolution properties. Thus, the cores may be coated one or more layers. In some embodiments, the cores are coated with two or more layers, i.e., a multilayer tablet. In further embodiments, the cores are coated with an immediate release formulation layer. In other embodiments, the cores are coated with a modified release formulation layer. In yet further embodiments, the core is coated with an immediate release formulation and coated with a modified release formulation.

Other layers may be applied as a topcoat or in between the other layers. The layers may contain pharmaceutically inert components, i.e., as a buffer layer, or pharmaceutically active components, as determined by those skilled in the art.

The oral dosage units comprise one or more of phloroglucinol, trimethylphloroglucinol, or a pharmaceutically acceptable salt of phloroglucinol or trimethylphloroglucinol. In some embodiments, the oral dosage unit comprises phloroglucinol or a pharmaceutically acceptable salt thereof. In other embodiments, the oral dosage unit comprises trimethylphloroglucinol or a pharmaceutically acceptable salt thereof. In further embodiments, the oral dosage unit comprises phloroglucinol or a pharmaceutically acceptable salt thereof and trimethylphloroglucinol or a pharmaceutically acceptable salt thereof

In some embodiments, pharmaceutically acceptable salts can be formed from organic and inorganic acids including, e.g., acetic, propionic, lactic, citric, tartaric, succinic, fumaric, maleic, malonic, mandelic, malic, phthalic, hydrochloric, hydrobromic, phosphoric, nitric, sulfuric, methanesulfonic, napthalenesulfonic, benzenesulfonic, toluenesulfonic, camphorsulfonic, and similarly known acceptable acids.

In other embodiments, pharmaceutically acceptable salts may also be formed from inorganic bases, desirably alkali metal salts including, e.g., sodium, lithium, or potassium, such as alkali metal hydroxides. Examples of inorganic bases include, without limitation, sodium hydroxide, potassium hydroxide, calcium hydroxide, and magnesium hydroxide. Pharmaceutically acceptable salts may also be formed from organic bases, such as ammonium salts, mono-, di-, and trimethylammonium, mono-, di- and triethylammonium, mono-, di- and tripropylammonium, ethyldimethylammonium, benzyldimethylammonium, cyclohexylammonium, benzyl-ammonium, dibenzylammonium, piperidinium, morpholinium, pyrrolidinium, piperazinium, 1-methylpiperidinium, 4-ethylmorpholinium, 1-isopropylpyrrolidinium, 1,4-dimethylpiperazinium, 1 n-butyl piperidinium, 2-methylpiperidinium, l-ethyl-2-methylpiperidinium, mono-, di- and triethanolammonium, ethyl diethanolammonium, n-butylmonoethanolammonium, tris(hydroxymethyl)methylammonium, phenylmono-ethanolammonium, diethanolamine, ethylenediamine, and the like. In one example, the base is sodium hydroxide, lithium hydroxide, potassium hydroxide, or mixtures thereof.

The compounds discussed above may be used in the form of salts derived from pharmaceutically or physiologically acceptable acids, bases, alkali metals and alkaline earth metals.

Desirably, the amount of phloroglucinol, trimethylphloroglucinol, or a pharmaceutically acceptable salt thereof is sufficient to treat a patient as set forth below. In some embodiments, the oral dosage units contain about 50 to about 1000 mg of phloroglucinol, trimethylphloroglucinol, or a pharmaceutically acceptable salt thereof, or combination thereof. In other embodiments, the oral dosage unit contains about 50 to about 900 mg, about 50 to about 800 mg, about 50 to about 700 mg, about 50 to about 600 mg, about 50 to about 500 mg, about 50 to about 400 mg, about 50 to about 300 mg, about 50 to about 200 mg, about 50 to about 100 mg, about 100 to about 900 mg, about 100 to about 800 mg, about 100 to about 700 mg, about 100 to about 600 mg, about 100 to about 500 mg, about 100 to about 400 mg, about 100 to about 300 mg, about 100 to about 200 mg, about 100 to about 100 mg, about 200 to about 900 mg, about 200 to about 800 mg, about 200 to about 700 mg, about 200 to about 600 mg, about 200 to about 500 mg, about 200 to about 400 mg, about 200 to about 300 mg, about 300 to about 900 mg, about 300 to about 800 mg, about 300 to about 700 mg, about 300 to about 600 mg, about 300 to about 500 mg, about 300 to about 400 mg, about 400 to about 900 mg, about 400 to about 800 mg, about 400 to about 700 mg, about 400 to about 600 mg, about 400 to about 500 mg, about 500 to about 900 mg, about 500 to about 800 mg, about 500 to about 700 mg, about 500 to about 600 mg, about 600 to about 900 mg, about 600 to about 800 mg, or about 600 to about 700 mg of phloroglucinol, trimethylphloroglucinol, or a pharmaceutically acceptable salt thereof, or combination thereof.

The oral dosage unit contains one or more immediate release and one or more modified release components. In some embodiments, the oral dosage unit contains at least one immediate release formulation and at least one modified release formulation. In other embodiments, the dosage unit contains at least one immediate release formulation and at least two modified release formulations, i.e., a first modified release formulation and a second modified release formulation. In further embodiments, the dosage unit contains at least one immediate release formulation and at least three modified release formulations, i.e., a first modified release formulation, a second modified release formulation, and a third modified release formulation. In yet other embodiments, the dosage unit contains at least one immediate release formulation and at least fourth modified release formulations, i.e., a first modified release formulation, a second modified release formulation, a third modified release formulation, and a fourth modified release formulation.

Both the immediate release formulation and modified release formulation contain phloroglucinol, trimethylphloroglucinol, or a pharmaceutically acceptable salt thereof. In some embodiments, the immediate release formulation contains phloroglucinol or a pharmaceutically acceptable salt thereof. In other embodiments, the immediate release formulation contains trimethylphloroglucinol or a pharmaceutically acceptable salt thereof. In further embodiments, the immediate release formulation contains (i) phloroglucinol or a pharmaceutically acceptable salt thereof and (ii) trimethylphloroglucinol or a pharmaceutically acceptable salt thereof In yet other embodiments, the modified release formulation contains phloroglucinol or a pharmaceutically acceptable salt thereof. In still further embodiments, the modified release formulation contains trimethylphloroglucinol or a pharmaceutically acceptable salt thereof. In other embodiments, the modified release formulation contains (i) phloroglucinol or a pharmaceutically acceptable salt thereof and (ii) trimethylphloroglucinol or a pharmaceutically acceptable salt thereof.

The oral dosage unit comprises about 10 to about 50% by weight, based on the weight of the oral dosage unit, of the immediate release formulation. In some embodiments, the oral dosage unit comprises about 10 to about 50% by weight, about 10 to about 40% by weight, about 10 to about 30% by weight, about 10 about 20% by weight, about 20 to about 50% by weight, about 20 to about 40% by weight, about 20 to about 30% by weight, about 30 to about 50% by weight, or about 40 to about 50% by weight, based on the weight of the oral dosage unit, of the immediate release formulation. In other embodiments, the oral dosage unit comprises about 10, 15, 20, 25, 30, 35, 40, 45, or 50% by weight, based on the weight of the oral dosage unit, of the immediate release formulation.

The oral dosage unit comprises about 10 to about 50% by weight, based on the weight of the oral dosage unit, of the modified release formulation. In some embodiments, the oral dosage unit comprises about 10 to about 50% by weight, about 10 to about 40% by weight, about 10 to about 30% by weight, about 10 about 20% by weight, about 20 to about 50% by weight, about 20 to about 40% by weight, about 20 to about 30% by weight, about 30 to about 50% by weight, or about 40 to about 50% by weight, based on the weight of the oral dosage unit, of the modified release formulation. In other embodiments, the oral dosage unit comprises about 10, 15, 20, 25, 30, 35, 40, 45, or 50% by weight, based on the weight of the oral dosage unit, of the modified release formulation.

As for the oral dosage unit described above, one or both of the immediate release formulation or modified release formulation is a tablet, capsule (hard or soft), sachet, soft gel, liquid, gel, strip, film, or tablet-in-capsule. In other embodiments, one or both of the immediate release formulation or modified release formulation is a tablet, capsule, sachet, softgel, or liquid.

The term "immediate release" as used herein refers a dosage unit that, upon oral ingestion by a human, releases substantially all of phloroglucinol, trimethylphloroglucinol or pharmaceutically acceptable salts thereof, by weight, into a portion of the gastrointestinal tract (e.g., the stomach or the intestine, preferably the stomach) for biological uptake in a short time. *In vitro* methods of measuring a release profile of a dosage unit, for the purpose of determining whether a dosage unit exhibits an immediate release or extended release dissolution profile, are known in the pharmaceutical arts. By such methods, the dosage units as described herein can be measured to be capable of releasing substantially all of a total amount of phloroglucinol, trimethylphloroglucinol, or a pharmaceutically acceptable salt thereof contained in the immediate release formulation. In some embodiments, at least about 90% weight, based on the weight of the immediate release formulation, the phloroglucinol, trimethylphloroglucinol, or a pharmaceutically acceptable salt thereof is released into a solution (e.g., acidic aqueous solution). In other embodiments, about 90 to about 100% by weight, about 95 to about 100% by weight, about 98 to 100% by weight, about 99 to 100% by weight, or 90, 91, 92, 93, 94, 95, 96, 97, 89, 99, or 100% by weight of the phloroglucinol, trimethylphloroglucinol, or a pharmaceutically acceptable salt thereof is released from the immediate release formulation. The phloroglucinol, trimethylphloroglucinol, or a pharmaceutically acceptable salt thereof is released from the immediate release formulation in about 5 minutes to about 2 hours, e.g., about 5 minutes to about 1.5 hours, about 5 minutes to about 1 hour, about 5 minutes to about 45 minutes, about 5 minutes to about 30, about 15, or about 10 minutes. In some embodiments, a release profile of the immediate release formulation portion of the dosage unit described herein is measured by the USP 711 method. In some embodiments, a release profile of the immediate release formulation portion of the dosage unit described herein is measured by the USP 2 paddle method. In other embodiments, a release profile the immediate release formulation portion of the dosage unit described herein is measured by a method that exposes the immediate release formulation portion to a speed of about 45 to about 55 rpm (e.g., 45, 46, 47, 48, 49, 50, 51, 52, 53, 54, or 55 rpm, preferably about 50 rpm) and a volume of up to about 900 mL (e.g., about 300 mL, 750 mL, or about 900 mL, based on various test methods) of hydrochloric acid (about 0.01 to about 0.2N, preferably about 0.1N, e.g., aqueous hydrochloric acid) and at a temperature of about 35 to about 40 (e.g., 35, 36, 37, 38, 39, or 40°C, preferably about 37°C). For example, a release profile of the immediate release formulation of the dosage unit of the present description may be measured by a method that exposes the dosage unit to about 50 rpm in about 750 mL of an aqueous solution comprising about 0.1N HCl solution at about 37°C.

The term "modified release" as used herein refers to the slow release of the phloroglucinol, trimethylphloroglucinol, or a pharmaceutically acceptable salt thereof over several hours into the gastrointestinal tract (e.g., the stomach or the intestine) and colon for biological uptake over a long time. *In vitro* methods of measuring a release profile of a dosage unit, for the purpose of determining whether a dosage unit exhibits a modified release dissolution profile, are known in the pharmaceutical arts. By such methods, the dosage units as described herein can be measured to be capable of releasing substantially all of a total amount of phloroglucinol, trimethylphloroglucinol, or a pharmaceutically acceptable salt thereof contained in the modified release formulation. In some embodiments, at least about 90% weight, based on the weight of the modified release formulation, the phloroglucinol, trimethylphloroglucinol, or a pharmaceutically acceptable salt thereof is released into a solution (e.g., acidic aqueous solution). In other embodiments, about 90 to about 100% by weight, about 95 to about 100% by weight, about 98 to 100% by weight, about 99 to 100% by weight, or 90, 91, 92, 93, 94, 95, 96, 97, 89, 99, or 100% by weight of the phloroglucinol, trimethylphloroglucinol, or a pharmaceutically acceptable salt thereof is released from the modified release formulation. The phloroglucinol, trimethylphloroglucinol, or a pharmaceutically acceptable salt thereof is released from the modified release formulation in at least about 2 hours, about 2 to about 12 hours, about 2 to about 11 hours, about 2 to about 10 hours, about 2 to about 9 hours, about 2 to about 8 hours, about 2 to about 7 hours, about 2 to about 6 hours, about 2 to about 5 hours, about 2 to about 4 hours, about 2 to about 3 hours, about 4 to about 12 hours, about 4 to about 11 hours, about 4 to about 10 hours, about 4 to about 9 hours, about 4 to about 9 hours, about 4 to about 8 hours, about 4 to about 7 hours, about 4 to about 6 hours, about 6 to about 12 hours, about 6 to about 11 hours, about 6 to about 10 hours, about 6 to about 9 hours, about 6 to about 8 hours, about 8 to about 12 hours, about 8 to about 11 hours, about 8 to about 10 hours, or about 10 to about 12 hours. Desirably, each modified release formulation contains a different release profile. In some embodiments, a first modified release formulation releases the phloroglucinol, trimethylphloroglucinol, or a pharmaceutically acceptable salt thereof from the modified release formulation in about 5 minutes to about 2 hours. In other embodiments, a second modified release formulation, if present, release the phloroglucinol, trimethylphloroglucinol, or a pharmaceutically acceptable salt thereof from the modified release formulation in at least about 2 hours. In further embodiments, a second modified release formulation, if present, release the phloroglucinol, trimethylphloroglucinol, or a pharmaceutically acceptable salt thereof from the modified release formulation in at least about 4 to about 6 hours.

In some embodiments, a release profile of the modified immediate release formulation portion of the dosage unit described herein is measured by the USP 2 paddle method. In other embodiments, a release profile the modified release formulation portion of the dosage unit described herein is measured by a method that exposes the modified release formulation portion to a speed of about 45 to about 55 rpm (e.g., 45, 46, 47, 48, 49, 50, 51, 52, 53, 54, or 55 rpm, preferably about 50 rpm) and a volume of up to about 1100 mL (e.g., about 300 mL, about 500 mL, about 750 mL, or about 1000 mL, preferably about 1000 mL based on various test methods) of an aqueous solution comprising a phosphate based buffer (e.g., an aqueous solution comprising hydrochloric acid hydrochloric acid (about 0.01 to about 0.2N, preferably about 0.1N, e.g., aqueous hydrochloric acid) and sodium phosphate tribasic (about 10 to about 30 mM, about 10 to about 20 mM, about 20 to about 30 mM, about 15 to about 25 mM, preferably about 20 mM) and at a pH of about 6.5 to about 7 (e.g., 6.5, 6.6, 6.7, 6.8, 6.9, or 7, preferably 6.8), and at a temperature of about 35 to about 40 (e.g., 35, 36, 37, 38, 39, or 40°C, preferably about 37°C). In some embodiments, a release profile of the modified release formulation of the dosage unit of the present description is measured by a method that exposes the dosage unit to about 50 rpm in about 1000 mL of an aqueous solution comprising about 0.1N HCl and about 20 mM sodium phosphate tribasic at a pH of about 6.8 at about 37°C. In other embodiments, a release profile of the modified release formulation of the dosage unit is measured by a method that exposes the dosage unit to about 50 rpm in about 1000 mL of an aqueous solution comprising about 0.1N HCl and about 20 mM sodium phosphate tribasic at a pH of about 6.8 at about 37°C. In further embodiments, the dosage unit contains a modified release formulation, wherein at least about 90% by weight, based on the weight of the modified release formulation, phloroglucinol, trimethylphloroglucinol, or a pharmaceutically acceptable salt thereof, is released from the dosage unit after at least about 2 hours, as measured by the USP 2 paddle method at about 50 rpm in about 1000 mL of an aqueous solution comprising about 0.1N HCl and about 20 mM sodium phosphate tribasic at a pH of about 6.8 at about 37°C. In yet other embodiments, the dosage unit contains a modified release formulation, wherein at least about 90% by weight, based on the weight of the modified release formulation, phloroglucinol, trimethylphloroglucinol, or a pharmaceutically acceptable salt thereof, is released from the dosage unit after between about 4 to about 6 hours, as measured by the USP 2 paddle method at about 50 rpm in about 1000 mL of an aqueous solution comprising about 0.1N HCl and about 20 mM sodium phosphate tribasic at a pH of about 6.8 at about 37°C. In still other embodiments, the dosage unit contains two modified release formulations (i) a modified release formulation, wherein at least about 90% by weight, based on the weight of the modified release formulation, phloroglucinol, trimethylphloroglucinol, or a pharmaceutically acceptable salt thereof, is released from the dosage unit after at least about 2 hours, as measured by the USP 2 paddle method at about 50 rpm in about 1000 mL of an aqueous solution comprising about 0.1N HCl and about 20 mM sodium phosphate tribasic at a pH of about 6.8 at about 37°C; and (ii) a modified release formulation, wherein at least about 90% by weight, based on the weight of the modified release formulation, phloroglucinol, trimethylphloroglucinol, or a pharmaceutically acceptable salt thereof, is released from the dosage unit after between about 4 to about 6 hours, as measured by the USP 2 paddle method at about 50 rpm in about 1000 mL of an aqueous solution comprising about 0.1N HCl and about 20 mM sodium phosphate tribasic at a pH of about 6.8 at about 37°C.

In some embodiments, the oral dosage unit has an immediate release profile and a modified release profile. In other embodiments, the oral dosage unit has an immediate release profile defined as not less than 90% of phloroglucinol, trimethylphloroglucinol, or a pharmaceutically acceptable salt thereof released in about 5 minutes to about 2 hours, and an extended release profile defined as not less than about 90% by weight of phloroglucinol, trimethylphloroglucinol, or a pharmaceutically acceptable salt thereof released in at least about 2 hours.

In some embodiments, the immediate release formulation contains about 50 to about 800 mg of phloroglucinol, trimethylphloroglucinol, or a pharmaceutically acceptable salt thereof. In other embodiments, the immediate release formulation contains about 50 to about 700 mg, about 50 to about 600 mg, about 50 to about 500 mg, about 50 to about 400 mg, about 50 to about 300 mg, about 50 to about 200 mg, about 50 to about 100 mg, about 100 to about 800 mg, about 100 to about 700 mg, about 100 to about 600 mg, about 100 to about 500 mg, about 100 to about 400 mg, about 100 to about 300 mg, about 100 to about 200 mg, about 100 to about 100 mg, about 200 to about 800 mg, about 200 to about 700 mg, about 200 to about 600 mg, about 200 to about 500 mg, about 200 to about 400 mg, about 200 to about 300 mg, about 300 to about 800 mg, about 300 to about 700 mg, about 300 to about 600 mg, about 300 to about 500 mg, about 300 to about 400 mg, about 400 to about 800 mg, about 400 to about 700 mg, about 400 to about 600 mg, about 400 to about 500 mg, about 500 to about 800 mg, about 500 to about 700 mg, about 500 to about 600 mg, about 600 to about 800 mg, or about 600 to about 700 mg of phloroglucinol, trimethylphloroglucinol, or a pharmaceutically acceptable salt thereof.

Similarly, the modified release formulation contains about 50 to about 800 mg of phloroglucinol, trimethylphloroglucinol, or a pharmaceutically acceptable salt thereof. In other embodiments, the modified release formulation contains about 50 to about 700 mg, about 50 to about 600 mg, about 50 to about 500 mg, about 50 to about 400 mg, about 50 to about 300 mg, about 50 to about 200 mg, about 50 to about 100 mg, about 100 to about 800 mg, about 100 to about 700 mg, about 100 to about 600 mg, about 100 to about 500 mg, about 100 to about 400 mg, about 100 to about 300 mg, about 100 to about 200 mg, about 100 to about 100 mg, about 200 to about 800 mg, about 200 to about 700 mg, about 200 to about 600 mg, about 200 to about 500 mg, about 200 to about 400 mg, about 200 to about 300 mg, about 300 to about 800 mg, about 300 to about 700 mg, about 300 to about 600 mg, about 300 to about 500 mg, about 300 to about 400 mg, about 400 to about 800 mg, about 400 to about 700 mg, about 400 to about 600 mg, about 400 to about 500 mg, about 500 to about 800 mg, about 500 to about 700 mg, about 500 to about 600 mg, about 600 to about 800 mg, or about 600 to about 700 mg of phloroglucinol, trimethylphloroglucinol, or a pharmaceutically acceptable salt thereof.

When the dosage unit described herein contains both phloroglucinol and trimethylphloroglucinol, or pharmaceutically acceptable salts thereof, the ratio of phloroglucinol to trimethylphloroglucinol (or salts thereof) is about 90:10 to about 10:90. In some embodiments, the ratio of phloroglucinol to trimethylphloroglucinol (or salts thereof) is about 80:20 to about 20:80, about 70:30 to about 30:70, about 60:40 to about 40:60, about 50:50 to about 50:50, about 40:60 to about 60:40, about 30:70 to about 70:30, or about 20:80 to about 80:20. In other embodiments, the ratio of phloroglucinol to trimethylphloroglucinol (or salts thereof) is about 90:10, about 85:15, about 80:20, about 75:25, about 70:30, about 65:35, about 60:40, about 55:45, about 50:50, about 45:55, about 40:60, about 35:65, about 30:70, about 25:75, about 20:80, about 15:85, or about 10:90.

The dosage unit may contain separate and discrete portions of the immediate release formulation and one or more modified release formulations, i.e., they are physically separated. Thus, in some embodiments, the dosage unit may contain an immediate release formulation and a modified release formulation. In other embodiments, the dosage unit may contain an immediate release formulation, a first modified release formulation, and a second modified release formulation.

Alternatively, a portion of the immediate release formulation and a portion of modified release formulation are attached, i.e., one formulation is a layer on the other formulation. The term "portion" as used herein refers to the surface of a formulation. In some embodiments, portion refers to at least about 50% by weight, e.g., at least about 55, about 60, about 65, about 70, about 75, about 80, about 85, about 90, about 95, about 98, about 99, or 100% by weight, based on the weight of the formulation. In some embodiments, the immediate release formulation contains a layer of the modified release formulation. In other embodiments, the modified release formulation contains a layer of the immediate release formulation. When the dosage unit contains two or more modified release formulations, in some embodiments, the immediate release formulation is coated with the first modified release formulation, which is then coated with the second modified release formulation. In other embodiments, the immediate release formulation is coated with the second modified release formulation, which is then coated with the first modified release formulation. In some embodiments, the oral dosage unit comprises a plurality of beads, each bead comprising a core that is in the form of an immediate release formulation comprising phloroglucinol, trimethylphloroglucinol, or a pharmaceutically acceptable salt thereof, a coating over the core that is (i) a modified release formulation comprising phloroglucinol, trimethylphloroglucinol, or a pharmaceutically acceptable salt thereof, (ii) a modified release formulation comprising phloroglucinol, trimethylphloroglucinol, or a pharmaceutically acceptable salt thereof, (iii) or a combination of (i) and (ii).

The modified release formulation comprises an agent that provides the modified release profile discussed above. In some embodiments, the modified release formulation comprises an enteric polymer. An enteric polymer refers to a polymer that is resistant to degradation in gastric juice (i.e., relatively insoluble at the low pH levels found in the stomach), but dissolves at the higher pH levels found in the intestinal tract. Examples of enteric polymers include, without limitation, cellulose acetate phthalate, cellulose acetate trimellitate, hydroxypropyl methylcellulose phthalate, polyvinyl acetate phthalate such as the Sureteric^{®} polymer, carboxymethylethylcellulose, a copolymer of methacrylic acid/methacrylic acid methyl esters such as, e.g., EUDRAGIT^{®} L12.5, EUDRAGIT^{®} L100, or EUDRAGIT^{®} S12.5, S100, a copolymer of methacrylic acid and ethyl acrylate such as, e.g., Acryl-EZE^{®} polymer, or esters of aleurtic acid such as shellac. Aqueous colloidal polymer dispersions or re-dispersions can be also applied, e.g. EUDRAGIT^{®} L 30D-55, EUDRAGIT^{®} L100-55, EUDRAGIT^{®} S100, EUDRAGIT^{®} preparation 4110D (Rohm Pharma); AQUATERIC^{®}, AQUACOAT^{®} CPD 30 (FMC); KOLLICOAT MAE^{®} 30D and. 30DP (BASF); EASTACRYL^{®} 30D (Eastman Chemical). In some embodiments, the enteric polymer is a copolymer of methacrylic acid and ethyl acrylate such as, e.g., Acryl-EZE^{®} polymer. In further embodiments, the enteric polymer is a phthalate polymer such as the Sureteric^{®} polymer.

The compositions described can contain one or more pharmaceutically acceptable excipients that are considered safe and effective and may be administered to an individual without causing undesirable biological side effects or unwanted interactions. Exemplary excipients include, but are not limited to, antimicrobial agents, antioxidants, binders, diluents, disintegrants, emulsifiers, flavoring agents, glidants, isotonicity modifying agents, lubricants, pH modifying agents, plasticizers, preservatives, sweeteners, stabilizers, suspending agents, viscosity increasing agents, or combinations thereof, and.

Binders include acacia, gum tragacanth, corn starch, gelatin, sucrose, pre-gelatinized starch, starch, sodium alginate, ammonium calcium alginate, methylcellulose, sodium cellulose derivatives such as methylcellulose, carboxymethyl cellulose, hydroxyethyl cellulose, hydroxypropyl cellulose, ethyl cellulose, hydroxypropylmethyl cellulose, polyvinylpyrrolidone, aluminum ciliate and polyacrylamide.

Disintegration agents or disintegrants include corn starch, potato starch, pregelatinized starch, cross-linked carboxymethylcellulose (AC-DI-SOL^{®}), sodium starch glycolate (EXPLOTAB^{®}), cross-linked polyvinylpyrrolidone (PLASDONE XL^{®}), etc.

Colorants or coloring agents include synthetic and natural dyes and combinations thereof.

Diluents or carriers may include water, alcohols, oils, glycols such as polyethyleneglycols, among others. Examples of diluents include, without limitation, arachis oil, almond oil, peanut oil, palm oil, palm kernel oil, peppermint oil, blackcurrent seed oil, rice bran oil, soybean oil, canola oil, corn oil, coconut oil, cotton seed oil, castor oil, olive oil, Linn oils (Neem), sesame oil, primrose oil, vegetable oil, LIPEX^{®} 108 (abitec), wheat germ oil, fish oil, rapeseed oil, sunflower oil and safflower oil, polyethylene glycols, polyoxyethylene 32 lauric glycerides (ACCONON^{®} M-44), polyoxyethylene 8 caprylicleapric glycerides (ACCONON^{®} MC-8), glyceryl stearates (IMWITOR^{®}), polyoxyethylated oleic glycerides (LABRAFIL^{®}), mineral oil, mono- and diglyceride emulsifiers such as glyceryl monooleate, glyceryl monocaprate, glyceryl monocaprylate, propylene glycol monocaprylate, and propylene glycol monolaurate (CAPMUL^{®}), dimethylpolysiloxanes such as simethicone, glycofurol, glycerin, ethanol glycerol, propylene glycol, or polyethylene glycols (PEG)-400. In some embodiments, the carrier is water or an alcohol.

Flavorings or flavoring agents can be used to mask unpleasant odors and tastes of fill formulations. Suitable flavorings include synthetic and natural flavorings.

Humectants can be used to suppress the water activity of the softgel. Suitable humectants include glycerin and sorbitol, which are often components of the plasticizer composition.

Opacifiers are used to opacify the capsule shell when the encapsulated active agents are light sensitive. Suitable opacifiers include titanium dioxide, zinc oxide, calcium carbonate and combinations thereof.

Plasticizers are chemical agents added to gelatin to make the material softer and more flexible. Suitable plasticizers include, but are not limited to, glycerin, sorbitol solutions which are mixtures of sorbitol and sorbitan, and other polyhydric alcohols such as propylene glycol and maltitol or combinations thereof.

Preservatives include alkyl esters of p-hydroxy benzoic acid such as methyl, ethyl, propyl, butyl and heptyl esters (collectively known as "parabens") or combinations thereof.

Solubilizers include citric acid, succinic acid, fumaric acid, malic acid, tartaric acid, maleic acid, glutaric acid, sodium bicarbonate, sodium carbonate, among others.

Sweeteners include sucrose, lactose, dextrose, mannitol or saccharin.

Surfactants include ionic, non-ionic, and/or bile salt surfactants, with anionic surfactants including sodium alkyl sulfate (sodium lauryl sulfate) and sulfosuccinate derivatives such as docusate sodium, non-ionic surfactants including polyoxyethylene sorbitan fatty acid esters (polysorbates) such as TWEEN^{®} 20, TWEEN^{®} 80, TWEEN^{®} 40, SPAN^{®} 20, fatty acid esters of polyethylene glycols such as GELUCIRE^{®} 44/14, GELUCIRE^{®} 50/13, saturated polyglycolized (including mono, di or tri)glycerides, medium chain monoglycerides (6-10 carbons) such as glyceryl monocaprylate (IMWITOR^{®} 308), glyceryl monocaproate (CAPMUL^{®} MCM C-8), glyceryl caprylate/caprate (CAPMUL^{®} MCM), polyoxyethylene glyceryl caprylate, and polyoxyethylene glyceryl caproate (LABRASOL^{®}), medium chain fatty acid esters such as glyceryl tri caprate and glyceryltricarilate (MIGLYOL^{®} 612), block polymers of ethylene oxide and propylene oxide, polyoxyethylene-polyoxy propylene block copolymers such as poloxamer 188 (PLURONIC^{®} F-68), poloxamer 237 (PLURONIC^{®} F-87), poloxamer 338 (PLURONIC^{®} F-108), poloxamer 407 (PLURONIC^{®} F-127), poloxamer 124 (PLURONIC^{®} L-44), polyoxy stearate-polyethoxylated (40) stearic acid (MYRJ^{™} 52), ethoxylated castor oil-polyethoxylated (60) hydrogenated castor oil (CREMOPHOR^{®} EL), ethoxylated hydrostearic acid polyethylene glycol 660 hydroxystearate (SOLUTOL^{®} HS 15), polyoxyethylene alkyl ethers (12-18 carbons) such as polyoxy 20 cetostearyl ether (ATLAS^{™} G-3713), polyoxy 10 oleyl ether (BRIJ^{™} 96, BRIJ^{™} 97, Oleth 10), polyethylene glycol ether (TRITON^{™} X-100, TRITON^{™} X-114, TRITON^{™} X-405, TRITON^{™} N-101) and lecithins such as phospholipids (dimyristoyl DL-alpha-phophatidylcholine), bile salt surfactants including deoxycholic acid, sodium deoxycholate, cholic acid, sodium taurocholate; etc.

Stabilizers include antioxidation agents, buffers, acids, etc. Examples of antioxidants may include, but are not limited to, ascorbic acid, ascorbyl palmitate, butylated hydroxyanisole (BHA), butylated hydroxytoluene (BHT), diethylenetriaminepentaacetic acid (DTPA), edetates (EDTA), monothioglycerol, sodium ascorbate, sodium formaldehyde sulfoxylate, sodium metabisulfite, sodium bisulfite, triglycoamate, vitamin E or a derivative thereof, propyl gallate, combinations thereof, or the like.

Viscosity increasing agents include gelatin, glycerin, carrageenan, colloidal silicon dioxide, hydrogenated vegetable oil; povidone, or propylene glycol alginate.

The dosage units may contain another pharmaceutically active component in addition to phloroglucinol or trimethylphloroglucinol. In some embodiments, the dosage units may also contain antispasmodic agents such as alverine citrate, meberverine, otilonium bromide, pinaverium bromide, dicyclomine hydrochloride, XIFASAN (rifaximin), VIBERZI^{®} (eluxadoline), or LOTRONEX^{®} (alosetron), among others. Other antispasmodic agents include those discussed in Annaházi, "Role of antispasmodics in the treatment of irritable bowel syndrome," World J. Gastroenterol., May 28, 2014, 20(20): 6031-6043.

Other layers/coatings may be applied as a topcoat or in between the other layers/coatings. Thus, coatings may be provided to minimize dust during handling, improve appearance, improve swallowability, provide a gloss, act as a sealant, minimize static, and/or provide color, among others. Suitable coating thicknesses may be determined by those skilled in the art. The layers may contain pharmaceutically inert components or pharmaceutically active components, as determined by those skilled in the art.

In some embodiments, an oral dosage unit is provided and comprises (i) an immediate release formulation comprising phloroglucinol, trimethylphloroglucinol, or a pharmaceutically acceptable salt thereof, wherein at least about 90% by weight, based on the weight of the immediate release formulation, of phloroglucinol, trimethylphloroglucinol, or a pharmaceutically acceptable salt thereof is released from the dosage unit from about 5 minutes to about 2 hours, as measured by the USP 2 paddle method at about 50 rpm in about 750 mL of an aqueous solution comprising about 0.1N HCl solution at about 37°C; and (ii) a modified release formulation comprising phloroglucinol, trimethylphloroglucinol, or a pharmaceutically acceptable salt thereof, wherein at least about 90% by weight, based on the weight of the modified release formulation, of phloroglucinol, trimethylphloroglucinol, or a pharmaceutically acceptable salt thereof, is released from the dosage unit after at least about 2 hours, as measured by the USP 2 paddle method at about 50 rpm in about 1000 mL of an aqueous solution comprising about 0.1N HCl and about 20 mM sodium phosphate tribasic at a pH of about 6.8 at about 37°C.

In other embodiments, an oral dosage unit is provided and comprises (i) a plurality of beads, each bead comprising an immediate release formulation comprising phloroglucinol, trimethylphloroglucinol, or a pharmaceutically acceptable salt thereof, wherein at least about 90% by weight, based on the weight of the immediate release formulation, of phloroglucinol, trimethylphloroglucinol, or a pharmaceutically acceptable salt thereof is released from the dosage unit after about 1 hour, as measured by the USP 2 paddle method at about 50 rpm in about 750 mL of an aqueous solution comprising about 0.1N HCl at about 37°C; and (ii) a plurality of beads, each bead comprising a modified release formulation comprising phloroglucinol, trimethylphloroglucinol, or a pharmaceutically acceptable salt thereof, wherein at least about 90% by weight, based on the weight of the modified release formulation, phloroglucinol, trimethylphloroglucinol, or a pharmaceutically acceptable salt thereof, is released from the dosage unit after at least about 2 hours, as measured by the USP 2 paddle method at about 50 rpm in about 1000 mL of an aqueous solution comprising about 0.1N HCl and about 20 mM sodium phosphate tribasic at a pH of about 6.8 at about 37°C.

In further embodiments, oral dosage unit is provided and comprises a plurality of beads, each bead comprising (a) a core that is in the form of an immediate release formulation comprising phloroglucinol, trimethylphloroglucinol, or a pharmaceutically acceptable salt thereof, wherein at least about 90% by weight, based on the weight of the immediate release formulation, of phloroglucinol, trimethylphloroglucinol, or a pharmaceutically acceptable salt thereof is released from the dosage unit after about 1 hour, as measured by the USP 2 paddle method at about 50 rpm in about 750 mL of an aqueous solution comprising about 0.1N HCl at about 37°C; and (b) a coating over the core that is (i) a modified release formulation comprising phloroglucinol, trimethylphloroglucinol, or a pharmaceutically acceptable salt thereof, wherein at least about 90% by weight, based on the weight of the modified release formulation, phloroglucinol, trimethylphloroglucinol, or a pharmaceutically acceptable salt thereof, is released from the dosage unit after at least about 2 hours, as measured by the USP 2 paddle method at about 50 rpm in about 1000 mL of an aqueous solution comprising about 0.1N HCl and about 20 mM sodium phosphate tribasic at a pH of about 6.8 at about 37°C; or (ii) a modified release formulation comprising phloroglucinol, trimethylphloroglucinol, or a pharmaceutically acceptable salt thereof, wherein at least about 90% by weight, based on the weight of the modified release formulation, phloroglucinol, trimethylphloroglucinol, or a pharmaceutically acceptable salt thereof, is released from the dosage unit after between about 4 to about 6 hours, as measured by the USP 2 paddle method at about 50 rpm in about 1000 mL of an aqueous solution comprising about 0.1N HCl and about 20 mM sodium phosphate tribasic at a pH of about 6.8 at about 37°C; or (iii) a combination of (i) and (ii).

The dosage units and formulations described herein are useful in treating spasmodic conditions in a subject. The methods comprise administering an oral dosage unit described herein to the subject. In some embodiments, the spasmodic condition is a sudden involuntary muscle contraction of a body part, such as an organ or muscle, of the subject. In other embodiments, the spasmodic condition is a sudden involuntary muscle contraction of the bronchi, stomach, intestine, ureter, gall bladder, kidney, or bile duct. In further embodiments, the spasmodic condition is a urinary tract spasm, gallstones, a gastrointestinal disorder, inflammatory bowel syndrome, renal colicky pain, or a spastic condition of the biliary tract.

Unless otherwise indicated, a formulation is a dosage form. A tablet is a non-limiting example of a dosage form. Dispersion and disintegration of the formulation are used synonymously. As used herein, the active ingredient, abbreviated as "active", is phloroglucinol, trimethylphloroglucinol, or combinations of phloroglucinol and trimethylphloroglucinol. As used herein, extended release and sustained release are generally used synonymously.

Each of a bead and a pellet is any discrete component of a dosage form, e.g., a capsule shell may be filled with a plurality of beads and/or a plurality of pellets.

An immediate release formulation and a delayed release formulation indicate the onset of release of the active in relationship to administration. An immediate release formulation indicates release of the active from the formulation beginning within a relatively shorter period of time post administration, e.g. a few minutes or less. A delayed release formulation indicates release of the active from the formulation does not begin within a relatively shorter period of time after administration, but instead is delayed and begins or is triggered after a relatively longer period of time post administration, e.g., more than one hour.

A rapid release formulation and a slow release formulation indicate the rate of release after onset. Once delivery of the active begins, the active may be released relatively rapidly or relatively slowly. A rapid release indicates that, after onset, a maximum or peak dose is reached in a relatively shorter period of time. A slow release indicates that, after onset, a maximum or peak dose is reached in a relatively longer period of time. Once reached, the maximum dose may fall off at any rate, e.g. fast, slow, or constant.

A sustained release formulation and a continuous release formulation indicate the period of on-going release, and means that the delivery of active continues or is sustained for an extended period of time after initial onset, typically more than one hour, whatever the shape of the dose release profile. For example, the release of active is sustained between a maximum and minimum value of more than zero for some relatively longer period of time. This release may be at a constant dose, or at a dose that diminishes over time.

A constant release formulation indicates the dose that is being released. A constant release means that an active is delivered at a relatively constant dose over a moderate or extended period of time. This can be represented by a dose release profile that is relatively flat or only gently sloped after initial onset, i.e. without highly distinct peaks and valleys. Thus, a constant release is typically sustained or continuous, but a sustained or continuous release may not be constant.

A pulsed release formulation indicates that an active is delivered in one or more doses that fluctuate between a maximum dose and a minimum dose over a period of time. This can be represented by a dose release profile having one or more distinct peaks or valleys. However, two or more pulsed releases may produce an overlapping, overall, or composite release profile that appears to be or effectively is constant. When two or more pulsed releases occur, there may or may not be a period of no release between pulses. Typically, pulsed release results in release of essentially all of an active within about 60 minutes or less.

An extended release formulation provides either a release of active within a targeted dose range for a relatively longer period, or a plasma level of drug within a targeted dose range for a relatively longer period, without regard for the particular mechanism or character of release, e.g. as sustained, pulsed, or constant.

A release profile for an orally administered drug indicates the manner and timing by which a formulation releases or delivers the active to the stomach, intestines, etc. upon administration. Various methods are known to evaluate drug release and produce release profiles, including *in vitro* tests that model *in vivo* behavior of a formulation and that include USP dissolution testing for immediate release and controlled release solid dosage forms.

Drug release profiles are distinct from plasma profiles. A plasma profile indicates the dose or level of active in the bloodstream of a mammal, e.g. a patient receiving a drug formulation. When an active is released from a formulation, e.g. into the gut, the amount of active present in the bloodstream over time can be determined.

A drug release profile may be designed to produce a desired or targeted plasma profile, and a plasma profile may mimic a release profile. For example, while a sustained release of active would be expected to produce a sustained dose in the plasma, and a pulsed release would be expected to produce a pulsed (peak and valley) plasma profile, this is not necessarily the case. The half-life (T_{1/2}) of the active in the blood stream (its rate of decay) may be such that a sustained or continuous plasma profile could result from a pulsed delivery profile. Other factors may also play a role, such as bioabsorption, bioavailability, and first pass effect. The plasma profile produced by a particular active release profile may also vary from patient to patient.

Measures of bioavailability are known in the art and include the area under the plasma concentration-time curve (AUC), the concentration maximum (Cₘₐₓ), and the time to Cₘₐₓ (Tₘₐₓ).

AUC measures the area under a plasma concentration-time curve, and represents the amount of drug absorbed following administration of a single dose of a drug (Remington: The Science and Practice of Pharmacy, Gennaro Ed. 2000, p. 999).

Cₘₐₓ is the maximum plasma concentration achieved after oral drug administration (Remington, page 999). An oral drug administration results in one Cₘₐₓ, but may result in more than one peak plasma concentration, e.g., following administration of a pulsed dose formulation.

Tₘₐₓ is the amount of time necessary to achieve the Cₘₐₓ after oral drug administration, and is related to the rate of absorption of the active (Remington p. 999).

A controlled-release coating encompasses coatings that delay release, sustain release, prevent release, and/or otherwise prolong the release of a drug from a particle coated with a controlled-release coating. The term controlled-release encompasses sustained-release, delayed release, and timed, pulsatile release. Thus a controlled-release coating encompasses a sustained release coating, timed, pulsatile release coating, or lag-time coating.

An enteric polymer refers to a pH sensitive polymer that is resistant to gastric juice (i.e., relatively insoluble at the low pH levels found in the stomach), and which dissolves at the higher pH levels found in the intestinal tract.

Immediate release, in reference to a pharmaceutical composition that can be a dosage form or a component of a dosage form, refers to a pharmaceutical composition that releases greater than or equal to about 50% of the active, in another embodiment greater than about 75% of the active, in another embodiment greater than about 90% of the active, and in other embodiments greater than about 95% of the active within about one hour following administration of the dosage form. The term can also refer to pharmaceutical compositions in which the relatively rapid release of active occurs after a lag time in which little or no release of active occurs.

An immediate release (IR) bead or immediate release particle broadly refers to a bead or particle containing active that exhibits immediate release properties with respect to the active.

A sustained release (SR) bead or sustained release particle broadly refers to a bead or particle containing a SR coating disposed over an active-containing core.

A lag-time coating or timed, pulsatile release coating (TPR) refers to a controlled-release coating combining water-insoluble and enteric polymers; a TPR coating by itself provides an immediate release pulse of the active after a predetermined lag-time. A timed, sustained release (TSR) bead with a TPR coating disposed over a barrier coating (SR coating) provides a sustained active-release profile after a predetermined lag time.

A delayed release (DR) bead or delayed release particle broadly refers to an active-containing core. A DR coating refers to a controlled-release coating comprising an enteric polymer, optionally in combination with a plasticizer.

A controlled release (CR) bead or controlled release particle broadly refers to an active-containing core having an inner SR coating optionally followed by an outer DR or TPR coating or an inner TPR coating followed by an outer DR coating.

Lag-time refers to a time period where less than about 10% of the active is released from a pharmaceutical composition after ingestion of the composition or a dosage form comprising the composition, or after exposure of the composition or dosage form comprising the composition, to simulated body fluid(s), e.g., evaluated with a USP apparatus using a two-stage dissolution medium (first 2 hours in 700 mL of 0.1N HCl at 37°C followed by dissolution testing at pH 6.8 obtained by the addition of 200 mL of a pH modifier).

Disposed over, e.g. in reference to a coating over a substrate, refers to the relative location of e.g. the coating in reference to the substrate, but does not require that the coating be in direct contact with the substrate. For example, a first coating "disposed over" a substrate can be in direct contact with the substrate, or one or more intervening materials or coatings can be interposed between the first coating and the substrate. For example, a SR coating disposed over an active-containing core can refer to a SR coating deposited directly over the active-containing core or acid crystal or acid-containing core, or can refer to a SR coating deposited onto a protective seal coating deposited on the active-containing core.

A sealant layer or protective seal coating refers to a protective membrane disposed over an active-containing core particle or a functional polymer coating, protecting the particle from abrasion and attrition during handling, and/or minimizing static during processing.

An orally disintegrating tablet or ODT refers to a tablet that disintegrates rapidly in the oral cavity after administration without chewing. See, e.g., FIG. 17. The disintegration rate can vary, but is faster than the disintegration rate of conventional solid dosage forms (e.g., tablets or capsules) that are intended to be swallowed immediately after administration, or faster than the disintegration rate of chewable solid dosage forms, when tested e.g. the USP <701> test method

The term substantially disintegrates refers to a level of disintegration amounting to disintegration of at least about 50%, at least about 60%, at least about 70%, at least about 80%, at least about 90%, or about 100% disintegration. Disintegration is distinguished from dissolution; disintegration refers to the breaking up of or loss of structural cohesion of, e.g., the constituent particles comprising a tablet, whereas dissolution refers to the solubilization of a solid in a liquid, e.g., the solubilization of a drug in solvents or gastric fluids.

A water-insoluble polymer is a polymer that is insoluble or very sparingly soluble in aqueous media, independent of pH, or over a broad pH range (e.g., pH 0 to pH 14). A polymer that swells but does not dissolve in aqueous media can be water-insoluble.

A water-soluble polymer is a polymer that is soluble, i.e., a significant amount dissolves, in aqueous media, independent of pH.

An enteric polymer is a polymer that is soluble, i.e., a significant amount dissolves, under intestinal conditions; i.e., in aqueous media under neutral to alkaline conditions and insoluble under acidic conditions (i.e., low pH).

A reverse enteric polymer or gastro-soluble polymer refers to a polymer that is soluble under acidic conditions and insoluble under neutral and alkaline conditions.

Unless stated otherwise, the amount of the various coatings or layers (the coating weight) is expressed as the percentage weight gain of the particles or beads provided by the dried coating, relative to the initial weight of the particles or beads prior to coating; e.g., 10% coating weight refers to a dried coating that increases the weight of a particle by 10%.

Bioequivalence is the absence of a significantly different rate and extent of absorption in the availability of the active ingredient when administered at the same dose under similar conditions. Bioequivalence can be measured by pharmacokinetic parameters, e.g., AUC and Cₘₐₓ.

One embodiment is an oral phloroglucinol formulation that contains a modified release formulation (MR). In this embodiment, a single dosage form contains both an immediate release (IR) dosage form and an extended release (XR) dosage form. As used herein, an immediate release dosage form releases active immediately upon administration. As used herein, an extended release dosage form encompasses delayed release, time release, controlled release, or sustained release forms. As used herein, an extended release dosage form releases active at a predetermined rate over time in order to maintain a constant drug concentration for a specific period of time with minimum side effects. Extended release formulations may be achieved by a variety of formulations as subsequently described with illustrative but not limiting examples, including polymer conjugates with the active and liposome formulations of the active.

The delivery system may comprise a core, seed, or matrix that may or may not be loaded with active, and one or more coating layers comprising active and/or comprising a layer having release characteristics that controls the onset and release characteristics of the active. The core, seed, or matrix may be prepared or obtained commercially. As only one example, there may be a sugar or microcrystalline cellulose core, with a hydrophilic matrix made from, e.g., hydroxypropyl methylcellulose (HPMC), hydroxypropyl cellulose (HPC), poly(ethylene oxide), poly(vinyl alcohol), xanthan gum, carbomer, carrageenan, zooglan, etc.

Coating layers can provide immediate release, delayed pulsed release, or sustained release. Immediate release of the active from the immediate-release layer can be by, e.g., using a very thin layer or coating that gastric fluids can quickly penetrate, facilitating rapid leaching of the active; or incorporating the active in a mixture that includes a supporting binder or other inert material that readily dissolves and release active in gastric fluid; or using a supporting binder or other inert material that rapidly disintegrates upon contact with gastric fluid, with both the material and the active quickly dispersing into gastric fluid as small particles. Such rapidly disintegrating and dispersing materials include, e.g., lactose and microcrystalline cellulose. Hydroxypropyl methylcellulose is an example of a suspending agent and binder.

Enteric coatings for the delayed pulsed release component can be pH-dependent or pH-independent. Enteric coatings for the sustained release component are pH dependent. A pH dependent coating is activated to release drug within a known pH range, which typically is matched to the local pH of the environment where delayed release is desired. Exemplary pH dependent coatings include cellulose acetate phthalate, cellulose acetate trimellitate, hydroxypropyl methylcellulose phthalate, polyvinyl acetate phthalate, carboxymethylethylcellulose, copolymerized methacrylic acid/methacrylic acid methyl esters such as, e.g., materials known under the trade name EUDRAGIT^{®} L12.5, L100, or EUDRAGIT^{®} S12.5, S100 or similar compounds used to obtain enteric coatings. Aqueous colloidal polymer dispersions or re-dispersions can be also applied, e.g. EUDRAGIT^{®} L 30D-55, EUDRAGIT^{®} L100-55, EUDRAGIT^{®} S100, EUDRAGIT^{®} preparation 4110D (Rohm Pharma); AQUATERIC^{®}, AQUACOAT^{®} CPD 30 (FMC); KOLLICOAT MAE^{®} 30D and. 30DP (BASF); EASTACRYL^{®} 30D (Eastman Chemical).

A pH independent coating includes materials susceptible to enzymatic activation by azo-reductases in intestinal bacteria (i.e., azo-polymers) or materials susceptible to degradation by polysaccaridases in the colon (natural polysaccharides). Non-limiting examples of azo-polymers include co-polymers of 2-hydroxyethyl methacrylate (HEMA) and methyl methacrylate (MMA). Non-limiting examples of natural polysaccharides include amylose, chitosan, chondroitin, dextran, and xylan.

The sustained release component can include sustained release coatings, sustained release matrices, and sustained release osmotic systems. Sustained release coatings can be prepared using a water-insoluble polymer, a combination of water-insoluble polymers, or a combination water-insoluble and water-soluble polymers. Conventional sustained release polymers are known to those of ordinary skill in the art can be used for the sustained release matrix.

Exemplary sustained release coatings include polyvinyl acetate, cellulose acetate, cellulose acetate butyrate, cellulose acetate propionate, ethyl cellulose, fatty acids and esters thereof, alkyl alcohols, waxes, zein (prolamine from corn), and aqueous polymeric dispersions such as EUDRAGIT^{®} RS and RL30D, EUDRAGIT^{®} NE30D, AQUACOAT^{®}, SURELEASE^{®}, KOLLICOAT^{®} SR30D, and cellulose acetate latex.

Pellets or beads can be made of any pharmaceutically acceptable materials, based on compatibility with the active and the physicochemical properties of the pellets or beads.

Binders include cellulose derivatives such as methylcellulose, hydroxyethyl cellulose, hydroxypropyl cellulose, hydroxypropyl methylcellulose, polyvinylpyrrolidone, polyvinylpyrrolidone/vinyl acetate copolymer, etc.

Disintegration agents include corn starch, pregelatinized starch, cross-linked carboxymethylcellulose (AC-DI-SOL^{®}), sodium starch glycolate (EXPLOTAB^{®}), cross-linked polyvinylpyrrolidone (PLASDONE XL^{®}), etc.

Filling agents include lactose, calcium carbonate, calcium phosphate, calcium sulfate, microcrystalline cellulose, dextran, starches, sucrose, xylitol, lactitol, mannitol, sorbitol, sodium chloride, polyethylene glycol, etc.

Surfactants include sodium lauryl sulfate, sorbitan monooleate, polyoxyethylene sorbitan monooleate, bile salts, glyceryl monostearate, PLURONIC^{®} line (BASF), etc.

Solubilizers include citric acid, succinic acid, fumaric acid, malic acid, tartaric acid, maleic acid, glutaric acid, sodium bicarbonate, sodium carbonate, etc.

Stabilizers include antioxidation agents, buffers, acids, etc. Examples of antioxidants may include, but are not limited to, ascorbic acid, ascorbyl palmitate, butylated hydroxyanisole (BHA), butylated hydroxytoluene (BHT), diethylenetriaminepentaacetic acid (DTPA), edetates (EDTA), monothioglycerol, sodium ascorbate, sodium formaldehyde sulfoxylate, sodium metabisulfite, sodium bisulfite, triglycoamate, vitamin E or a derivative thereof, propyl gallate, combinations thereof, or the like.

The following information illustrates exemplary but non-limiting manufacturing methods.

The core may be prepared by extrusion-spheronization, high-shear granulation, solution or suspension layering,

In extrusion-spheronization, the active and other additives are granulated by adding a binder solution. The wet mass is passed through an extruder equipped with a certain size screen. The extrudates are spheronized in a marumerizer. The resulting pellets are dried and sieved.

In high-shear granulation, the active and other additives are dry-mixed, then the mixture is wetted by adding a binder solution in a high shear-granulator/mixer. The granules are kneaded after wetting by the combined actions of mixing and milling. The resulting granules or pellets are dried and sieved.

In solution or suspension layering, a drug solution or dispersion with or without a binder is sprayed onto starting seeds with a certain particle size in a fluid bed processor or other suitable equipment, thus coating the active on the surface of the starting seeds. The active-loaded pellets are dried.

Core particles have a diameter ranging from about 50 microns -1500 microns; preferably 100 microns - 800 microns. The core particles may be coated in a fluidized bed apparatus with an alternating sequence of coating layers. The core may be coated directly with a layer or layers of the active, and/or the active may be incorporated into the core material. A separation or protective layer may be added on top of the active containing layer, and/or between active layers. A separation or protective layer may be added onto the surface of the active-loaded core, and then the enteric delayed pulsed or sustained release layer may be coated thereupon. Another active layer may also be added to the enteric delayed pulsed or sustained layer to deliver an initial dose. A protective coating layer may be applied immediately outside either an active-containing core or an active-layered core, by conventional coating techniques used in the art, such as pan coating or fluid bed coating, using solutions of polymers in water or suitable organic solvents, or aqueous polymer dispersions. Suitable materials for the protective layer include cellulose derivatives such as hydroxyethyl cellulose, hydroxypropyl cellulose, hydroxypropyl methylcellulose, polyvinylpyrrolidone, polyvinylpyrrolidone/vinyl acetate copolymer, ethyl cellulose aqueous dispersions (AQUACOAT^{®}, SURELEASE^{®}), EUDRAGIT^{®} RL 30D, OPADRY^{®}, cellulose acetate, cellulose acetate butyrate, cellulose acetate propionate, ethyl cellulose, fatty acids and their esters, waxes, zein, and aqueous polymer dispersions such as EUDRAGIT^{®} RS and RL 30D, EUDRAGIT^{®} NE 30D, AQUACOAT^{®}, SURELEASE^{®}, and/or cellulose acetate latex, alone or combined with hydrophilic polymers such as hydroxyethyl cellulose, hydroxypropyl cellulose (KLUCEL^{®}, Hercules Corp.), hydroxypropyl methylcellulose (METHOCEL^{®}, Dow Chemical Corp.), polyvinylpyrrolidone, etc. Coating levels range from about 1% w/w to about 6% w/w, preferably about 2% w/w to about 4% w/w.

The enteric delayed pulsed release or sustained release coating layer is applied to the core, with or without seal coating, by conventional coating techniques known in the art, e.g., pan coating or fluid bed coating, using solutions of polymers in water or suitable organic solvents, or using aqueous polymer dispersions. Suitable coaters are known in the art, e.g., commercially available pH-sensitive polymers so that the active is not released in the acidic stomach environment (pH < 4.5), but is released and become available when the pH-sensitive layer dissolves at a higher pH, after a certain delayed time, or after the unit passes through the stomach.

Enteric polymers for the delayed pulsed release component and sustained release component include, e.g., cellulose acetate phthalate, cellulose acetate trimellitate, hydroxypropyl methylcellulose phthalate, polyvinyl acetate phthalate, carboxymethylethylcellulose, copolymerized methacrylic acid/methacrylic acid methyl esters such as, e.g., materials known under the trade name EUDRAGIT^{®} L12.5, L100, or EUDRAGIT^{®} S12.5, S100 or similar compounds used to obtain enteric coatings. Aqueous colloidal polymer dispersions or re-dispersions can be also applied, e.g. EUDRAGIT^{®} L 30D-55, EUDRAGIT^{®} L100-55, EUDRAGIT^{®} S100, EUDRAGIT^{®} preparation 4110D (Rohm Pharma); AQUATERIC^{®}, AQUACOAT^{®} CPD 30 (FMC); KOLLICOAT MAE^{®} 30D and. 30DP (BASF); EASTACRYL^{®} 30D (Eastman Chemical).

The enteric delayed pulsed release and sustained release polymers can be modified by mixing with other known coating products that are not pH sensitive, e.g., neutral methacrylic acid esters with a small portion of trimethylammonioethyl methacrylate chloride commercially available as EUDRAGIT^{®} RS and EUDRAGIT^{®} RL; a neutral ester dispersion without any functional groups commercially available as EUDRAGIT^{®} NE30D; and other pH independent coating products.

The modifying component of the protective layer used over the enteric delayed pulsed release or sustained release coating can include a water penetration barrier layer (semipermeable polymer) that can be successively coated after the enteric coating to reduce the water penetration rate through the enteric coating layer and thus increase the lag time of the active release. Coating is performed as previously described.

A protective or colorant overcoating layer can optionally be applied. OPADRY^{®}, OPADRY II^{®} (Colorcon) and corresponding color and colorless grades from Colorcon can protect the pellets from being tacky and provide colors to the product. In one embodiment the protectant or color coating ranges from 1% w/w/ to 6% w/w, preferably about 2% w/w to about 3% w/w. Talc can also be used.

Components may be incorporated into the overcoating formula, e.g., to facilitate and provide even more rapid release. Such components include, e.g., plasticizers including acetyltriethyl citrate, triethyl citrate, acetyltributyl citrate, dibutylsebacate, triacetin, polyethylene glycols, propylene glycol, etc.; lubricants including talc, colloidal silica dioxide, magnesium stearate, calcium stearate, titanium dioxide, magnesium silicate, etc.

The composition may be incorporated into hard gelatin capsules, either alone or with additional excipients. The composition may be incorporated into a tablet, e.g., by incorporation into a tablet matrix that rapidly disperses the particles after ingestion. To prevent particle destruction during the tableting process, a filler/binder is required, e.g., microcrystalline cellulose (AVICEL^{®}), soy polysaccharide (EMCOSOY^{®}), pre-gelatinized starches (STARCH^{®} 1500, NATIONAL^{®} 1551), and polyethylene glycols (CARBOWAX^{®}), present in the range of about 5% w/w to about 75% w/w, with a preferred range of about 25% w/w to about 50% w/w.

Excipients typically include, but are not limited to, one or more inert fillers including microcrystalline cellulose, soy polysaccharides, calcium phosphate dihydrate, calcium sulfate, lactose, sucrose, sorbitol, etc.; one or more materials that impart flow to powders including fumed silicon dioxide, silica gel, magnesium stearate, calcium stearate, etc.; one or more lubricants to insure proper tableting including polyethylene glycol, leucine, glyceryl behenate, magnesium stearate, calcium stearate, stearic acid, hydrogenated vegetable oil, etc. present in the range of about 0.1% w/w to about 10% w/w, with a preferred range of about 0.3% w/w to about 3.0% w/w.

Disintegrants are added to disperse the beads once the tablet is ingested. Disintegrants include, but are not limited to, cross-linked sodium carboxymethyl cellulose (AC-DI-SOL^{®}), sodium starch glycolate (EXPLOTAB^{®}, PRIMOJEL^{®}), cross-linked polyvinylpolypyrrolidone (Plasone-XL), etc., present in the range of about 3% w/w to about 15% w/w, with a preferred range of about 5%w/w to about 10% w/w.

In one embodiment, tablets are formed from particles that are introduced into a blender with AVICEL^{®}, disintegrants, and lubricant, mixed for a defined time (minutes) to achieve a homogeneous blend, then the blend is placed in the hopper of a tablet press with which tablets are compressed. The compression force used is adequate to form a tablet but not to fracture the beads or coatings.

A tablet can be constructed in three layers, where the immediate release component is dry blended, and the delayed pulsed release and the sustained release components are wet granulated. The tablet is then formed in a one layer or a three layer compression. Upon dissolution of layers, each component is released and acts as formulated: e.g., the immediate release particles provide immediate release, the delayed pulsed release particles provide delayed pulsed release, and the sustained release particles provide sustained release after a lag time.

The polymeric film coating can be applied to the active particles in any suitable manner. In one embodiment, the polymeric film is applied as a uniform coating having a smooth surface structure and a relatively constant thickness, e.g. using pneumatic spray guns. The pneumatic spray guns may have a nozzle diameter of from about 0.8 mm to about 2 mm, and may be operated at an air pressure of from about 0.5 to about 3 bar. The spraying rate can be regulated using peristaltic pumps or pressure vessels. Spraying may be continuous with simultaneous drying, so that the particles do not become too moist (over wet) and agglomerate. Fluidized-bed processes are suitable for coating small particles, e.g., AEROMATIC^{™}, GLATT^{®} with WURSTER HS^{™} Column, operate in closed cylindrical apparatuses into which an air stream is introduced from below to fluidize the particles and dry the films during spraying. Modified coating drums (usually cylindrical horizontally rotating units with a perforated wall) can be used to coat small particles.

The particles having a polymeric controlled release coating can be further manufactured into various types of oral dosage forms. As one example, the release coated particles can be compressed, either alone or in combination with excipients, adjuvants and/or other active ingredients, into pills, tablets, etc. As another example, the release coated particles can be loaded into either soft gelatin capsules or hard gelatin capsules. As another example, the release coated particles can be packaged into a pouch with other active or inactive ingredients, and dispersed into water in the form of a suspension.

The coating composition may include minor amounts of emulsifiers, wetting agents, and stabilizers such as isononylphenylpolyoxethylene glycol ethers. Minor amounts of talc can also be incorporated into the coating composition, or can be subsequently applied to improve or enhance the flow properties of the coated particles.

Suitable coating thicknesses can range from about 2 micrometers to about 15 micrometers, depending on the desired diffusion properties. The weight of the coating is generally between 2 and 15% of the weight of the phloroglucinol particles.

In another embodiment, the release coated particles can be combined with uncoated particles to provide an orally administrable pharmaceutical formulation having both immediate-release and sustained-release components. The uncoated particles may generally have substantially the same characteristics as the release coated particles prior to coating. As with the release coated particles, the uncoated particles may contain minor amounts of excipients, adjuvants and/or other active ingredients.

Other release-coatings can be used, including soluble, insoluble, permeable, impermeable or bio-degradable coatings in place of water-insoluble, water-permeable, and water-swellable polymer coatings. The polymer coating can be comprised of one or more polymers, including copolymers, terpolymers and other polymers having three or more different monomeric units. The polymers may include natural or synthetic polymers. Natural polymers that are used in sustained-release coatings include polypeptides, polysaccharides and alginic acid. Synthetic polymers include aqueous cellulose, hydroxyacyl cellulose, cellulose ether, cellulose esters, nitrocellulose, polymers of acrylic and methacrylic acids and esters, polyamides, polycarbonates, polyalkylenes, polyalkylene glycol, polyalkylene oxides, polyalkylene terephthalates, polyvinyl alcohols, polyvinyl ethers, polyvinyl esters, polyvinyl halides, polyvinyl pyrrolidone, polyglycolides, polysiloxanes and polyurethanes and copolymers thereof

Specific polymers for use in sustained-release coating of a combined immediate-release/sustained-release formulation include methyl cellulose, ethyl cellulose, hydroxypropyl cellulose, hydroxypropylmethyl cellulose, hydroxybutylmethyl cellulose, cellulose acetate, cellulose propionate (lower, medium or higher molecular weight), cellulose acetate propionate, cellulose acetate butyrate, cellulose acetate phthalate, carboxymethyl cellulose, cellulose triacetate, cellulose sulphate sodium salt, polymethylmethacrylate, polyethylmethacrylate, polypropylmethacrylate, polybutylmethacrylate, polyisobutylmethacrylate, polyhexomethacrylate, polyisodecylmethacrylate, poly(lauryl methacrylate), poly(phenyl methacrylate), polymethalacrylate, polyisopropylacrylate, polyisobutylacrylate, polyoctadecylacrylate, polyethylene (low or high density), polypropolyne, polyethylene glycol, polyethylene oxide, polyethylene terephthalate, polyvinyl alcohol, polyvinyl isobutyl ether, polyvinyl acetate, polyvinyl chloride, and polyvinyl pyrrolidone. Examples of suitable copolymers include butylmethacrylate-/isobutylmethacrylate copolymer, high molecular weight, methylvinyl ether/maleic acid copolymer, methylvinyl ether/maleic acid, monoethyl ester copolymer, methylvinyl ether/maleic and anhydride copolymer and vinyl alcohol/vinyl acetate copolymer. Examples of suitable biodegradable polymers include polylactides, polyglycolides, polyethylene terathatic and polyurethane. Examples of suitable acrylate and methacrylate are polyacrylic and methacrylic polymers such as those sold under the trademark EUDRAGIT^{®}.

The combination immediate-release/sustained-release formulation can be comprised of substantially any amount of active in immediate-release form which is effective and non-toxic, and any amount of active in sustained-release form which is therapeutically effective and non-toxic over the sustained-release period when used in combination with the selected quantity of immediate-release active.

The uncoated and coated particles can be combined in various oral pharmaceutical dosage forms or formulations, e.g. capsules, tablets, pouches, etc. The sustained-release coated particles and the uncoated particles can be combined with various excipients, adjuvants, etc. and/or other actives.

In one embodiment, particles of the active that are coated with a water-insoluble, water-permeable, and slightly water-swellable polymeric coating provides diffusion controlled sustained-release of the active at a highly reproducible, predictable rate. This rate is independent of inter- and intra-subject physiological variations such as pH. The particles can be combined with an uncoated active that can be the same as or different from the sustained-release coated active. The resulting combined immediate-release/sustained-release formulation provides higher reproducability of drug release rates than other sustained-release dosage forms using conventional enteric sustained-release coating compositions, while providing both immediate and sustained-release of medicaments.

Any of the above embodiments can be formulated into a variety of different types of orally administrable pharmaceutical dosage forms, typical formulations are pills or tablets. Tablets preferably have a hardness of from about 11 to about 19 SCU, and most preferably a hardness of about 15 SCU. The tablets preferably have a friability of less than about 0.8% weight loss after 6 minutes.

Pharmaceutically acceptable excipients and adjuvants are used in sustained-release compositions, e.g. fillers and diluents such as lactose, sucrose, dextrose, mannitol, calcium sulfate, dicalcuim sulfate, tricalcium sulfate; starches such as rice starch and micro-crystalline cellulose; binders including acacia, tragacanth, gelatin, sucrose, pre-gelatinized starch, starch, sodium alginate, almonium calcium alginate, methylcellulose, sodium carboxymethyl cellulose, ethyl cellulose, hydroxypropylmethyl cellulose, polyvinylpyrrolidone, aluminum ciliate and polyacrylamide; disintegrants including cross-linked polyvinylpyrrolidone, starch derivatives such as carboxymethyl cellulose and cellulose derivatives; lubricants; guidance and anti-adhesive agents including metallic stearates such as magnesium stearate, talc, high melting point waxes, and colordacylica.

In one embodiment, a combination of disintegrants/binders includes cross-linked polyvinyl-pyrrolidone such as POLYPLASDONE^{®} XL (GAF) or croscarmellose sodium such as AC-DI-SOL^{®} (FMC Corporation); or micro-crystalline cellulose in combination with cross-linked polyvinylpyrrolidone or croscarmellose sodium. Disintegrants/binders are used in effective amounts that can be readily determined using known techniques.

The active may be administered in a variety of dosage forms, e.g., solid dosage forms such as tablets, coated tablets, capsules, or liquid dosage forms such as syrups or suspensions. Tablet formation can use a conventional tableting apparatus, e.g. a Manesty Rotary Press, a Stokes Rotary Press, etc., at about 15°C to about 30°C and at a pressure of about 0.4 ton to about 3.0 tons.

The tablets are desirably provided with a coating to minimize dusting during handling and in the bottle, and to improve appearance and swallowability. The examples show possible tablet coatings, and the coating may have an overcoat of carnauba wax for gloss.

A tablet may have multiple cores of active with varying dissolution properties. The initial and second doses may be administered in a single dosing step. In one embodiment the initial and second doses are administered in a single dosing step, e.g., in a single solid dosage form. Such a dosage form may be a single dosing step with an immediate release portion containing the initial dose of active, and a sustained release portion containing the second dose of active. Such a single solid dosage form may be a multilayer tablet where the first immediate release dose is one layer and the second sustained release dose is in a second layer. A single solid dosage form may also be a multiparticulate tablet where the first immediate release dose is in one portion of particulates and the second sustained release dose is in a second portion of particulates, etc.

A formulation, e.g., a tablet, can be constructed by an additive manufacturing processes, i.e., three dimensional printing, as known in the art, e.g., WO 2014/144512, U.S. Patent No. 6,471,992, and U.S. Publication Nos. 2012/0207929 and 2003-0133975 disclosing three-dimensionally printed rapidly dispersing dosage forms. In this embodiment, a rapidly dispersible solid dosage form has a three-dimensionally printed matrix comprising phloroglucinol, trimethylphloroglucinol, polymer coated phloroglucinol, and/or polymer coated trimethylphloroglucinol, and at least one excipient, with the matrix formed by depositing a printing fluid to a powder and the particles of the powder becoming bound. The matrix is porous with a defined overall bulk density, disintegration (dispersion) time in aqueous fluid, dissolution time in aqueous fluid, and moisture content. The matrix provides a balance of improved chemical stability, sufficient hardness, low friability, and extremely rapid dispersion time in a small volume of aqueous liquid.

Increasing the content of many different types of water soluble excipients in this embodiment generally results in increased hardness and increased dispersion time. However, increasing the content of glycerin increases hardness but decreases dispersion time. Thus, in one embodiment, the printing fluid comprises glycerin and at least one pharmaceutically acceptable solvent. The general method steps are as follows: (a) depositing an incremental layer of active-containing powder onto a surface, (b) depositing a sufficient amount of printing fluid containing glycerin and at least one pharmaceutically acceptable solvent onto the incremental layer to bind particles in the powder, then repeating steps (a) and (b) to form the tablet or other formulation.

Upon administration, the dosage form undergoes very rapid disintegration/dispersion of its solid matrix. The active and excipients in the matrix undergo a rapid dispersion even when placed in a small volume of aqueous fluid, such as water, saliva, juice, milk, beverage, body fluid, soda, etc. Typically, dispersion overlaps with dissolution, and the matrix has a three-dimensional shape that is dispersed within the desired time period upon contact with at least a small volume of aqueous fluid.

An incremental layer of bulk powder of predetermined thickness is spread onto a prior layer of powder, and printing fluid is applied to the incremental layer as droplets according to a predetermined saturation level, line spacing, and printing fluid flowrate to bind the particles. This two-step process is completed until a matrix is formed with the desired amount of printed incremental layers.

The disclosed formulation may also contain other therapeutic actives. The actives in addition to phloroglucinol and/or trimethylphloroglucinol may be formulated in the same dosage form, or may be formulated separately, and the other active(s) can be administered simultaneously or sequentially in any order. Doses may be in the same ranges as for each active separately or, where synergistic effects occur, one or more of the combined actives may have a lower dose.

One embodiment of the invention is an oral trimethylphloroglucinol formulation that contains, in a single dosage form, both an immediate release form and an extended release form. One embodiment of the invention is an oral phloroglucinol-trimethylphloroglucinol formulation that contains, in a single dosage form, both an immediate release form and an extended release form. In one embodiment, the immediate release form is phloroglucinol and the extended release form is trimethylphloroglucinol. In one embodiment, the immediate release form is trimethylphloroglucinol and the extended release form is phloroglucinol.

A dosage form of phloroglucinol and/or trimethylphloroglucinol that combines both an immediate release formulation of 80 mg, ranging from 10 mg to 160 mg, and an extended release formulation of 160 mg, ranging from 20 mg to 480 mg, provides agent delivery to the patient continuously over about a 12 hr period. Such a dosage formulation provides coverage for bowel and/or urinary spasm control over 12 hrs with a single patient dosage, providing patient convenience and extended therapy, e.g., a patient may beneficially experience a complete night of sleep, a complete work day, a complete leisure day, etc. without symptoms.

Embodiments of the inventive formulation include the following: 100% phloroglucinol, 100% trimethylphloroglucinol, combinations of phloroglucinol: trimethylphloroglucinol at any ratios including but not limited to 90:10, 80:20, 70:30, 60:40, 50:50, 40:60, 30:70, 20:80, or 10:90. The inventive formulation contains an immediate release (IR) portion or component of the composition, and an extended release (XR) portion or component, or combinations thereof. The immediate release portion delivers 100% of the immediate release dose in less than about hour, and the extended release portion delivers the extended release dose over a period of 12 hours. Any amount or percent of phloroglucinol and/or trimethylphloroglucinol, including either no phloroglucinol with all trimethylphloroglucinol or all phloroglucinol with no trimethylphloroglucinol may be in either the immediate release portion or extended release portion, thus the inventive formulation is not limited. In one embodiment, both phloroglucinol and trimethylphloroglucinol may be in both immediate release portion and extended release portion. In this embodiment, both phloroglucinol and trimethylphloroglucinol may be in the same formulation or in a different formulation.

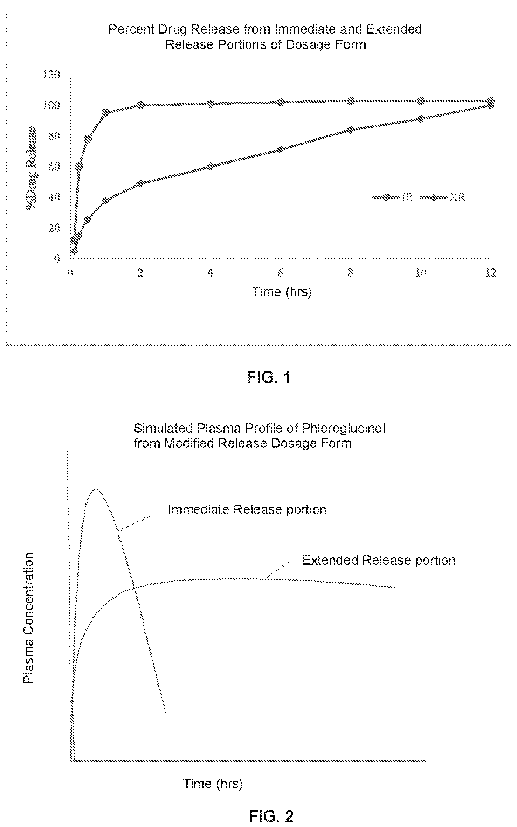

A typical dissolution profile, also termed a release profile, of phloroglucinol or trimethylphloroglucinol is shown in FIG. 1. The percent of drug release approaches 100% in less than or within one hour in the immediate release portion of the delivery system, and about 100% within or less than 12 hours for the extended release portion of the delivery system. FIG. 2 is a schematic of a simulated plasma concentration of phloroglucinol, where the plasma drug concentration from the immediate release portion peaks at about twice the concentration at the same time the drug from the extended release portion reaches a plateau, about half of that from the immediate release portion.

For management of spastic conditions of the urinary tract, including renal colicky pain, an oral dose of phloroglucinol and/or trimethylphloroglucinol may be 80 mg six times daily. For management of spastic conditions of the biliary tract with moderate abdominal pain, an oral dose of phloroglucinol and/or trimethylphloroglucinol may be 80 mg six times daily.

In one embodiment, an oral dose of 62.2 mg phloroglucinol and 80 mg trimethylphloroglucinol three times daily may be used for patients with IBS. The maximum dose is 80 mg six times a day.

In one embodiment, the active may be administered parenterally. Parenteral administration of phloroglucinol and/or trimethylphloroglucinol may be 40 mg two times daily, or 40 mg three times daily. Parenteral administration by a health care provider may be useful in a hospital setting.

In one embodiment, the active may be administered rectally. Rectal administration of the active for management of spastic conditions of the urinary tract and of the biliary tract may be 150 mg three times daily. Rectal administration may be by a suppository formulation. In one embodiment, the formulation is administered rectally, e.g., by suppository. In one embodiment, the formulation is administered parenterally, e.g., by intravenous, subcutaneous, or intramuscular injection.

The composition may take a variety of delivery forms or systems. The following formulations may be used, these are exemplary only and non-limiting. Oral formulations include a tablet, capsule, sachet, soft gel, liquid, gel, strip, film, powder, granule, pulsatile release, coated core, delayed extended release form, banded drug form, sustained release form, tablet capsule, granulation caplet, layered tablet, etc., including combinations of these, e.g., a tablet capsule, a granulation caplet, a layered tablet, etc. with active and at least one pharmaceutically acceptable excipient.

A tablet formulation is known to one skilled in the art. The tablet may be of any shape or size convenient for oral administration, e.g., circular, elliptical, etc. In one embodiment, the tablet contains 100% of either phloroglucinol or trimethylphloroglucinol or mixtures as previously described, may be either for immediate release (IR), extended release (XR), or combinations thereof. The tablet may be a bilayer tablet containing IR and XR layers adjacent to each other (FIG. 3); a trilayer tablet containing both IR and XR layers separated by a pharmaceutically acceptable buffer layer (FIG. 4); or a XR tablet containing the active in the matrix layer and coated with an IR layer of active (FIG. 5).

The composition may also be provided in other delivery forms, e.g., a capsule containing an IR tablet, a plug, and a XR tablet within an osmotic drug delivery system for controlled delivery of the composition over a duration of 12 hours (FIG. 6); a capsule containing IR beads and XR beads mixed in the appropriate ratios (FIG. 7); a capsule containing IR mini-tablets mixed with XR mini-tablets (FIG. 8); a capsule containing IR granules and XR granules that are coated with extended release polymers (FIG. 9); a capsule containing XR beads that are coated with a IR layer (FIG. 10), etc. Other delivery forms of the active may be a compressed tablet containing IR granules and coated XR beads that are embedded within the tablet (FIG. 11); a compressed tablet containing a XR tablet embedded within the IR tablet (FIG. 12); or a XR tablet suspended in an immediate release liquid drug solution within a capsule (FIG. 13).

Another delivery form is a sachet. A sachet may contain a mixture of IR and XR granules or beads (FIG. 14), or it may contain a mixture of effervescent IR granules and coated XR granules (FIG. 15).

Other immediate, extended, or sustained, modified, and delayed pulse release systems are disclosed in U.S. Publication Nos. 2005/0095295, 2005/0106247, and 2007/0264323; and U.S. Patent Nos. 6,126,969 and 8,211,465. As one example, U.S. Publication No. 2005/0106247 describes a drug (cyclobenzaprine hydrochloride) in extended release particles such as beads, pellets, granules, etc. having an extended release coating comprising a water insoluble polymer, and/or water soluble polymer, and some of the particles are contained in a gelatin capsule. As another example, U.S. Publication No. 2007/0264323 describes delivery systems for a drug (ADDERALL^{®}) such as beads within capsules, tablets, or sachets including coating layers, delayed pulsed release components, immediate release formulations, intermediate release formulations, sustained release formulations, and controlled release capsules. U.S. Patent No. 6,126,969 describes delivery systems for a drug (acetaminophen) such as a combination of coated and uncoated drug particles for an immediate-release/sustained release dosage form. U.S. Patent No. 8,211,465 describes dosage forms for an initial release of a drug (NSAID such as ibuprofen) and a second sustained release of the same drug. An osmotic delivery system is described in Patra et al. Osmotic Drug Delivery Systems: Basis and Design Approaches, Recent Patents on Drug Delivery and Formulation, 7 (2013) 1-12.

The active core of the dosage form may be an inert particle or an acidic or alkaline buffer crystal, which is coated with a drug-containing film-forming formulation. In one embodiment a water-soluble film forming composition forms a water-soluble/dispersible particle. Alternatively, the active may be prepared by granulating and milling and/or by extrusion and spheronization of a polymer composition containing the active. The amount of active in the core depends on the dose that is required, and typically varies from about 5 weight % to 60 weight %. The polymeric coating on the active core will typically be from about 4 % to 20% based on the weight of the coated particle, depending on the type of release profile required and/or the selected polymers and coating solvents. Those skilled in the art will be able to select an appropriate amount of active for coating onto or incorporating into the core to achieve the desired dosage. In one embodiment, the inactive core may be a sugar sphere or a buffer crystal or an encapsulated buffer crystal such as calcium carbonate, sodium bicarbonate, fumaric acid, tartaric acid, etc. which alters the microenvironment of the active to facilitate its release.

The drug-containing particle may be coated with an extended release (XR) coating comprising a water insoluble polymer or a combination of a water insoluble polymer and a water soluble polymer to provide XR beads. In embodiments, the water insoluble polymer and the water soluble polymer may be present at a weight ratio of from 100:0 to 65:35, or from about 95:5 to 70:30, or from about 85:15 to 75:25. The extended release coating is applied in an amount necessary to provide the desired release profile. In embodiments, the extended release coating is from about 1% to 15% by weight of the coated beads, or from about 7% to 12% by weight of the coated beads.

The modified release dosage form, including a mixture of two bead populations, may be made as follows. A drug-containing core is prepared by coating an inert particle, such as a non-pareil seed, an acidic buffer crystal or an alkaline buffer crystal with an active and a polymeric binder or by granulation and milling or by extrusion/spheronization to form an IR bead. The IR bead is coated with a plasticized water-insoluble polymer alone such as ethylcellulose or in combination with a water soluble polymer such as hydroxypropylmethylcellulose to form an XR bead. Hard gelatin capsules XR beads, alone or combined with IR beads, are filled at a desired ratio to produce modified release (MR) capsules providing the desired release profile.

IR beads using the following dissolution procedure have been reported to release at least about 70%, more specifically at least about 90%, of the active within 30 minutes.

A USP Apparatus 2 (paddles at 50 rpm) is used with the following dissolution medium: 900 mL 0.1 N HCl (or suitable dissolution medium) at 37°C, with active release determined by HPLC.

An aqueous or a pharmaceutically acceptable solvent may be used for preparing active-containing core particles. The type of film forming binder that is used to bind the drug to the inert sugar sphere is not critical but usually water soluble, alcohol soluble, or acetone/water soluble binders are used. Binders such as polyvinylpyrrolidone (PVP), polyethylene oxide, hydroxypropyl methylcellulose (HPMC), hydroxypropylcellulose (HPC), polysaccharides such as dextran, corn starch may be used at concentrations from about 0.5 weight % to about 5 weight %, with other concentrations also being used. The active may be present in this coating formulation in the solution form or may be dispersed at a solid content up to about 35 weight % depending on the viscosity of the coating formulation.

The active, optionally a binder such as PVP, a dissolution rate controlling polymer if used, and optionally other pharmaceutically acceptable excipients are blended in a planetary mixer or a high shear granulator such as FIELDER^{®} and granulated by adding/spraying a granulating fluid such as water or alcohol. The wet mass can be extruded and spheronized to produce spherical particles (beads) using an extruder/marumerizer. In these embodiments, the active load may be as high as 90% by weight based on the total weight of the extruded/spheronized core.

Illustrative but not limited examples of water insoluble polymers useful in the XR coating include ethylcellulose powder or an aqueous dispersion (e.g., AQUACOAT^{®} ECD-30), cellulose acetate, polyvinyl acetate (KOLLICOAT^{®} SR 30 D, BASF), neutral copolymers based on ethyl acrylate and methylmethacrylate, copolymers of acrylic and methacrylic acid esters with quaternary ammonium groups such as EUDRAGIT^{®} NE, RS and RS30D, RL or RL30D, etc. Illustrative but not limiting water soluble polymers include low molecular weight hydroxypropyl methylcellulose (HPMC), methylcellulose, hydroxypropylcellulose, polyvinylpyrrolidone, and/or polyethylene glycol (PEG) of molecular weight > 3000. The extended release coating is typically applied at a thickness ranging from about 1 weight % up to 15 weight % depending on the solubility of the active in water and the solvent or latex suspension based coating formulation used.

The coating compositions used in forming the membranes are usually plasticized. Illustrative but not limiting plasticizers include triacetin, tributyl citrate, triethyl citrate, acetyl tri-n-butyl citrate diethyl phthalate, polyethylene glycol, polypropylene glycol, castor oil, dibutyl sebacate, and/or acetylated monoglycerides, etc. The plasticizer may comprise about 3 weight % to about 30 weight %, more typically about 10 weight % to about 25 weight % based on the polymer. The type of plasticizer and its content depends on the polymer or polymers and nature of the coating system (e.g., aqueous or solvent based, solution or dispersion based and the total solids).

The particle may be primed by applying a thin hydroxypropyl methylcellulose (HPMC; OPADRY^{®} Clear) film before applying an extended release membrane coating to separate the different membrane layers. HPMC is typically used, but other primers such as hydroxypropylcellulose (HPC) can also be used.

The membrane coatings can be applied to the core using any coating techniques used in the pharmaceutical industry. In one embodiment, fluid bed coating is used.

Multi-dose forms may be used, i.e., products in the form of multi-particulate dosage forms (pellets, beads, granules, mini-tablets, etc.) or in other forms suitable for oral administration. As used herein, these terms are used interchangeably to refer to multi-particulate dosage forms.

An extended release dosage form that includes a mixture of two or more bead populations can be made as follows. An inert particle such as a non-pareil seed, an acidic buffer crystal, or an alkaline buffer crystal is coated with an active and a polymeric binder to form an active particle, i.e., immediate release (IR) bead, that may be in the unit dosage form to act as a bolus dose. The active particle is coated with a solution or suspension of a water insoluble polymer or a mixture of water soluble and water insoluble polymers to form an extended release coated active particle, i.e., extended release (XR). Hard gelatin capsule XR beads alone and optionally, in combination with IR beads at a ratio ranging from 95:5 to 70:30 (XR beads : IR beads), are filled to produce a modified release (MR) capsule exhibiting a target active release profile.

In one embodiment, the dosage form has an immediate release portion of active dispersed in an oily or lipid system, and another portion that is formulated in a waxy matrix or particles of active coated with hydrophobic carriers. At least 15%-50% of the active is an immediate release portion and is in a dosage form suitable for immediate release. The remainder of the tablet capsule, by weight, can include a sustained release formulation of active or a portion of the sustained release formulation of active. The active may be formulated in a lipid-based delivery system. Encapsulating or solubilizing the active in lipid excipients can lead to increased solubilization and absorption resulting in enhanced bioavailability.

Lipid excipients are commercially available. Because lipids affect absorption, it is necessary to know lipid excipient characteristics. Factors that determine the choice of excipients for lipid-based formulations include miscibility, solvent capacity, self-dispersibility and ability to promote self-dispersion of the formulation, digestibility and fate of digested products, irritancy, toxicity, purity, chemical stability, capsule compatibility, melting point, cost, etc.

Dietary oils composed of medium and long chain triglycerides, along with various solvents and surfactants, are frequently used to prepare lipid-based formulation. Many lipids are amphiphilic, i.e., they have a lipophilic portion (fatty acid) and a hydrophilic portion. The melting point increases as the fatty acid chain length increases, but the melting point decreases with an increase in the unsaturation of the fatty acid which also increases susceptibility to oxidation. Solubilizing agents used in lipid-based formulations are provided in the following table:

| **Solubilizing excipients used in commercially available lipid-based oral formulations:** | | |
|---|---|---|
| **Water-insoluble excipients** | **Triglycerides** | **Surfactants** |
| Bees wax | Long-chain triglycerides | Polysorbate 20 (TWEEN^{®} 20) |
| Oleic acid | Hydrogenated soybean oil | Polysorbate 80 (TWEEN^{®} 80) |
| Soy fatty acids | Hydrogenated vegetable oil | Sorbitanmonolaurate (SPAN^{®} 20) |
| D-*α*-tocopherol (vitamin E) | Corn oil | D-α-tocopherol PEG 1000 succinate (TPGS) |
| Corn oil mono-di-triglycerides | Olive oil | Glycerylmonooleate |
| Medium chain (C8/C10) mono and diglycerides | Soybean oil | Polyoxyl 35 castor oil diglycerides (CREMOPHOR^{®} EL), |
| Propylene glycol esters of fatty acids | Peanut oil | Polyoxyl 40 hydrogenated castor oil (CREMOPHOR^{®} RH40) |
| | Sesame oil | Polyoxy 60 hydrogenated castor oil (CREMOPHOR^{®} RH60) |
| | Medium-chain triglycerides | PEG 300 oleic glycerides (LABRAFIL^{®} M-1944CS) |
| | Caprylic/capric | PEG 300 linoleic glycerides (LABRAFIL^{®} M-2125CS) |
| | triglycerides derived from coconut oil or palm seed oil | PEG 400 caprylic/capric glycerides (LABRASOL^{®}) |
| | | PEG 1500 lauric glycerides (GELUCIRE^{®} 44/14) |

Triglyceride vegetable oils are the most common lipid excipients. They are fully digested and absorbed, eliminating safety issues. Triglycerides are long chain triglycerides (LCT), medium chain triglycerides (MCT) and short chain triglycerides (SCT). Their solvent capacity for an active is mainly due to the effective concentration of ester groups. MCT have a higher solvent capacity than LCT and are less prone to oxidation. Oils from different vegetable sources have different proportions of each fatty acid. The fatty acid composition in various lipid excipients is shown below.

| **Composition of fatty acids found in lipid-based excipients** | | |
|---|---|---|
| **(number of carbons)** | **Common name** | **Melting temperature (°C)** |
| 8 | caprylic acid | 16.5 |
| 10 | capric acid | 31.6 |
| 12 | lauric acid | 44.8 |
| 14 | myristic acid | 54.4 |
| 16 | palmitic acid | 62.9 |
| 18 | stearic acid | 70.1 |
| 18 | oleic acid | 16.0 |
| 18 | linoleic acid | -5.0 |
| 18 | *γ*-linoleic acid | -11.0 |
| 18 | ricinoleic acid | 6.0 |
| 20 | arachidic acid | 76.1 |
| 22 | behenic acid | 80.0 |

D-α-tocopherol polyethylene glycol 1000 succinate (Vitamin E TPGS) is derived from vegetable tocopherols. It is water soluble and acts as absorption enhancer for poorly water-soluble drugs. Pure triglycerides are presented in refined vegetable oils.

Mixed glycerides are obtained by partial hydrolysis of vegetable oils. The triglyceride starting material and the extent of hydrolysis determine the chemical composition of the mixed glycerides produced. Medium chain mixed glycerides are not susceptible to oxidation, have greater solvent capacity, and promote emulsification. These polar oily excipients also improve solvent capacity and the dispersibility of the formulation. Examples of polar oils include sorbitan trioleate (SPAN^{®} 85) and oleic acid.

Co-solvents, e.g., ethanol, glycerol, propylene glycol, polyethylene glycols (PEG)-400, etc. increase the solvent capacity of the formulation for actives and aid the dispersion of systems that contain a high proportion of water soluble surfactants. Practical limits related to co-solvents include precipitation of the solubilized active from the solvent due to loss of the solvent capacity following dilution, immiscibility of some co-solvents with oils, and incompatibilities of low molecular weight solvents with capsule shells.

Water insoluble surfactants are lipid excipients with intermediate hydrophilic-lipophilic balance (HLB 8-12) that adsorb at oil-water interfaces. Depending on the degree of ethoxylation, they have a finite solubility in water. They can form emulsions if subjected to shear and may be referred as being 'dispersible' in water. They can form micelles but cannot self-emulsify due to their insufficiently hydrophilic nature. Oleate esters such as polyoxyethylene (20) sorbitan trioleate (TWEEN^{®}-85) and polyoxyethylene (20) glyceryl trioleate (TAGOT^{®}-TO) exemplify water-insoluble surfactants with HLB 11-11.5. However, a blend of TWEEN^{®}-80 and SPAN^{®}-80 with average HLB of 11 is not similar to TWEEN^{®}-85 in function. A blend of TWEEN^{®}-80 and SPAN^{®}-80 has both water-soluble and water-insoluble molecules, but TWEEN^{®}-85 has predominantly water-insoluble molecules.

Water-soluble surfactants are the most common surfactants for formulating self-emulsifying drug delivery systems. Materials with HLB ≥ 12 can form micellar solutions at low concentrations by dissolving in pure water above their critical micellar concentration (CMC). Water-soluble surfactants are synthesized by PEG with hydrolyzed vegetable oils, or alternatively alcohols can be made to react with ethyleneoxide to produce alkyl ether ethoxylate, a commonly used surfactant (e.g., cetostearyl alcohol ethoxylate or CETOMACROGOL^{™}). A reaction of sorbitan esters with ethylene oxide produces polysorbates, predominantly ether ethoxylates. CREMOPHOR^{®} RH40 and RH60 (ethoxylated hydrogenated castor oil) are examples of this type, obtained from hydrogenation of materials derived from vegetable oils. CREMOPHOR^{®} EL (ethoxylated castor oil), which is not hydrogenated, is also widely used. CREMOPHOR^{®} enhances absorption by inhibiting the efflux pumps; while the inhibition mechanism is not determined it may be a non-specific conformational change due to penetration of the surfactant molecules into the membrane, adsorption on to the surface of the efflux pumps, or interaction of molecules with intracellular domains of efflux pump.

Additives may be added to protect the formulation from oxidation. Examples include lipid soluble anti-oxidants such as ascorbyl palmitate, α-tocopherol, β-carotene, propyl gallate, butylated hydroxyl toluene (BHT), butylated hydroxyanisole (BHA), etc.

Lipid behavior during formulation is assessed because lipid excipients have different chemical compositions that lead to broad melting ranges. Thermal properties of lipids, e.g., crystallization temperature, melting point, glass transition temperature, and determination of solid fat content of the excipient versus temperature, are evaluated using differential scanning calorimetry (DSC). Lipid organization during heating or cooling is assessed by hot-stage microscopy. Crystallinity of a lipid excipient is confirmed by X-ray diffraction (XRD).

High performance liquid chromatography (HPLC) and gas chromatography (GC) can determine the exact composition of ethers, esters, and fatty acid distribution. Other chemical indices include the molecular weight of fatty acids determined from their saponification value, saturation of hydrocarbon chains determined by an iodine-based assay, oxidative changes determined by measuring peroxides, free fatty acids measured from acid content, and free hydroxyl groups determined by measuring hydroxyl group content.

The FDA-required dissolution testing does not correlate to the *in vivo* behavior of lipid-based formulations. Lipids in the gastrointestinal tract are subjected to digestion processes in the presence of lipases (gastric and pancreatic) that also affect the emulsification and dispersion properties of the lipid excipients, leading to altered solubilization capacity *in vivo.* Hence, the digestibility of the lipid excipients must be considered when selecting lipid-based formulations. Dissolution testing in biorelevant media can assess such effects and predict *in vivo* behavior. The effectiveness of self-emulsifying formulations can be determined by dispersion testing (emulsification capacity and particle size). Photon correlation spectroscopy (PCS) or laser light diffraction can be used to measure the particle size, and visual observation can help predict emulsification capacity.

Lipid-based excipients enhance the oral absorption of drugs by affecting various physiological processes, e.g., stimulating bile flow and pancreatic juice secretion, prolonging gastric emptying, increasing the membrane fluidity, opening of tight junctions, promoting lymphatic transport of drugs thus avoiding first pass metabolism, and inhibiting efflux transporters. To assess these effects various *in vitro* models are available, including intestinal microsomes, Caco-2 cells, everted gut sac using chamber and *in situ* perfusion assays.

Liposomes may be used; these spherical bilayer structures resemble the cell membrane in their arrangement and are mainly amphiphilic phospholipids (hydrophilic head and hydrophobic fatty acid tail). When hydrated, these phospholipids form spherical bilayer structures, oriented with their hydrophobic tails toward the structure interior and hydrophilic heads toward the structure exterior. Hydrophilic substances can be embedded in the aqueous internal spaces of the globules, while hydrophobic active can be embedded within the inner fatty acid layers.

Solid lipid nanoparticles (SLN) may be used. SLN can enhance bioavailability along with controlled and site-specific drug delivery, so are potential carriers for oral intestinal lymphatic delivery. SLNs are typically spherical particles ranging from 10 nm to 1000 nm with a solid lipid core matrix (stabilized by surfactants) that can solubilize lipophilic molecules. Lipids mainly used include monoglycerides such as glycerol monostearate, diglycerides such as glycerol behenate, triglycerides such as tristearin, fatty acids such as stearic acid, steroids such as cholesterol, and waxes such as cetyl palmitate. Oral bioavailability of one drug was improved by formulating a N-carboxymethyl chitosan polymer that coated the drug loaded SLN using a monoglyceride lipid and soya lecithin and poloxamer 188 surfactants (Venishetty et al.)

In spray congealing, also termed spray cooling, molten lipid is sprayed into a cooling chamber and, on air contact, congeals into spherical solid particles. The solid particles are collected from the bottom of the chamber and filled into hard gelatin capsules or compressed into tablets. Ultrasonic atomizers generate solid particles in the spray cooling process. Parameters to be considered are the melting point of the excipient, the viscosity of the formulation, and the cooling air temperature inside the chamber to allow instant solidification of the droplets. Drug granules have been reported to be prepared by melt granulation using PEG 4000 or Poloxamer 188 as a meltable binder and lactose monohydrate as filler. Microparticles with narrow size distribution were reported when stearoyl polyoxyglycerides (GELUCIRE^{®} 50/13) were used as an excipient and significantly enhanced solubility of poorly water soluble drugs (Cavallari et al. Thermal and Fractal Analysis of Diclofenac/Gelucire 50/13 Microparticles Obtained by Ultrasound-Assisted Atomization, J. Pharm. Sci. 94 (2005) 1124-340).

Melt granulation, also referred to as pelletization, transforms a powder mix of active into granules or pellets. A meltable binder (molten state) is sprayed onto the powder mix in presence of high-shear mixing ('pump on' technique), or the meltable binder is blended with powder mix and melts due to the friction of particles (solid/semisolid) during high-shear mixing. The melted binder forms liquid bridges between powder particles and forms small granules that transform into spheronized pellets under controlled conditions. Depending on powder fineness, 15%-25% of the lipid-based binder can be used. Parameters to be considered during the process are binder particle size, mixing time, impellar speed, and viscosity of the binder on melting. The dissolution rate of a drug was improved by formulating melt agglomerates containing solid dispersions of drug (Seo et al.). Lactose monohydrate was melt-agglomerated with a meltable binder, e.g., PEG 3000 of GELUCIRE^{®} 50/13 in a high shear mixer. Polyoxyglycerides, partial glycerides or polysorbates, and lecithins are exemplary lipid excipients used in the melt granulation technique to form self-micro-emulsifying systems.

In embodiments, sustained release matrix tablets may be formulated using hydrophobic carriers or meltable binders such as stearic acid, carnauba wax, and bees wax, by melt granulation techniques, rendering the carriers hydrophobic for sustained delivery.

In any of the following examples, either of phloroglucinol or trimethylphloroglucinol may be used as long as one of phloroglucinol or trimethylphloroglucinol, referred to as the active or API, is present.

| **Solubility of Active (Phloroglucinol) in Various Excipients** | | |
|---|---|---|
| **No.** | **Vehicle** | **Solubility (mg/g)** |
| 1 | KOLLIPHOR^{®} RH 40 | 45.5 |
| 2 | PECEOL^{™} | 37.2 |
| 3 | IMWITOR^{®} 742 | 82.1 |
| 4 | PEG 400 | 82.7 |
| 5 | TWEEN^{®} 80 | 43.3 |
| 6 | LAUROGLYCOL^{™} 90 | 71.2 |
| 7 | Propylene Glycol | 81.8 |
| 8 | KOLLIPHOR^{®} EL | 26.3 |
| 9 | LABRAFIL^{®} M1944CS | N/A |
| 10 | Medium chain triglycerides | 6.3 |
| 11 | Oleic Acid | 0.1 |
| 12 | TWEEN^{®} 20 | 32.4 |
| 13 | LABRASOL^{®} | 82.9 |
| 14 | PLUROL^{®} Oleique | 25.3 |
| 15 | Corn Oil | 1.5 |
| 16 | Soybean Oil | 1.7 |
| 17 | CAPMUL^{®} MCM | 83.0 |
| 18 | LABRAFAC^{™} PG | 10.1 |
| 19 | MAISINE^{®} 35-1 | 30.7 |

A lipid-based formulation is one including a lipid in the excipient; exemplary lipid-based formulations are shown below.

| | **F1** | **F2** | **F3** | **F4** | **F5 (Solution)** |
|---|---|---|---|---|---|
| Phloroglucinol | 80 mg | 80 mg | 80 mg | 80 mg | 80 mg |
| PEG 400 | | | | | 820 mg |
| Soybean Oil | 45 mg | 45 mg | 45 mg | 45 mg | |
| Oleic Acid | 117 mg | 117 mg | 117 mg | 117 mg | |
| Medium chain triglycerides | 55.8 mg | 55.8 mg | 55.8 mg | 55.8 mg | |
| Canola Oil | 35.76 mg | 35.76 mg | 35.76 mg | 35.76 mg | |
| Yellow Bees wax | 9.99 mg | | | | |
| GELUCIRE^{®} 43/01 | | 9.99 mg | | | |
| COMPRITOL^{®} ATO 888 | | | 9.99 mg | | |
| LABRASOL^{®} | | | | 9.99 mg | |
| Fill weight | 343.55 mg | 343.55 mg | 343.55 mg | 343.55 mg | 900 mg |

| | **Composition** | **Amount (g)** |
|---|---|---|
| 1. | Phloroglucinol and/or trimethylphloroglucinol | 2.38 |
| | PECEOL^{™} | 16.37 |
| | CREMOPHOR^{®} RH40 | 20.77 |
| | ACCONON^{®} MC8 | 10.42 |
| | Ascorbyl Palmitate | 0.6 |
| | Total | 50.54 |
| 2. | Phloroglucinol and/or trimethylphloroglucinol | 3.33 |
| | CAPMUL^{®} MCM | 41.25 |
| | TWEEN^{®} 20 | 4.58 |
| | Ascorbyl Palmitate | 0.1 |
| | Total | 49.26 |
| 3. | Phloroglucinol and/or trimethylphloroglucinol | 3.48 |
| | CAPMUL^{®} MCM | 19.57 |
| | PEG 400 | 10.87 |
| | ACCONON^{®} MC8 | 15.22 |
| | Ascorbyl Palmitate | 0.1 |
| | Total | 49.24 |
| 4. | Phloroglucinol and/or trimethylphloroglucinol | 3.08 |
| | PEG 400 | 37.61 |
| | Polyethylene oxide 301 | 9.23 |
| | Butylated Hydroxyanisole (BHA) | 0.06 |
| | Butylated Hydroxytoluene (BHT) | 0.02 |
| | Total | 50.0 |

In any of the following embodiments, either phloroglucinol and/or trimethylphloroglucinol may be used as long as one of phloroglucinol or trimethylphloroglucinol, referred to as the active, is present.

In one embodiment, a pulsatile release form is used. The pulsatile release form includes an active core having one or more coatings, termed a coated core formulation. The coated core may also be used in combination with an amount of the active suitable for immediate release.

In one embodiment, an amount of active formulated for immediate release in combination with at least a second amount of active, either phloroglucinol and/or trimethylphloroglucinol, formulated so the second amount has a delay before onset and release of the second portion is or can be extended over time, referred to as a "delayed extended release" formulation. Each of these pulsatile release dosage formulations is further described, with all percentages by weight unless indicated otherwise.

The coated core formulation is an active core of the dosage that includes an inert particle such as a commercially available nonpareil sugar sphere. The amount of active in the core is varied depending on the desired dose to be delivered. In one embodiment, the core contains about 5% active to about 90% active. In one embodiment, the core contains about 5% active to about 60% active. The amount of active is based on the total weight of the core. Those skilled in the art will be able to select an appropriate amount of active for coating or incorporation into the core to achieve the desired dosage form. Typically, the coated core can include about 80 mg, 160 mg, up to about 480 mg active. An aqueous or a pharmaceutically acceptable solvent medium may be used for coating the core particles. Any type of pharmaceutically acceptable inert binder may be used to bind the active to the inert particle. Water soluble binders may be used. Alcohol soluble binders may be used. Binders such as polyvinylpyrrolidone (PVP), carboxyalkylcelluloses, polyethylene oxide, polysaccharides such as dextran, corn starch, hydroxypropyl methylcellulose (HPMC or hypromellose), hydroxypropylcellulose, etc. may be used by dispersing them in water at a concentration from about 0.5 weight % to 5 weight %. The active can be in this coating formulation in solution form or suspension form. The concentration of active may vary from about 0.1 weight % to about 20 weight %, depending on the viscosity of the coating formulation.

In one embodiment, the active core is prepared by granulation or by extrusion and spheronization. The active, a binder such as PVP, an optional dissolution rate controlling polymer such as high viscosity HPMC (hypromellose), and optionally other pharmaceutically acceptable excipients are blended in a high shear granulator (e.g., FIELDER^{®} granulator), or a fluid bed granulator (e.g., GLATT^{®} GPCG granulator), granulated to form agglomerates by adding/spraying a granulating fluid, such as water or alcohol, and dried. The wet mass is extruded and spheronized to produce spherical particles (beads) using an extruder. In these embodiments, the drug load may be 90% by weight based on the total weight of the extruded or granulated core.

In one embodiment, one layer of membrane coating on the particle containing the active includes a plasticized enteric polymer, and the other layer includes a mixture of a water insoluble polymer and a plasticized water dispersible/enteric polymer. The water insoluble polymer and the water dispersible polymer are present at a weight ratio of about 10:1 to 1:1, or about 4:1 to 1:1. The total weight of the coatings is about 15 weight % to 80 weight %, or about 20 weight % to about 60 weight % based on the total weight of the multiparticulate dosage form.

An intermediate acid-containing membrane is optional. If included, the intermediate acid-containing membrane may include an organic acid, e.g., fumaric acid, citric acid, succinic acid, tartaric acid, malic acid, maleic acid, etc.; and a binder, e.g., PVP. Water soluble polymers or alcohol soluble polymers are usually used. The weight of this acid-containing membrane is about 5% to about 20% based on the total weight of the coated beads. The acid in the acid-containing membrane delays dissolution of the enteric polymer in the inner layer, thereby increasing the lag time as well as decreasing the rate of release of the active from the coated bead. The composition of the outer layer of the polymeric membrane, and the individual weights of the inner, intermediate, and outer membrane layers, are further optimized to achieve pulsatile release profiles for the active based on predicted *in vitro*/*in vivo* correlations. Thus, the pulsatile release dosage formulation is optimized to release an amount of active after a predetermined time period and/or at a particular point in the digestive tract of the individual administered the formulation.

Examples of enteric polymers include, but are not limited to, the following compounds or composition, either alone or in combination: esters of cellulose and its derivatives (cellulose acetate phthalate, hydroxypropyl methylcellulose phthalate, hydroxypropyl methylcellulose acetate succinate), polyvinyl acetate phthalate, pH-sensitive methacrylic acid-methamethacrylate copolymers, and shellac. These polymers may be used as a dry powder or an aqueous dispersion. Methacrylic acid copolymers EUDRAGIT^{®} L100, S100, L30D are available (Rohm Pharma), cellulose acetate phthalate CELLACEFATE^{®} (Eastman Chemical Co.), cellulose acetate phthalate aqueous dispersion AQUATERIC^{®} (FMC Corp.), and hydroxypropyl methylcellulose acetate succinate aqueous dispersion AQOAT^{®} (Shin Etsu K.K.).

Examples of water insoluble polymers include, but are not limited to, the following compounds or composition, either alone or in combination: cellulose derivatives (e.g. ethylcellulose), polyvinyl acetate (KOLLICOAT^{®} SR 30 D, BASF), neutral copolymers based on ethyl acrylate and methylmethacrylate, copolymers of acrylic and methacrylic acid esters with quaternary ammonium groups such as EUDRAGIT^{®} NE, RS or RS30D, RL or RL30D, etc.

Membrane coatings can be applied to the core using any pharmaceutical coating method known in the art. For example, fluid bed coating may be used.

A pulsatile release dosage formulation may be prepared by (i) coating an inert particle, e.g., a non-pareil seed (sugar sphere), with the active and polymeric binder, or by preparing the particle containing the active by granulation and/or extrusion/spheronization to form an active particle; (ii) coating the active particle with a plasticized enteric coating, forming the plasticized enteric coated active particle; and (iii) coating the plasticized enteric coated active particle with a mixture of a water insoluble polymer and an enteric polymer. The release characteristics can be modulated by interchanging parts (ii) and (iii). An organic acid, as previously described, can be added to the membrane between parts (ii) and (iii) to further modulate the lag time and active release profile from the particle.

In one embodiment, the formulation may use a single form of the particulate to provide a time-controlled pulsatile release of the active several hours after oral administration, or to target to specific absorption sites. In one embodiment, dosage forms incorporating the multicoated active containing particles are combined in a composite dosage formulation with an amount of active for immediate release, e.g., in a gelatin, either hard gelatin or soft gelatin, capsule. This embodiment provides a composite dosage form having both an immediate release portion and time-controlled pulsatile release portion of active.

The optional immediate release portion and the active of the coated core can each include about 10 mg, 20 mg, 40 mg, 80 mg, etc. of active, a coated core dosage form of the present invention can contain about 10 to 800 mg of active.

In one embodiment, a delayed extended release form is used.

In one embodiment, a dosage form can provide at least a bi-modal blood profile of active, e.g., the profile shown in FIG. 2. In this embodiment, the dosage form contains at least a first amount of active for immediate release, and a second amount of active for delayed extended release. For example, a first portion of active is immediately released during the first hour after administration from the inventive dosage form. There is an elapsed time period where substantially no active is released and/or is capable of entering the circulation, and/or is bioavailable from a second portion of administered active. Then, after another elapsed time, e.g., a few hours, additional active is released, and the release of this second portion occurs over an extended period of time, e.g., up to 12 hours after initial administration or even longer. This release of the second portion typically occurs after a lag time during which no active is released, so such dosage forms that can exhibit a delay before the initiation of release of an amount of active are termed delayed extended release dosage forms. Such a dosage form can be administered alone, or it can be administered in combination with other dosage forms.

It is desirable for the blood level of active to increase, with the blood concentration corresponding to the amount of active that is bioavailable after the immediate release in the first hour after administration. After a time, blood levels of active decreases to less than desirable or therapeutic levels. The second portion of active can enter the circulation after the immediate release portion of active has been released. In embodiments, after blood levels of active begin to decrease, the formulation desirably increases and/or maintains blood levels at or above about the desired concentration without the need to administer a second dose of active.

The following example illustrates one embodiment. The first immediate-release portion of active has an initial pharmacokinetic profile. Fillers, excipients, etc. can account for the final weight percent.

Formulations for delayed sustained or extended release are as follows. Each sustained release composition includes an amount of active formulated to release the active over a period of 4 hours to 12 hours, typically 6 to 12 hours.

Polyalcohols such as mannitol, coagulants such as a POLYOX^{®}, coagulants and lubricants such as stearic acid are added to yield a granulation that can provide a delayed and extended release active formulation. Caplets, tablets, or other dosage forms of the delayed release formulation are prepared using procedures known in the art, including encapsulating procedures. Such dosage forms, without more, typically exhibit sustained release blood profiles, i.e., the dosage forms typically immediately releases active after ingestion and continues to release active over time. These compositions can also be formulated into a dosage form, and can exhibit extended release profiles, releasing active for a period of a few hours up to 12 hours after ingestion.

In one embodiment, the dosage forms formed from the compositions can be optionally base coated to seal the tablets for subsequent processing. Sealers include, e.g., HPMC, (poly)ethylene glycol (PEG), etc.

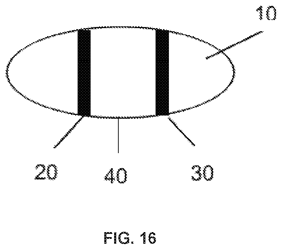

In one embodiment, a dosage form is banded with one or more bands of one or more polymeric materials, as subsequently described and shown in FIG. 16. One or more circumferential or other types of bands of polymeric material are used, e.g., a relatively insoluble polymeric material that does only minimally or does not erode or degrade during the dispensing period. Typical insoluble polymers include the water insoluble polymers previously described. The number of bands, the position or spacing between bands, and the thickness of the bands can control the rate of release of active. For example, a space of 0.5 mm, 1.0 mm, 1.5 mm, 2.0 mm, 2.5 mm, or 3.0 mm can be present between bands if multiple bands are used. For example, each band can be 0.5 mm, 1.0 mm, 1.5 mm, or 2.0 mm wide and have a thickness of about 0.1 micron to 100 micron, or 0.1 micron to 50 micron, or 0.1 micron to 20 micron. As shown in FIG. 16, in one embodiment, a caplet has two circumferential polymeric bands, each band 20 and 30 has a width of about 1 mm and a spacing 40 of about 2 mm. The banded formulation slows the release of the active and extends the period of time over which the active can be released and/or enter the circulation, i.e., to be rendered bioavailable. In embodiments, the band(s) delays the onset of release of active such that there is a lag time, also termed a delay of onset or delayed release during which no active is released. A delay of onset can be from 0 hour to 4 hours, or may be 0 hour to 3 hours, or may be 0.5 hour to 4 hours, or may be 1 hour to 2 hours after administration.

In one embodiment, the banded dosage form can be optionally coated with a suitable enteric coating known in the art, e.g., EUDRAGIT^{®} L30D-55 and PEG and/or polymers, examples of which are shown in the following table:

| **Polymer** | **Type** | **Level (%)** |
|---|---|---|
| EUDRAGIT^{®} | Acrylate-methacrylate polymers | |
| RSPO | Insoluble, High Permeability | 10 |
| RLPO | Insoluble, Low Permeability | 10, 15, 30 |
| NE30D-Suspension* | Insoluble, Permeable | 20 |
| CARBOPOL^{®} | Crosslinked polyacrylic acid polymers | |
| 971P | Light cross linking, slow release in SGF | 10, 15 |
| 934P | High cross linking, release throughout GIT | 10 |
| 974P | Rigid crosslinking, rapid drug release in SGF | 10, 20 |
| METHOCEL^{™} | Water soluble HPMC | |
| K4M* | Viscosity: 4000 millipascal-seconds | 15, 18, 30 |
| METHOCEL^{™}:AVICEL^{®} | Water soluble:Insoluble MCC | 10, 14, 14.5, 15, 16.5, 18, 30 |
| K4M | Viscosity: 4000 millipascal-seconds | 5 |
| K100M | Viscosity: 100000 millipascal-seconds | 10 |
| K15M | Viscosity: 15000 millipascal-seconds | 7, 10, 12 |
| K4M* | | |

| POLYOX^{™} | water soluble poly (ethylene oxide) polymer | |
|---|---|---|
| Coagulant | MW: 5,000,000 | 5, 8, 9, 9.5, 10, 20 |
| WSRN301 | MW: 4,000,000 | 10, 12.5, 15, 20 |
| WSRN60K | MW: 2,000,000 | 20, 30, 40 |
| KELTONE^{®} | Alginate Salt | |
| HVCR | High viscosity | 10 |
| Ethyl Cellulose | Water insoluble ethylcellulose | |
| ETHOCEL^{™} 100FP | Particle size 40 microns | 5-15 |
| KOLLIDON^{®} SR | 80% Polyvinyl acetate and 19% Povidone, Partly soluble in water | 20 |

| | | |
|---|---|---|
| * can be added by wet granulation | | |

The enteric coating may also include other excipients or fillers, e.g., talc, lactose, dicalcium phosphate, lubricants such as magnesium stearate, etc.

The banded dosage form can be coated at a level of about 2 µg/cm² to 10 µg/cm², typically about 7 µg/cm². The enteric coating delays the onset of active such that there is time during which no active is released after administration of the dosage form. Typically, after enteric coating, delay of onset of active from a coated banded dosage form (e.g., an enteric coated banded caplet) can be from 0.5 hour to 4 hours, typically 1 hour to 2 hours.

In one embodiment, an immediate release dose of active previously described is combined with an enterically coated banded caplet using methods known in the art to produce a single composite dosage form, e.g., into a single gelatin capsule. The formulation may be tailored to provide a specific desired blood profile.

In embodiments, the compositions include at least an immediate release formulation and a sustained release formulation, subsequently described below. Sustained release formulations do not typically exhibit a delayed onset of active. Sustained release formulation do not typically exhibit a significant time period during which no drug is made bioavailable from the dosage form after administration.

In one embodiment, a tablet capsule is a capsule containing a first portion of active in a tablet form that is formulated for immediate release upon ingestion or administration, and at least a second portion of active that is in a tablet form that is formulated for sustained release, i.e., the second portion continues to release an amount of active up to 6-12 hours after ingestion. At least 15%-50% of the active is an immediate release formulation and is in a tablet form and is suitable for immediate release. The remainder of the tablet capsule, by weight, can include a sustained release formulation of active or a portion of the sustained release formulation of active. The tablet containing an immediate release formulation of active and the tablet containing a sustained release formulation of active may be combined in a single dosage form, e.g. a gelatin capsule, using methods known in the art.

In one embodiment, a granulation caplet is a capsule or caplet containing a first portion of a granulation of active that is formulated for immediate release, and at least a second portion of active that is in tablet form that is formulated for sustained release. At least 15%-50% of active is an immediate release formulation and can be in granules versus a tablet. In one embodiment, at least about 80% of the granulation capsule includes a composition of active for immediate release in a granular form, typically contained in a separate caplet. The remainder of the granulation caplet, by weight, may include a sustained release formulation of active, or the granulation caplet may include a portion of the sustained release formulation of active. The caplet containing an immediate release formulation of active and the caplet containing a sustained release formulation of active may be combined in a single dosage form, e.g. a gelatin capsule, using methods known in the art.

In one embodiment, a layered tablet contains a tablet having two or more layers with the active that is formulated for immediate release, and a layer of active that is formulated for sustained release. The layered tablet contains an amount of active for immediate release upon ingestion, and at least a second portion of active that can immediately provide an amount of active for up to 6 hours - 12 hours after layered tablet ingestion. At least 15%-50% of active is an immediate release formulation. In one embodiment, at least about 80% of the layered tablet includes a composition of active for immediate release. The remainder of the layered tablet, by weight, may include a sustained release formulation of active, or may include a portion of the sustained release formulation of active. The formulations can be combined in a conventional manner, e.g. in a tablet press, so that after processing, the final tabletted dosage form has two or more layers, at least a first layer containing the immediate release formulation of active and a second layer containing the sustained release formulation of active.

In one embodiment, 100% of the active is in an immediate release dosage form and is not combined with a dosage form suitable for sustained release. The active can be dispersed in a lipid-based formulation, e.g., an oily or lipid system as described above.

In one embodiment, the active is least 20% to 30%, 30% to 60%, or 70% by weight of the sustained release composition, with the remaining weight of the composition excipients, e.g., fillers, lubricants, polymers, etc. The polymer can be present from 5% to 20% by weight of the sustained release composition in one embodiment, and from 7% to 10% by weight of the sustained release composition in one embodiment, and from 10% to 16.5% by weight of the sustained release composition in one embodiment. In one embodiment, the polymer is a cellulosic polymer, e.g. METHOCEL^{™} K4M and is present at about 10% by weight. The sustained release formulation can be prepared by direct compression or wet granulation.

The formulation may be compressed into tablets, or may be incorporated directly with food. Such compositions should contain at least 0.1% of active compound. The percentage of the compositions and preparations may vary, e.g., about 2% to about 60% of the weight of the unit.

Excipients include, but are not limited to, one or more of a pharmaceutically acceptable inert diluent; an assimilable edible carrier; a disintegrant to facilitate disintegration, e.g., modified cellulose derivatives, modified starch derivatives, etc., noting that one skilled in the art appreciates that other ingredients including binders and lubricants can also affect the dissolution profile of the dosage form; a hard or soft shell gelatin capsule; dicalcium phosphate; a binder such as gum tragacanth, acacia, corn starch, or gelatin; a disintegrating agent such as corn starch, potato starch, alginic acid, etc.; a lubricant such as magnesium stearate; a sweetening agent such as sucrose, lactose, or saccharin; a flavoring agent such as peppermint, oil of wintergreen, cherry flavoring; one or more surfactants such as ionic, non-ionic, and/or bile salt surfactants, with anionic surfactants including sodium alkyl sulfate (sodium lauryl sulfate) and sulfosuccinate derivatives such as docusate sodium, non-ionic surfactants including polyoxyethylene sorbitan fatty acid esters (polysorbates) such as TWEEN^{®} 20, TWEEN^{®} 80, TWEEN^{®} 40, SPAN^{®} 20, fatty acid esters of polyethylene glycols such as GELUCIRE^{®} 44/14, GELUCIRE^{®} 50/13, saturated polyglycolized (including mono, di or tri)glycerides, medium chain monoglycerides (6-10 carbons) such as glyceryl monocaprylate (IMWITOR^{®} 308), glyceryl monocaproate (CAPMUL^{®} MCM C-8), glyceryl caprylate/caprate (CAPMUL^{®} MCM), polyoxyethylene glyceryl caprylate, and polyoxyethylene glyceryl caproate (LABRASOL^{®}), medium chain fatty acid esters such as glyceryl tri caprate and glyceryltricarilate (MIGLYOL^{®} 612), block polymers of ethylene oxide and propylene oxide, polyoxyethylene-polyoxy propylene block copolymers such as Poloxamer 188 (PLURONIC^{®} F-68), Poloxamer 237 (PLURONIC^{®} F-87), Poloxamer 338 (PLURONIC^{®} F-108), Poloxamer 407 (PLURONIC^{®} F-127), Poloxamer 124 (PLURONIC^{®} L-44), polyoxy stearate-polyethoxylated (40) stearic acid (MYRJ^{™} 52), ethoxylated castor oil-polyethoxylated (60) hydrogenated castor oil (CREMOPHOR^{®} EL), ethoxylated hydrostearic acid polyethylene glycol 660 hydroxystearate (SOLUTOL^{®} HS 15), polyoxyethylene alkyl ethers (12-18 carbons) such as polyoxy 20 cetostearyl ether (ATLAS^{™} G-3713), polyoxy 10 oleyl ether (BRIJ^{™} 96, BRIJ^{™} 97, Oleth 10), polyethylene glycol ether (TRITON^{™} X-100, TRITON^{™} X-114, TRITON^{™} X-405, TRITON^{™} N-101) and lecithins such as phospholipids (dimyristoyl DL-alpha-phophatidylcholine), bile salt surfactants including deoxycholic acid, sodium deoxycholate, cholic acid, sodium taurocholate; etc. A capsule dosage form may also contain a liquid carrier. Other materials may be present as coatings or to otherwise modify the physical form of the dosage form, e.g., tablets, pills, or capsules may be coated with shellac and/or sugar. A syrup or elixir may contain the active, sucrose as a sweetening agent, methyl and propylparabens as preservatives, a dye, and a flavoring agent.

In embodiments, other actives may be included in the formulation.

In one embodiment the dosage forms are a liquid filled soft gel capsule containing excipients that have lipids, surfactants and solvents. The capsules may contain formulations for immediate release, delayed release, sustained release, or controlled release.

The formulation may contain excipients such as one or more fatty acids. The method involves dissolving, melting, or suspending a poorly water soluble active agent in one or more fatty acids, conjugated fatty acids, (semi-) solid surfactants having a high HLB value, and/or hydrophilic polymers. Suitable fatty acids include C₁₀-C₁₈ fatty acids, preferably C₁₆-C₁₈ fatty acids. Suitable conjugated fatty acids include C₁₀-C₁₈ fatty acids, preferably C₁₆-C₁₈ fatty acids, conjugated with glycerol (e.g., monoglycerides), monosaccharides, and/or polyethylene glycol (PEG). Suitable hydrophilic polymers include poloxomers and poloxamines.

Suitable fatty acids include C₁₀-C₁₈ fatty acids, more preferably C₁₆-C₁₈ fatty acids. Exemplary fatty acids include, but are not limited to, dodecanoic (lauric) acid, tetradecanoic (myristic) acid, hexadecanoic (palmitic) acid, heptadecanoic (margaric) acid, octadecanoic (stearic) acid, eicosanoic (arachidic) acid, docosanoic (behenic) acid, tetracosanoic (lignoceric) acid, hexacosanoic (cerotic) acid, heptacosanoic (carboceric) acid, octacosanoic (montanic) acid, triacontanoic (melissic) acid, dotriacontanoic (lacceroic) acid, tritriacontanoic (ceromelissic) acid, tetratriacontanoic (geddic) acid, and pentatriacontanoic (ceroplastic) acid. The fatty acids can be saturated fatty acids, monounsaturated fatty acids, polyunsaturated fatty acid, or combinations thereof.

Oils, for example, vegetable oils, such as soybean oil can be used alone or in combination with the coating materials listed above. Soybean oil contains 14.4% saturated fatty acids, 23.3% monounsaturated fatty acids, such as oleic acid, and 57.9% polyunsaturated fatty acids, such as linoleic acid and alpha linoleic acid.

In one embodiment, the fatty acid is covalently coupled to glycerol, a monosaccharide, such as sorbitol or sorbitan, a polyalkylene oxide, such as polyethylene glycol and polypropylene glycol, or combinations thereof. These materials are referred to as conjugated fatty acids. Suitable conjugated fatty acids include, but are not limited to, polyethylene glycol esters of fatty acids, such as those available commercially under the tradename GELUCIRE^{®}, sorbitan esters of fatty acids, such as sorbitan monostearate, glycerol fatty acid esters of the fatty acids listed above, such as glycerol behenate and glyceryl monostearate, and combinations thereof.

The concentration range of the fatty acid is from about 1 to about 20% by weight of the composition, preferably from about 5 to about 15% by weight of the composition (microparticles and carrier).

The water-insoluble active agent can be coated with one or more surfactants, alone or in combination with or more fatty acids or conjugated fatty acids and/or one or more hydrophilic polymers. In one embodiment, the surfactant has an HLB value greater than about 10, greater than about 12, greater than about 14, or greater than about 16 (on a scale of 1-18). Surfactants having the desired HLB are known in the art. The surfactant can be anionic, cationic, or non-ionic. In one embodiment, the surfactant is a non-ionic surfactant.

Examples of such surfactants include, but are not limited to, polysorbate 20, 40, and 80 (marketed under the name TWEEN^{®}), polyoxyethylene monostearate, some sugar esters, such as sucrose monolaurate, ethoxylated nonyl phenols, alpha olefin sulfonates, ethoxylated tallow amines, ethylene oxide/propylene oxide block copolymers, ethoxylated soya amines, fatty acids and alcohols, polyethoxylated castor oil, polysorbates, polyoxyethylene alkyl ethers, and polyoxyethylene stearates.

In one embodiment, the surfactant is a high HLB surfactant containing a fatty acid chain. Suitable surfactants include, but are not limited to, polyethoxylated castor oil, polysorbates, polyoxyethylene alkyl ethers, and polyoxyethylene stearates.

Polyoxyethylene castor oil derivatives contain mainly ricinoleyl glycerol ethoxylated with 30-50 molecules of ethylene oxide. Polysorbates or polyoxyethylene sorbitan fatty acid esters are a series of partial fatty acids esters of sorbitol and its anhydrides copolymerized with approximately 20, 5, or 4 moles of ethylene oxide for each mole of sorbitol and its anhydrides. The resulting product is a mixture of molecules having a wide range of molecular weights. Polyoxyethylene alkyl ethers are a series of polyoxyethylene glycol ethers of linear fatty alcohols (n-alcohols), such as lauryl, myristyl, cetyl, and stearyl alcohol. Polyoxyethylene stearates are produced by polyethoxylation of stearic acid.

Without desiring to be bound by any theory, it is believed that the hydrophilic part of the surfactant enhances the compatibility of the active agent with the aqueous dissolution media *in vitro* or *in vivo* and that the fatty acid side chain enhances absorption via fatty acid oxidation. During fatty acid oxidation, intracellular Ca²⁺ is consumed which results in the widening of gap junctions, allowing passage of the active agent between cells. Further, such coated particles may be more stable than drug alone, for example, by preventing oxidation of the active agent.

The concentration of the surfactant is from about 1 to about 50%, preferably from about 5 to about 15% by weight of the composition (microparticles and carrier).

Suitable hydrophilic polymers include, but are not limited to, poloxamers, poloxamines, polyethylene glycols, polyvinyl alcohols, polyvinylpyrrolidone, poly(vinyl alcohol), cellulosic materials, such as hydroxypropylcellulose, hydroxymethylcellulose, hydroxypropylmethyl-cellulose, gelatin, carboxymethyl cellulose, and polypeptides.

The concentration of the hydrophilic polymer is from about 1 to about 50% by weight of the composition, more preferably from about 5 to about 15% by weight of the composition. If the hydrophilic polymer is a polyethylene glycol, the concentration is from about 1 to about 80% by weight of the composition, from about 30 to about 60%, from about 35% to about 60%, or from about 40% to about 60% by weight of the composition (microparticles and carrier).

In one embodiment, the microparticles are formed by adding a mixture of the drug and coating material(s) to a pharmaceutically acceptable carrier. In one embodiment, the carrier is a hydrophilic or lipophilic carrier. The resulting particles are suspended in the carrier. The carrier may be a single component or a mixture of components. The carrier can include solvents, surfactants, or other excipients. The carrier materials can alter or modify the rate of release of the drug from the microparticles and/or the rate of dissolution of the drug. The compositions may exhibit a biphasic release profile due to the controlled release properties of the microparticles and the controlled release properties of the carrier. Varying the qualitative and quantitative composition of the carrier materials may allow one to modulate the release profile of the active agent. The carrier may contain one or more rate controlling excipients which regulate release of the active agent. Exemplary rate controlling excipients include, but are not limited to, glyceryl behenate, GELUCIRE^{®}, Cremophor, hydrogenated vegetable oil, bees wax, cellulosic polymers such as hypromellose, alginates, CARBOPOL^{®} and combinations thereof.

In one embodiment, the carrier is a hydrophilic carrier containing a surfactant having a HLB value greater than about 10, greater than about 12, greater than about 14, or greater than about 16, and/or is water soluble. Exemplary hydrophilic carriers include, but are not limited to, polyethylene glycols, polyoxyethylene 32 lauric glycerides (available from Abitech under the tradename ACCONON^{®} M-44), polyoxyethylene 8 caprylicleapric glycerides (available from Abitech under the tradename ACCONON^{®} MC-8) and glycofurol. The hydrophilic vehicle can further contain one or more miscible solvents such as glycerin, ethanol, glycofurol, and caprylocaproyl macrogol-8 (available from Gattefosse S.A., Saint Priest, France under the tradename LABRASOL^{®}).

In one embodiment, the hydrophilic carrier is water or an alcohol. In another embodiment, the carrier is a hydrophilic carrier mixture containing polyethylene glycol, and optionally one or more surfactants and/or water. In a particular embodiment, the hydrophilic carrier is a mixture of PEG 400 (e.g., 57% by weight of the composition), water (e.g., 8% by weight of the composition), and TWEEN^{®} 20 (e.g., 10% by weight of the composition). The hydrophilic carrier can also contain CREMOPHOR^{®} RH 40. The concentration of the hydrophilic carrier is generally from about 50% to about 85% by weight of the composition (microparticles and carrier), preferably from about 70 to about 80% by weight of the composition.

In another embodiment, the carrier is a lipophilic carrier. In a preferred embodiment, the lipophilic carrier has an HLB value of less than about 10 and/or is oil soluble. Exemplary lipophilic oily vehicles include, but are not limited to, vegetable oils, medium chain mono-, di-, and triglycerides, glyceryl stearates (available from Sasol under the tradename IMWITOR^{®}), polyoxyethylated oleic glycerides (available from Gattefosse, SA., Saint Priest, France, under the tradename LABRAFIL^{®}), mineral oil, mono- and diglyceride emulsifiers such as glyceryl monooleate, glyceryl monocaprate, glyceryl monocaprylate, propylene glycol monocaprylate, and propylene glycol monolaurate (available from Abitec Corp., Columbus, Ohio, under the tradename CAPMUL^{®}), and dimethylpolysiloxanes such as simethicone.

The concentration of the lipophilic carrier is generally from about 10% to about 50% by weight of the composition (microparticles and carrier), preferably from about 5 to about 35% by weight of the composition.

The compositions described can contain one or more pharmaceutically acceptable excipients that are considered safe and effective and may be administered to an individual without causing undesirable biological side effects or unwanted interactions. Exemplary additives include, but are not limited to, solvents, suspending agents, dispersants, buffers, pH modifying agents, isotonicity modifying agents, preservatives, antimicrobial agents, and combinations thereof.

Suitable additives for inclusion in the compositions described herein include, but are not limited to, antioxidants (e.g., alpha tocopherols, such as vitamin E acetate, ascorbic acid, butylated hydroxyanisole, and butylated hydroxytoluene); polar solvents (e.g., water, propylene glycol, and glycerin); hydrophobic solvents (e.g., corn oil, castor oil, soybean oil, olive oil, fish oil, peanut oil, peppermint oil, safflower oil, sesame oil, medium chain triglycerides, caprylic triglycerides, capric triglycerides derived from coconut oil or palm seed oil); and viscosity increasing agents (e.g., gelatin, glycerin, carrageenan, colloidal silicon dioxide, hydrogenated vegetable oil; povidone, and propylene glycol alginate).

The microparticle compositions described herein are generally formulated for oral or parenteral administration. Suitable oral dosage forms include capsules, such as hard or soft, gelatin or non-gelatin capsules, or oral suspensions or syrups. Suitable parenteral formulations include suspensions.

In one embodiment, the microparticle compositions (microparticles suspended in a hydrophilic or lipophilic carrier) are encapsulated in a capsule, such as a hard or soft capsule. The capsules can be prepared from natural and/or synthetic film forming polymers. Suitable natural film forming materials include, but are not limited to gelatin. Non-gelatin capsules include, but are not limited to, capsules made from carageenan, shellac, alginates, pectin, and zeins. Suitable synthetic film-forming polymers include, but are not limited to, methyl cellulose, hydroxypropyl methyl cellulose acetate succinate, hydroxypropyl methyl cellulose phthalate, cellulose acetate phthalate, and acrylates such as poly(meth)acrylate.

The compositions can also be encapsulated in an enteric capsule, wherein the capsule is coated with an enteric coating or the capsule shell contains an enteric polymer as described in WO 2004/030658 to Banner Pharmacaps, Inc.

Hard shell capsules are typically prepared by forming the two capsule halves, filling one of the halves with the fill solution, and then sealing the capsule halves together to form the finished capsule. Soft gelatin capsules are typically prepared using a rotary die encapsulation process. Such processes are known in the art.

The capsule shell can contain one or more additives. Suitable shell additives include plasticizers, opacifiers, colorants, humectants, preservatives, flavorings, and buffering salts and acids, and combinations thereof.

Plasticizers are chemical agents added to gelatin to make the material softer and more flexible. Suitable plasticizers include, but are not limited to, glycerin, sorbitol solutions which are mixtures of sorbitol and sorbitan, and other polyhydric alcohols such as propylene glycol and maltitol or combinations thereof.

Opacifiers are used to opacify the capsule shell when the encapsulated active agents are light sensitive. Suitable opacifiers include titanium dioxide, zinc oxide, calcium carbonate and combinations thereof.

Colorants can be used to for marketing and product identification/differentiation purposes. Suitable colorants include synthetic and natural dyes and combinations thereof.

Humectants can be used to suppress the water activity of the softgel. Suitable humectants include glycerin and sorbitol, which are often components of the plasticizer composition. Due to the low water activity of dried, properly stored softgels, the greatest risk from microorganisms comes from molds and yeasts. For this reason, preservatives can be incorporated into the capsule shell. Suitable preservatives include alkyl esters of p-hydroxy benzoic acid such as methyl, ethyl, propyl, butyl and heptyl esters (collectively known as "parabens") or combinations thereof.

Flavorings can be used to mask unpleasant odors and tastes of fill formulations. Suitable flavorings include synthetic and natural flavorings. The use of flavorings can be problematic due to the presence of aldehydes which can cross-link gelatin. As a result, buffering salts and acids can be used in conjunction with flavorings that contain aldehydes in order to inhibit cross-linking of the gelatin.

Medium chain triglycerides may also be used. As used herein, "medium chain triglycerides" means C6-C12 ester chains formed via the esterification of glycerol with three fatty acids. There are various sources of medium chain triglycerides, for example coconut oil, palm kernel oils, etc. Fractionated coconut oils are the most commonly used sources for medium chain triglycerides. Examples of commercially available medium chain triglycerides may include MIGLYOL^{®} 810, 812 or 881 produced by Sasol Germany GMBH, CAPTEX^{®} 300, 355, or 810D produced by the Abitec Corporation, NEOBEE^{®} M5 by the Stepan Company, CRODAMOL^{®} GTC/C produced by Croda Inc, and LABRAFAC^{®} Lipophile WL 1349 produced by the Gattesfosse Group. In one exemplary embodiment, the medium chain triglyceride may comprise CAPTEX^{®} 355, which is a triglyceride of caprylic (C8)/capric (C10) acid.

Various amounts of the medium chain triglycerides may be included in the pharmaceutical formulation. In one or more embodiments, the pharmaceutical formulation may comprise about 50 to about 95% by weight medium chain triglycerides, or about 85 to about 95% by weight medium chain triglycerides. Moreover, in exemplary embodiments, the pharmaceutical formulation may include from about 100 to about 300 mg, or from about 200 to 300 mg of the weight medium chain triglycerides, or about 225 to 275 mg of the weight medium chain triglycerides, or about 250 mg of the weight medium chain triglycerides.

Similar to medium chain triglycerides, "medium chain monoglycerides" and "medium chain diglycerides" are C6-C12 ester chains formed via the esterification of glycerol with one fatty acid or two fatty acids, respectively. Examples of commercially available medium chain mono/diglycerides may include the CAPMUL^{®} products produced by Abitec. It is also contemplated to use medium chain mono/diglyceride compounds that also include medium chain triglycerides, for example, the commercially available IMWITOR^{®} compositions produced by Sasol.

In exemplary embodiments, the medium chain mono/diglycerides may comprise CAPMUL^{®} MCM, which include medium chain mono/diglycerides of caprylic (C8)/ capric (C10) acid. While all grades of the CAPMUL^{®} MCM product line are suitable for use in the present invention, e.g., national formulary (NF) grade or CAPMUL^{®} MCM EP, it may be desirable to use to EP grade as it includes 3% glycerol, whereas the NF grade includes 7% glycerol.

In accord with one or more embodiments, the pharmaceutical formulation may comprise about 5% to about 25% by weight medium chain mono/diglycerides, or from about 5% to about 15% by weight medium chain mono/diglycerides. In exemplary embodiments, the pharmaceutical formulation may include about 20 mg to 50 mg of the weight medium chain mono/diglycerides, or about 25 mg to 30 mg of the weight medium chain mono/diglycerides, or about 25 mg of the weight medium chain mono/diglycerides.

Without being bound by theory, the mixture of medium chain triglycerides and medium chain mono/diglycerides is important for the bioavailability of the active inside the liquid-filled hard gel capsule formulation. While a soft gel capsule may only include medium chain mono/diglycerides, a hard gelatin capsule with only medium chain mono/diglycerides may not provide the requisite physical stability of the finished dosage forms. A mixture of medium chain triglycerides and medium chain mono/diglycerides inside a hard gelatin capsule may achieve the desired product stability, solubility, and bioavailability of the active. Consequently, the ratio by weight of the medium chain triglycerides to the medium chain mono/diglycerides facilitates the solubility and stability of the active within the non-emulsified mixture prior to and after adding the mixture into the capsule. The medium chain triglycerides and medium chain mono/diglycerides may be present at a ratio by weight of from about 10:1 to about 5:1, or from about 10:1 to about 7:1.

The invention may include other excipients known to one or ordinary skill in the art, e.g., excipients in the oral composition may be selected from diluents, binders, lubricants, disintegrants, flavoring agents, coloring agents, stabilizers, glidants, plasticizers, preservatives, sweeteners, etc.

Diluents may include liquid diluents such as any long chain triglyceride (arachis oil, almond oil, peanut oil, palm oil, palm kernel oil, blackcurrent seed oil, rice bran oil, soybean oil, canola oil, corn oil, coconut oil, cotton seed oil, castor oil, olive oil, Linn oils (Neem), sesame oil, primrose oil, vegetable oil, LIPEX^{®} 108 (Abitec), wheat germ oil, fish oil, rapeseed oil, sunflower oil and saffola oil. In alternative embodiments, it is contemplated that other diluents may be used, for example, diluents selected from calcium-aluminum silicates (SIPERNAT^{®} 106PQ), calcium carbonate, calcium phosphate dibasic, calcium phosphate tribasic, calcium sulfate, microcrystalline cellulose, microcrystalline silicified cellulose, powdered cellulose, dextrates, dextrose, fructose, lactitol, lactose anhydrous, lactose monohydrate, lactose dihydrate, lactose trihydrate, mannitol sorbitol, starch, pregelatinized starch, sucrose, talc, xylitol, maltose maltodextrin, maltitol, silicon dioxide, HPMC and combinations thereof.

The formulation includes the route of administration, type of preparation, non-active ingredients release of active, stability, scale-up, new processes for preparation of active, new processes for formulation.

*In vivo* performance evaluation includes pharmacokinetic data such as pK/pD such as Tₘₐₓ, Cₘₐₓ, plasma concentration curve, efficacy, side effects, etc.

The active includes all forms of the active, and besides the trimethyl form, includes but is not limited to intermediates, metabolites, enantiomers, polymorphs, crystalline structure, hydrates, stereoisomers, salts, bases, complexes, carriers, and derivatives and conjugates.

Other release profiles include but are not limited to controlled, enteric, sustained, fast, multi-phasic, etc.

Other known and to be determined uses of the inventive formulations of phloroglucinol and trimethylphloroglucinol are encompassed by the invention.

### Aspects:

Aspect 1: A pharmaceutical composition comprising a formulation of phloroglucinol and/or trimethylphloroglucinol and at least one excipient, where at least one of phloroglucinol or trimethylphloroglucinol is in both an immediate release (IR) formulation and an extended release formulation.

Aspect 2. The composition of Aspect 1 comprising 100% phloroglucinol.

Aspect 3. The composition of Aspect 1 comprising 100% trimethylphloroglucinol.

Aspect 4. The composition of Aspect 1 comprising phloroglucinol : trimethylphloroglucinol in a ratio selected from the group consisting of 90:10, 80:20, 70:30, 60:40, 50:50, 40:60, 30:70, 20:80, and 10:90.

Aspect 5. A dosage form of phloroglucinol and/or trimethylphloroglucinol, the dosage form containing an immediate release (IR) portion of a dose and an extended release (XR) portion of a dose.

Aspect 6. The dosage form of Aspect 5 where the IR portion delivers 100% of the dose in less than one hour.

Aspect 7. The dosage form of Aspect 5 where the XR portion delivers the dose over a period of 12 hours.

Aspect 8. The dosage form of Aspect 5 selected from the group consisting of a bilayer tablet containing IR and XR layers,
a trilayer tablet containing IR, XR and buffer layer between the IR and XR layer,
an XR tablet containing PG or TMP in the matrix layer and coated with IR layer PG or TMP,
a capsule containing IR tablet, a plug and XR tablet within an osmotic system that delivers the drug over a duration of 12 hours
a capsule containing IR beads and XR beads mixed in the appropriate ratio,
a capsule containing IR minitablets mixed with XR minitablets,
a capsule containing IR and XR granules coated with extended release polymers,
a capsule containing coated XR beads which are coated with the IR layer as the top coat,
a compressed tablet containing IR granules and coated XR beads embedded within the tablet,
a compressed tablet containing XR tablet embedded within the IR tablet,
an XR tablet suspended in a liquid drug solution within a capsule,
a sachet containing a mixture of IR and XR granules or beads,
a sachet containing a mixture of effervescent IR granules and coated XR granules,
an orally disintegrating tablet containing coated, delayed/extended release drug particles, beads, or granules,
a capsule containing drug solution and coated, delayed/extended release drug particles, beads, or granules,
a softgel containing drug solution and coated, delayed/extended release drug particles, beads, or granules, and
a liquid vehicle coated, delayed/extended release drug particles, beads, or granules.

Aspect 9. A pharmaceutical formulation comprising a first plurality of first active beads and a second plurality of second active beads, the formulation providing double-pulsed essentially simultaneous delivery of each of the first active and the second active to a patient orally administered the formulation.

Aspect 10. A pharmaceutical composition for delivery of one or more active salts, the composition comprising
(a) one or more pharmaceutically active salts covered with an immediate release coating; and
(b) one or more pharmaceutically active salts covered with an enteric release coating that provides for delayed pulsed enteric release, where the enteric release coating releases essentially all of the one or more active salts coated with the enteric coating within about 60 minutes after initiation of the delayed pulsed enteric release.

Aspect 11. A pharmaceutical formulation for delivery of a mixture of active salts effective to treat a patient, the formulation comprising
an immediate release dosage form that provides immediate release of an active upon oral administration of the formulation to the patient,
a delayed enteric release dosage form that provides delayed release of the active upon oral admin of the formulation to the patient, and
a pharmaceutically acceptable carrier.

Aspect 12. A pharmaceutical composition comprising a lipid-based formulation of phloroglucinol and/or trimethylphloroglucinol and at least one excipient, where at least one of phloroglucinol or trimethylphloroglucinol is in an immediate release (IR) formulation.

Aspect 13. The composition of Aspect 12 comprising 100% phloroglucinol.

Aspect 14. The composition of Aspect 12 comprising 100% trimethylphloroglucinol.

Aspect 15. The composition of Aspect 12 comprising phloroglucinol : trimethylphloroglucinol in a ratio selected from the group consisting of 90:10, 80:20, 70:30, 60:40, 50:50, 40:60, 30:70, 20:80, and 10:90.

Aspect 16. A dosage form of phloroglucinol and/or trimethylphloroglucinol, the dosage form containing an immediate release (IR) formulation.

The following Examples are provided to illustrate some of the concepts described within this disclosure. While each Example is considered to provide specific individual embodiments of composition, methods of preparation and use, none of the Examples should be considered to limit the more general embodiments described herein.

In the following examples, efforts have been made to ensure accuracy with respect to numbers used (e.g. amounts, temperature, etc.) but some experimental error and deviation should be accounted for. Unless indicated otherwise, temperature is in degrees C, pressure is at or near atmospheric.

### EXAMPLES

### Example 1

An example of a modified release formulation of phloroglucinol is provided as follows:

| **Phloroglucinol 200 mg Capsules** | | |
|---|---|---|
| **Composition** | **%w/w** | **Amount per 200 mg Dosage (mg/capsule)** |
| Phloroglucinol Anhydrous | 42.6 | 200.0 |
| Cellulose Microcrystalline PH200 NF/EP | 21.3 | 100.0 |
| ACRYL-EZE 93F19255 | 14.9 | 70.0 |
| Povidone K30 | 1.0 | 5.0 |
| Pullulan Capsule, Size 0 | 20.2 | 95.0 |
| **Theoretical Total Quantity** | **100.0** | **470.0** |

### Process for making the beads:

### Extrusion:

A wet mass containing a mixture of phloroglucinol and cellulose microcrystalline PH200 (MCC) is prepared at a 1:1 ratio using a top driven mixer. A mixture of 500 g phloroglucinol and 500 g MCC is dry mixed for 5 minutes. While mixing, 900 mL of water is added over 5 min. The wet mass is extruded through a 1.0 mm screen and formed into beads using a spheronization unit with 2.0 mm crosshatch plates. The beads are dried in an oven for 2 hours at 65°C.

### Enteric Coated:

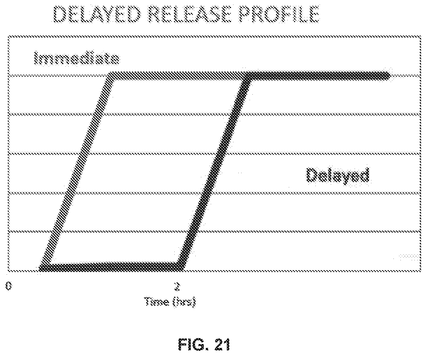

The beads are coated with Acryl-EZE Clear 93F1925 in a fluid bed with a Wurster column. The coating solution is applied through a 1 mm nozzle at a rate of 10 g/min. Enough coating solution is applied to support a theoretical weight gain of about 35%. The release profile of the coated and uncoated beads is analyzed using dissolution testing. See, e.g., FIG. 21 showing the release of the drug at different times.

### Each of the following references are included:

Chang and Robinson, chapter 4: Sustained Drug Release from Tablets and Particles Through Coating, Pharmaceutical Dosage Forms: Tablets, vol. 3, Eds. Lieberman, Lachman, and Schwartz, Marcel Dekker, Inc., 1991.

Campbell and Sackett, chapter 3: Film coating, Pharmaceutical Unit Operations: Coating, edited by Avis, Shukla, and Chang, Interpharm Press, Inc., 1999.

The embodiments shown and described in the specification are only specific embodiments of inventors who are skilled in the art and are not limiting in any way.

## Claims

1. An oral dosage unit, comprising:
an immediate release formulation comprising phloroglucinol, trimethylphloroglucinol, or a pharmaceutically acceptable salt thereof, wherein at least 90% by weight, based on the weight of the immediate release formulation, of phloroglucinol, trimethylphloroglucinol, or a pharmaceutically acceptable salt thereof is released from the dosage unit from 5 minutes to 2 hours, as measured by the USP 2 paddle method at 50 rpm in 750 mL of an aqueous solution comprising 0.1N HCl solution at 37°C; and
a modified release formulation comprising phloroglucinol, trimethylphloroglucinol, or a pharmaceutically acceptable salt thereof, wherein at least 90% by weight, based on the weight of the modified release formulation, of phloroglucinol, trimethylphloroglucinol, or a pharmaceutically acceptable salt thereof, is released from the dosage unit after at least 2 hours, as measured by the USP 2 paddle method at 50 rpm in 1000 mL of an aqueous solution comprising 0.1N HCl and 20 mM sodium phosphate tribasic at a pH of 6.8 at 37°C.

2. The oral dosage unit of claim 1, wherein one or both of the immediate release formulation or modified release formulation comprises phloroglucinol or a pharmaceutically acceptable salt thereof.

3. The oral dosage unit of claim 1 or 2, wherein one or both of the immediate release formulation or modified release formulation comprises trimethylphloroglucinol or a pharmaceutically acceptable salt thereof.

4. The oral dosage unit of claim 3, wherein the ratio of phloroglucinol to trimethylphloroglucinol is 90:10 to 10:90 such as 80:20, 70:30, 60:40, 50:50, 40:60, 30:70, or 20:80.

5. The oral dosage unit of any one of the preceding claims, wherein the phloroglucinol, trimethylphloroglucinol, or a pharmaceutically acceptable salt thereof is released from the modified release formulation over a period of 2 hours to 12 hours.

6. The oral dosage unit of any one of the preceding claims, wherein a portion of the immediate release formulation is coated with the modified release formulation.

7. The oral dosage unit of claim 6, comprising 10 to 50% by weight, based on the weight of the oral dosage unit, of the modified release formulation.

8. The oral dosage unit of any one of the preceding claims, further comprising a second modified release formulation optionally wherein a portion of the immediate release formulation is coated with the second modified release formulation.

9. The oral dosage unit of claim 8, wherein at least 90% by weight, based on the weight of the second modified release formulation, of phloroglucinol, trimethylphloroglucinol, or a pharmaceutically acceptable salt thereof is released from the dosage unit after between 4 to 6 hours, as measured by the USP 2 paddle method at 50 rpm in 1000 mL of an aqueous solution comprising 0.1N HCl and 20 mM sodium phosphate tribasic at a pH of 6.8 at 37°C.

10. The oral dosage unit of any one of the preceding claims, wherein the modified release formulation comprises an enteric polymer such as an acrylic polymer preferably the Acryl-EZE^{®} polymer or a polyvinyl acetate phthalate polymer preferably the Sureteric^{®} polymer.

11. The oral dosage unit of any one of the preceding claims, wherein the immediate release formulation, modified release formulation, or a combination thereof is in the form of a bead or granule.

12. The oral dosage unit of any one of the preceding claims, comprising beads comprising the immediate release formulation and beads comprising the modified release formulation.

13. The oral dosage unit of any one of the preceding claims, comprising 50 mg to 800 mg of phloroglucinol, trimethylphloroglucinol, or a pharmaceutically acceptable salt thereof in the modified release formulation.

14. The oral dosage unit of any one of the preceding claims, comprising 50 mg to 1000 mg, preferably 50 mg to 800 mg, of phloroglucinol, trimethylphloroglucinol, or a pharmaceutically acceptable salt thereof.

15. The oral dosage unit of claim 1, comprising:
(a) an oral dosage unit comprising:
a plurality of beads, each bead comprising an immediate release formulation comprising phloroglucinol, trimethylphloroglucinol, or a pharmaceutically acceptable salt thereof, wherein at least 90% by weight, based on the weight of the immediate release formulation, of phloroglucinol, trimethylphloroglucinol, or a pharmaceutically acceptable salt thereof is released from the dosage unit after 1 hour, as measured by the USP 2 paddle method at 50 rpm in 750 mL of an aqueous solution comprising 0.1N HCl at 37°C; and
a plurality of beads, each bead comprising a modified release formulation comprising phloroglucinol, trimethylphloroglucinol, or a pharmaceutically acceptable salt thereof, wherein at least 90% by weight, based on the weight of the modified release formulation, phloroglucinol, trimethylphloroglucinol, or a pharmaceutically acceptable salt thereof, is released from the dosage unit after at least 2 hours, as measured by the USP 2 paddle method at 50 rpm in 1000 mL of an aqueous solution comprising 0.1N HCl and 20 mM sodium phosphate tribasic at a pH of 6.8 at 37°C; or
(b) an oral dosage unit comprising a plurality of beads, each bead comprising:
a core that is in the form of an immediate release formulation comprising phloroglucinol, trimethylphloroglucinol, or a pharmaceutically acceptable salt thereof, wherein at least 90% by weight, based on the weight of the immediate release formulation, of phloroglucinol, trimethylphloroglucinol, or a pharmaceutically acceptable salt thereof is released from the dosage unit after 1 hour, as measured by the USP 2 paddle method at 50 rpm in 750 mL of an aqueous solution comprising 0.1N HCl at 37°C; and
a coating over the core that is:
(i) a modified release formulation comprising phloroglucinol, trimethylphloroglucinol, or a pharmaceutically acceptable salt thereof, wherein at least 90% by weight, based on the weight of the modified release formulation, phloroglucinol, trimethylphloroglucinol, or a pharmaceutically acceptable salt thereof, is released from the dosage unit after at least 2 hours, as measured by the USP 2 paddle method at 50 rpm in 1000 mL of an aqueous solution comprising 0.1N HCl and 20 mM sodium phosphate tribasic at a pH of 6.8 at 37°C; or
(ii) a modified release formulation comprising phloroglucinol, trimethylphloroglucinol, or a pharmaceutically acceptable salt thereof, wherein at least 90% by weight, based on the weight of the modified release formulation, phloroglucinol, trimethylphloroglucinol, or a pharmaceutically acceptable salt thereof, is released from the dosage unit after between 4 to 6 hours, as measured by the USP 2 paddle method at 50 rpm in 1000 mL of an aqueous solution comprising 0.1N HCl and 20 mM sodium phosphate tribasic at a pH of 6.8 at 37°C;
(iii) or a combination of (i) and (ii).

16. The oral dosage unit of any one of the preceding claims, for use in spasmodic condition in a subject.

17. The oral dosage unit for the use of claim 16, wherein the spasmodic condition is a sudden involuntary muscle contraction of the bronchi, stomach, intestine, ureter, gall bladder, kidney, or bile duct.

18. The oral dosage unit for the use of claim 16, wherein the spasmodic condition is a urinary tract spasm, gallstones, a gastrointestinal disorder, inflammatory bowel syndrome, renal colicky pain, or a spastic condition of the biliary tract.

## Patentansprüche

1. Orale Dosierungseinheit, umfassend:
eine Formulierung mit sofortiger Freisetzung, die Phloroglucinol, Trimethylphloroglucinol oder ein pharmazeutisch annehmbares Salz davon umfasst, wobei mindestens 90 Gew.-% (w/w), bezogen auf das Gewicht der Formulierung mit sofortiger Freisetzung, von Phloroglucinol, Trimethylphloroglucinol oder eines pharmazeutisch annehmbaren Salzes davon aus der Dosierungseinheit von 5 Minuten bis 2 Stunden freigesetzt werden, gemessen nach dem USP-2-Paddle-Verfahren bei 50 U/min in 750 ml einer wässrigen Lösung, die 0,1N HCl-Lösung, bei 37 °C umfasst; und
eine Formulierung mit modifizierter Freisetzung, die Phloroglucinol, Trimethylphloroglucinol oder ein pharmazeutisch annehmbares Salz davon umfasst, wobei mindestens 90 Gew.-% (w/w), bezogen auf das Gewicht der Formulierung mit modifizierter Freisetzung, von Phloroglucinol, Trimethylphloroglucinol oder eines pharmazeutisch annehmbaren Salzes davon aus der Dosierungseinheit nach mindestens 2 Stunden freigesetzt werden, gemessen nach dem USP-2-Paddle-Verfahren bei 50 U/min in 1000 ml einer wässrigen Lösung, die 0,1N HCl und 20 mM Trinatriumphosphat umfasst, bei einem pH-Wert von 6,8 bei 37 °C.

2. Orale Dosierungseinheit nach Anspruch 1, wobei eine oder beide der Formulierung mit sofortiger Freisetzung oder der Formulierung mit modifizierter Freisetzung Phloroglucinol oder ein pharmazeutisch annehmbares Salz davon umfasst.

3. Orale Dosierungseinheit nach Anspruch 1 oder 2, wobei eine oder beide der Formulierung mit sofortiger Freisetzung oder der Formulierung mit modifizierter Freisetzung Trimethylphloroglucinol oder ein pharmazeutisch annehmbares Salz davon umfasst.

4. Orale Dosierungseinheit nach Anspruch 3, wobei das Verhältnis von Phloroglucinol zu Trimethylphloroglucinol 90:10 bis 10:90 beträgt, wie etwa 80:20, 70:30, 60:40, 50:50, 40:60, 30:70 oder 20:80.

5. Orale Dosierungseinheit nach einem der vorhergehenden Ansprüche, wobei das Phloroglucinol, Trimethylphloroglucinol oder ein pharmazeutisch annehmbares Salz davon aus der Formulierung mit modifizierter Freisetzung über einen Zeitraum von 2 Stunden bis 12 Stunden freigesetzt wird.

6. Orale Dosierungseinheit nach einem der vorhergehenden Ansprüche, wobei ein Teil der Formulierung mit sofortiger Freisetzung mit der Formulierung mit modifizierter Freisetzung beschichtet ist.

7. Orale Dosierungseinheit nach Anspruch 6, welches 10 bis 50 Gew.-% (w/w), bezogen auf das Gewicht der oralen Dosierungseinheit, der Formulierung mit modifizierter Freisetzung umfasst.

8. Orale Dosierungseinheit nach einem der vorhergehenden Ansprüche, ferner eine zweite Formulierung mit modifizierter Freisetzung umfassend, wobei optional ein Teil der Formulierung mit sofortiger Freisetzung mit der zweiten Formulierung mit modifizierter Freisetzung beschichtet ist.

9. Orale Dosierungseinheit nach Anspruch 8, wobei mindestens 90 Gew.-% (w/w), bezogen auf das Gewicht der zweiten Formulierung mit modifizierter Freisetzung, von Phloroglucinol, Trimethylphloroglucinol oder einem pharmazeutisch annehmbaren Salz davon aus der Dosierungseinheit nach zwischen 4 bis 6 Stunden freigesetzt werden, gemessen nach dem USP-2-Paddle-Verfahren bei 50 U/min in 1000 ml einer wässrigen Lösung, die 0,1N HCl und 20 mM Natriumtriphosphat umfasst, bei einem pH-Wert von 6,8 bei 37 °C.

10. Orale Dosierungseinheit nach einem der vorhergehenden Ansprüche, wobei die Formulierung mit modifizierter Freisetzung ein magensaftresistentes Polymer, wie ein Acrylpolymer, vorzugsweise das Acryl-EZE^{®}-Polymer, oder ein Polyvinylacetatphthalat-Polymer, vorzugsweise das Sureteric^{®}-Polymer, umfasst.

11. Orale Dosierungseinheit nach einem der vorhergehenden Ansprüche, wobei die Formulierung mit sofortiger Freisetzung, Formulierung mit modifizierter Freisetzung oder eine Kombination davon in Form einer Kugel oder eines Granulats vorliegt.

12. Orale Dosierungseinheit nach einem der vorhergehenden Ansprüche, die Kugeln, die die Formulierung mit sofortiger Freisetzung umfassen, und Kugeln, die die Formulierung mit modifizierter Freisetzung umfassen, umfasst.

13. Orale Dosierungseinheit nach einem der vorhergehenden Ansprüche, die 50 mg bis 800 mg Phloroglucinol, Trimethylphloroglucinol oder eines pharmazeutisch annehmbaren Salzes davon in der Formulierung mit modifizierter Freisetzung umfasst.

14. Orale Dosierungseinheit nach einem der vorhergehenden Ansprüche, die 50 mg bis 1000 mg, vorzugsweise 50 mg bis 800 mg Phloroglucinol, Trimethylphloroglucinol oder eines pharmazeutisch annehmbaren Salzes davon umfasst.

15. Orale Dosierungseinheit nach Anspruch 1, umfassend:
(a) eine orale Dosierungseinheit, die Folgendes umfasst:
eine Vielzahl von Kugeln, wobei jede Kugel eine Formulierung mit sofortiger Freisetzung, die Phloroglucinol, Trimethylphloroglucinol oder ein pharmazeutisch annehmbares Salz davon umfasst, wobei mindestens 90 Gew.-% (w/w), bezogen auf das Gewicht der Formulierung mit sofortiger Freisetzung, des Phloroglucinol, Trimethylphloroglucinol oder eines pharmazeutisch annehmbaren Salzes davon aus der Dosierungseinheit nach 1 Stunde freigesetzt werden, gemessen nach dem USP-2-Paddle-Verfahren bei 50 U/min in 750 ml einer wässrigen Lösung, die 0,1N HCl umfasst, bei 37 °C; und
eine Vielzahl von Kugeln, wobei jede Kugel eine Formulierung mit modifizierter Freisetzung, die Phloroglucinol, Trimethylphloroglucinol oder ein pharmazeutisch annehmbares Salz davon umfasst, wobei mindestens 90 Gew.-% (w/w), bezogen auf das Gewicht der Formulierung mit modifizierter Freisetzung, des Phloroglucinol, Trimethylphloroglucinol oder eines pharmazeutisch annehmbaren Salzes davon aus der Dosierungseinheit nach mindestens 2 Stunden freigesetzt werden, gemessen nach dem USP-2-Paddle-Verfahren bei 50 U/min in 1000 ml einer wässrigen Lösung, die 0,1N HCl und 20 mM Trinatriumphosphat umfasst, bei einem pH-Wert von 6,8 bei 37 °C; oder
(b) eine orale Dosierungseinheit, die eine Vielzahl von Kugeln umfasst, wobei jede Kugel Folgendes umfasst:
einen Kern, der in der Form einer Formulierung mit sofortiger Freisetzung vorliegt, die Phloroglucinol, Trimethylphloroglucinol oder ein pharmazeutisch annehmbares Salz davon umfasst, wobei mindestens 90 Gew.-% (w/w), bezogen auf das Gewicht der Formulierung mit sofortiger Freisetzung, des Phloroglucinol, Trimethylphloroglucinol oder eines pharmazeutisch annehmbaren Salzes davon aus der Dosierungseinheit nach 1 Stunde freigesetzt werden, gemessen nach dem USP-2-Paddle-Verfahren bei 50 U/min in 750 ml einer wässrigen Lösung, die 0,1N HCl umfasst, bei 37 °C; und
eine Beschichtung über dem Kern, die Folgendes ist:
(i) eine Formulierung mit modifizierter Freisetzung, die Phloroglucinol, Trimethylphloroglucinol oder ein pharmazeutisch annehmbares Salz davon umfasst, wobei mindestens 90 Gew.-% (w/w), bezogen auf das Gewicht der Formulierung mit modifizierter Freisetzung, von Phloroglucinol, Trimethylphloroglucinol oder eines pharmazeutisch annehmbaren Salzes davon aus der Dosierungseinheit nach mindestens 2 Stunden freigesetzt werden, gemessen nach dem USP-2-Paddle-Verfahren bei 50 U/min in 1000 ml einer wässrigen Lösung, die 0,1N HCl und 20 mM Trinatriumphosphat umfasst, bei einem pH-Wert von 6,8 bei 37 °C; oder
(ii) eine Formulierung mit modifizierter Freisetzung, die Phloroglucinol, Trimethylphloroglucinol oder ein pharmazeutisch annehmbares Salz davon umfasst, wobei mindestens 90 Gew.-% (w/w), bezogen auf das Gewicht der Formulierung mit modifizierter Freisetzung, von Phloroglucinol, Trimethylphloroglucinol oder eines pharmazeutisch annehmbaren Salzes davon aus der Dosierungseinheit nach zwischen 4 bis 6 Stunden freigesetzt werden, gemessen nach dem USP-2-Paddle-Verfahren bei 50 U/min in 1000 ml einer wässrigen Lösung, die 0,1N HCl und 20 mM Trinatriumphosphat umfasst, bei einem pH-Wert von 6,8 bei 37 °C;
(iii) oder eine Kombination aus (i) und (ii).

16. Orale Dosierungseinheit nach einem der vorhergehenden Ansprüche zur Verwendung in einem krampfartigen Zustand bei einem Subjekt.

17. Orale Dosierungseinheit zur Verwendung nach Anspruch 16, wobei der krampfartige Zustand eine plötzliche unwillkürliche Muskelkontraktion der Bronchien, des Magens, des Darms, des Harnleiters, der Gallenblase, der Niere oder des Gallengangs ist.

18. Orale Dosierungseinheit zur Verwendung nach Anspruch 16, wobei der krampfartige Zustand ein Krampf der Harnwege, Gallensteine, eine Magen-Darm-Erkrankung, ein entzündliches Darmsyndrom, Nierenkolikschmerzen oder ein spastischer Zustand der Gallenwege ist.

## Revendications

1. Unité posologique orale, comprenant :
une formulation à libération immédiate comprenant du phloroglucinol, du triméthylphloroglucinol ou un sel pharmaceutiquement acceptable de ceux-ci, dans laquelle au moins 90 % en poids, sur la base du poids de la formulation à libération immédiate, de phloroglucinol, de triméthylphloroglucinol ou d'un sel pharmaceutiquement acceptable de ceux-ci sont libérés de l'unité posologique en de 5 minutes à 2 heures, telle que mesurée par la méthode de la palette USP 2 à 50 tr/min dans 750 ml d'une solution aqueuse comprenant une solution de HCl 0,1 N à 37 °C ; et
une formulation à libération modifiée comprenant du phloroglucinol, du triméthylphloroglucinol ou un sel pharmaceutiquement acceptable de ceux-ci, dans laquelle au moins 90 % en poids, sur la base du poids de la formulation à libération modifiée, de phloroglucinol, de triméthylphloroglucinol ou d'un sel pharmaceutiquement acceptable de ceux-ci sont libérés de l'unité posologique après au moins 2 heures, telle que mesurée par la méthode de la palette USP 2 à 50 tr/min dans 1000 ml d'une solution aqueuse comprenant du HCl 0,1 N et du phosphate de sodium tribasique 20 mM à un pH de 6,8 à 37 °C.

2. Unité posologique orale selon la revendication 1, dans laquelle l'une ou les deux parmi la formulation à libération immédiate ou la formulation à libération modifiée comprend du phloroglucinol ou un sel pharmaceutiquement acceptable de celui-ci .

3. Unité posologique orale selon la revendication 1 ou 2, dans laquelle l'une ou les deux parmi la formulation à libération immédiate ou la formulation à libération modifiée comprend du triméthylphloroglucinol ou un sel pharmaceutiquement acceptable de celui-ci.

4. Unité posologique orale selon la revendication 3, dans laquelle le rapport du phloroglucinol au triméthylphloroglucinol est de 90:10 à 10:90, comme 80:20, 70:30, 60:40, 50:50, 40:60, 30:70 ou 20:80.

5. Unité posologique orale selon l'une quelconque des revendications précédentes, dans laquelle le phloroglucinol, le triméthylphloroglucinol ou un sel pharmaceutiquement acceptable de ceux-ci est libéré de la formulation à libération modifiée sur une période de 2 heures à 12 heures.

6. Unité posologique orale selon l'une quelconque des revendications précédentes, dans laquelle une partie de la formulation à libération immédiate est enrobée avec la formulation à libération modifiée.

7. Unité posologique orale selon la revendication 6, comprenant 10 à 50 % en poids, sur la base du poids de l'unité posologique orale, de la formulation à libération modifiée.

8. Unité posologique orale selon l'une quelconque des revendications précédentes, comprenant en outre une seconde formulation à libération modifiée éventuellement dans laquelle une partie de la formulation à libération immédiate est enrobée avec la seconde formulation à libération modifiée.

9. Unité posologique orale selon la revendication 8, dans laquelle au moins 90 % en poids, sur la base du poids de la seconde formulation à libération modifiée, de phloroglucinol, de triméthylphloroglucinol ou d'un sel pharmaceutiquement acceptable de ceux-ci sont libérés de l'unité posologique après entre 4 et 6 heures, telle que mesurée par la méthode de la palette USP 2 à 50 tr/min dans 1000 ml d'une solution aqueuse comprenant du HCl 0,1 N et du phosphate de sodium tribasique 20 mM à un pH de 6,8 à 37 °C.

10. Unité posologique orale selon l'une quelconque des revendications précédentes, dans laquelle la formulation à libération modifiée comprend un polymère entérique tel qu'un polymère acrylique, de préférence le polymère Acryl-EZE^{®} ou un polymère de poly(acétate-phtalate de vinyle), de préférence le polymère Sureteric^{®}.

11. Unité posologique orale selon l'une quelconque des revendications précédentes, dans laquelle la formulation à libération immédiate, la formulation à libération modifiée ou une combinaison de celles-ci est sous la forme d'une bille ou d'un granulé.

12. Unité posologique orale selon l'une quelconque des revendications précédentes, comprenant des billes comprenant la formulation à libération immédiate et des billes comprenant la formulation à libération modifiée.

13. Unité posologique orale selon l'une quelconque des revendications précédentes, comprenant de 50 mg à 800 mg de phloroglucinol, de triméthylphloroglucinol ou d'un sel pharmaceutiquement acceptable de ceux-ci dans la formulation à libération modifiée.

14. Unité posologique orale selon l'une quelconque des revendications précédentes, comprenant de 50 mg à 1000 mg, de préférence 50 mg à 800 mg, de phloroglucinol, de triméthylphloroglucinol ou d'un sel pharmaceutiquement acceptable de ceux-ci.

15. Unité posologique orale selon la revendication 1, comprenant :
(a) une unité posologique orale, comprenant :
une pluralité de billes, chaque bille comprenant une formulation à libération immédiate comprenant du phloroglucinol, du triméthylphloroglucinol ou un sel pharmaceutiquement acceptable de ceux-ci, dans laquelle au moins 90 % en poids, sur la base du poids de la formulation à libération immédiate, de phloroglucinol, de triméthylphloroglucinol ou d'un sel pharmaceutiquement acceptable de ceux-ci sont libérés de l'unité posologique après 1 heure, telle que mesuré par la méthode de la palette USP 2 à 50 tr/min dans 750 ml d'une solution aqueuse comprenant du HCl 0,1 N à 37 °C ; et
une pluralité de billes, chaque bille comprenant une formulation à libération modifiée comprenant du phloroglucinol, du triméthylphloroglucinol ou un sel pharmaceutiquement acceptable de ceux-ci, dans laquelle au moins 90 % en poids, sur la base du poids de la formulation à libération modifiée, de phloroglucinol, de triméthylphloroglucinol ou d'un sel pharmaceutiquement acceptable de ceux-ci sont libérés de l'unité posologique après au moins 2 heures, telle que mesuré par la méthode de la palette USP 2 à 50 tr/min dans 1000 ml d'une solution aqueuse comprenant du HCl 0,1 N et du phosphate de sodium tribasique 20 mM à un pH de 6,8 à 37 °C ; ou
(b) une unité posologique orale comprenant une pluralité de billes, chaque bille comprenant :
un noyau qui est sous la forme d'une formulation à libération immédiate comprenant du phloroglucinol, du triméthylphloroglucinol ou un sel pharmaceutiquement acceptable de ceux-ci, dans laquelle au moins 90 % en poids, sur la base du poids de la formulation à libération immédiate, de phloroglucinol, de triméthylphloroglucinol ou d'un sel pharmaceutiquement acceptable de ceux-ci sont libérés de l'unité posologique après 1 heure, telle que mesuré par la méthode de la palette USP 2 à 50 tr/min dans 750 ml d'une solution aqueuse comprenant du HCl 0,1 N à 37 °C ; et
un enrobage sur le noyau qui est :
(i) une formulation à libération modifiée comprenant du phloroglucinol, du triméthylphloroglucinol ou un sel pharmaceutiquement acceptable de ceux-ci, dans laquelle au moins 90 % en poids, sur la base du poids de la formulation à libération modifiée, de phloroglucinol, de triméthylphloroglucinol ou d'un sel pharmaceutiquement acceptable de ceux-ci sont libérés de l'unité posologique après au moins 2 heures, telle que mesuré par la méthode de la palette USP 2 à 50 tr/min dans 1000 ml d'une solution aqueuse comprenant du HCl 0,1 N et du phosphate de sodium tribasique 20 mM à un pH de 6,8 à 37 °C ; ou
(ii) une formulation à libération modifiée comprenant du phloroglucinol, du triméthylphloroglucinol ou un sel pharmaceutiquement acceptable de ceux-ci, dans laquelle au moins 90 % en poids, sur la base du poids de la formulation à libération modifiée, de phloroglucinol, de triméthylphloroglucinol ou d'un sel pharmaceutiquement acceptable de ceux-ci sont libérés de l'unité posologique après entre 4 et 6 heures, telle que mesuré par la méthode de la palette USP 2 à 50 tr/min dans 1000 ml d'une solution aqueuse comprenant du HCl 0,1 N et du phosphate de sodium tribasique 20 mM à un pH de 6,8 à 37 °C ;
(iii) ou une combinaison de (i) et (ii).

16. Unité posologique orale selon l'une quelconque des revendications précédentes, destinée à être utilisée dans une affection spasmodique chez un sujet.

17. Unité posologique orale destinée à être utilisée selon la revendication 16, dans laquelle l'affection spasmodique est une contraction musculaire involontaire soudaine des bronches, de l'estomac, de l'intestin, de l'uretère, de la vésicule biliaire, des reins ou des voies biliaires.

18. Unité posologique orale destinée à être utilisée selon la revendication 16, dans laquelle l'affection spasmodique est un spasme des voies urinaires, des calculs biliaires, un trouble gastro-intestinal, un syndrome intestinal inflammatoire, une douleur due à des coliques rénales ou une affection spasmodique des voies biliaires.
